# EUROPEAN PATENT APPLICATION

(11) **EP 4 834 936 A1**
(43) Date of publication of application: **04.11.2026**
(21) Application number: 24911337.4
(22) Date of filing: 26.12.2024
(51) Int. Cl.: C07K 16/28, C07K 16/30, A61K 39/395, C12N 15/13

(54) **INVENTION AND USE OF FOLR1-SPECIFIC ANTIBODY**

(30) Priority: 28.12.2023 CN 202311835237
(71) Applicant: Shanghai Hongcheng Biopharmaceutical Co., Ltd., Shanghai 201315 (CN)
(72) Inventor: XI, Yue, Shanghai 201203 (CN); MIN, Hao, Shanghai 201203 (CN); WANG, Xingding, Shanghai 201203 (CN); XU, Jun, Shanghai 201203 (CN); WANG, Yonghui, Shanghai 201203 (CN); ZHAO, Wenkai, Shanghai 201203 (CN); HUANG, Fanjuan, Shanghai 201203 (CN)
(74) Representative: Santoro, Sofia
(86) International application number: PCT/CN2024/142744
(87) International publication number: WO 2025/140416

(57) **Abstract**

The present application relates to antibodies that specifically recognize FOLR1, methods for their preparation and uses thereof. The present application also relates to antibody-drug conjugates (ADCs) targeting FOLR1 and compositions containing said molecules. Furthermore, the present disclosure relates to the therapeutic and diagnostic uses of these antibodies, antibody fragments, and antibody-drug conjugates.

## Description

### TECHNICAL FIELD

The present application relates to antibodies that specifically recognize FOLR1, methods for preparing the same, and uses thereof. The present application also relates to antibody-drug conjugates (ADCs) targeting FOLR1 and compositions containing the same. In addition, the present disclosure also relates to therapeutic and diagnostic uses of these antibodies, antibody fragments, and antibody-drug conjugates.

### BACKGROUND

It is estimated that in 2020, there were 308,069 new cases of ovarian cancer worldwide and 193,811 deaths from ovarian cancer (PMID: 26557500). In China, the annual incidence of ovarian cancer ranks third among female reproductive system tumors, showing a trend of annual increase, while the mortality rate ranks first among malignant tumors of the female reproductive tract, making it a malignant tumor that seriously threatens women's health. Although the disease is initially sensitive to chemotherapy and most patients can achieve remission through first-line platinum-based chemotherapy, up to 80% of patients will eventually relapse and require further treatment. Patients who are sensitive to platinum-based therapy eventually develop resistance to platinum-based chemotherapy after multiple relapses. For platinum-resistant recurrent ovarian cancer (ROC), the response rate to platinum-based re-treatment is <10%, and the prognosis is extremely poor. Therefore, the treatment of platinum-resistant ROC is the most challenging problem in the clinical treatment of ovarian cancer, severely affecting the prognosis of ovarian cancer patients.

As one of the essential vitamins for the human body, folic acid needs to be taken from external sources, and is transported into cells through endocytosis mediated by folate receptors (FR). Folate receptors have four subtypes: folate receptor α (FRα, also known as folate receptor 1 (FOLR1)), folate receptor β (FRβ, also known as folate receptor 2 (FOLR2)), folate receptor γ (FRγ, also known as folate receptor 3 (FOLR3)), and folate receptor δ (FRδ, also known as folate receptor 4 (FOLR4)).

Folate receptor α is a 38-40 kDa glycosylphosphatidylinositol (GPI)-anchored cell surface glycoprotein (PMID: 2557328), encoded by the FOLR1 gene, and is a member of the high-affinity folate receptor (FRs) family that specifically binds to 5-methyltetrahydrofolate and free folic acid and participates in cellular folate uptake through endocytosis (PMID: 1968465). FRα is not expressed in most normal tissues, except for low expression in the kidneys and lungs (PMID: 1596899). FRα is highly expressed in a variety of tumors including ovarian cancer, triple-negative breast cancer, endometrial cancer, mesothelioma, and lung cancer (PMID: 25349970, PMID: 15745749, PMID: 32152484). In addition, FRα is continuously expressed in metastatic lesions and recurrent cancers of ovarian cancer patients, as well as in epithelial ovarian cancer and endometrial cancer after chemotherapy (PMID: 18222534, PMID: 23558051, PMID: 25904506). FRα overexpression is also associated with migration and invasion of tumor cells (PMID: 12776159, PMC3492371).

Antibody-drug conjugates (ADCs) are derivatives of traditional antibody drugs, composed of a monoclonal antibody targeting a specific antigen and a small molecule cytotoxic drug conjugated through a linker, combining both the targeting specificity of the antibody and the potent killing effect of traditional small molecule drugs, with the primary indication being malignant tumors. ADCs have the potential to combine the favorable pharmacokinetics, biodistribution, and tumor-targeting properties of antibodies with the potent cell-killing mechanism provided by the attached small molecule cytotoxic drugs (or payloads). ADCs have stringent requirements for target antigens; suitable targets for ADC development are generally antigens that are highly expressed in tumors but have low or almost no expression in healthy tissues; and the target antigen should possess internalization properties.

The high expression of FOLR1 in various tumors such as ovarian cancer, non-small cell lung cancer, and triple-negative breast cancer, combined with its lack of expression in most normal tissues, makes it a desirable target for ADC development. Several ADCs targeting FOLR1 have been developed. For example, mirvetuximab soravtansine (also known as IMGN853, trade name Elahere) is an antibody-drug conjugate targeting FOLR1, composed of an anti-folate receptor α antibody, a cleavable linker, and the maytansinoid DM4. IMGN853 has achieved favorable efficacy in patients with platinum-resistant ovarian cancer. In patients with FOLR1-overexpressing platinum-resistant ovarian cancer who had previously received bevacizumab treatment, IMGN853 treatment achieved an objective response rate (ORR) of 32.4%, a median duration of response (DOR) of 6.9 months, and a median progression-free survival (PFS) of 4.3 months (DOI: 10.1016/S0090-8258(22)01297-5).

However, IMGN853 is only effective in patients with high FOLR1 expression, and is ineffective in patients with low to moderate FOLR1 expression. In addition, there are concerns about the toxicity of IMGN853. In clinical trials, 31% of patients experienced serious adverse reactions when treated with IMGN853.

Therefore, there remains an urgent need in the field of tumor therapy to develop monoclonal antibodies or ADC drugs targeting FOLR1 with enhanced efficacy, and the present application meets this need to a certain extent.

### SUMMARY OF THE INVENTION

In a first aspect, the present disclosure provides antibodies targeting human FOLR1, which have the following advantages:
(1) binding to human FOLR1 and target cells expressing human FOLR1 with high affinity;
(2) having favorable cynomolgus monkey cross-reactivity;
(3) being capable of entering cells through endocytosis;
(4) not blocking the binding of folic acid to the FOLR1 receptor;
(5) being suitable for constructing antibody-drug conjugates for effective therapy;

In one embodiment, the present disclosure provides an anti-FOLR1 antibody and antigen-binding fragment thereof that specifically binds FOLR1, comprising:
1) three heavy chain CDRs (HCDR1, HCDR2, HCDR3) comprised in a heavy chain variable region as set forth in SEQ ID NO: 4 and/or three light chain CDRs (LCDR1, LCDR2, LCDR3) comprised in a light chain variable region as set forth in SEQ ID NO: 8;
2) three heavy chain CDRs (HCDR1, HCDR2, HCDR3) comprised in a heavy chain variable region as set forth in SEQ ID NO: 12 and/or three light chain CDRs (LCDR1, LCDR2, LCDR3) comprised in a light chain variable region as set forth in SEQ ID NO: 16;
3) three heavy chain CDRs (HCDR1, HCDR2, HCDR3) comprised in a heavy chain variable region as set forth in SEQ ID NO: 20 and/or three light chain CDRs (LCDR1, LCDR2, LCDR3) comprised in a light chain variable region as set forth in SEQ ID NO: 24; or
4) three heavy chain CDRs (HCDR1, HCDR2, HCDR3) comprised in a heavy chain variable region as set forth in SEQ ID NO: 28 and/or three light chain CDRs (LCDR1, LCDR2, LCDR3) comprised in a light chain variable region as set forth in SEQ ID NO: 32.

In one embodiment, the present disclosure provides an anti-FOLR1 antibody and antigen-binding fragment thereof that specifically binds FOLR1, comprising:
1) HCDR1, HCDR2, HCDR3 comprising sequences as set forth in SEQ ID NOs: 1, 2, and 3, or comprising sequences containing one or more amino acid substitutions (e.g., conservative substitutions), deletions, or insertions relative to SEQ ID NOs: 1, 2, and 3, respectively, or any combination thereof, or consisting of SEQ ID NOs: 1, 2, and 3; and/or LCDR1, LCDR2, LCDR3 comprising sequences as set forth in SEQ ID NOs: 5, 6, and 7, or comprising sequences containing one or more amino acid substitutions (e.g., conservative substitutions), deletions, or insertions relative to SEQ ID NOs: 5, 6, and 7, respectively, or any combination thereof, or consisting of SEQ ID NOs: 5, 6, and 7;
2) HCDR1, HCDR2, HCDR3 comprising sequences as set forth in SEQ ID NOs: 9, 10, and 11, or comprising sequences containing one or more amino acid substitutions (e.g., conservative substitutions), deletions, or insertions relative to SEQ ID NOs: 9, 10, and 11, respectively, or any combination thereof, or consisting of SEQ ID NOs: 9, 10, and 11; and/or LCDR1, LCDR2, LCDR3 comprising sequences as set forth in SEQ ID NOs: 13, 14, and 15, or comprising sequences containing one or more amino acid substitutions (e.g., conservative substitutions), deletions, or insertions relative to SEQ ID NOs: 13, 14, and 15, respectively, or any combination thereof, or consisting of SEQ ID NOs: 13, 14, and 15;
3) HCDR1, HCDR2, HCDR3 comprising sequences as set forth in SEQ ID NOs: 17, 18, and 19, or comprising sequences containing one or more amino acid substitutions (e.g., conservative substitutions), deletions, or insertions relative to SEQ ID NOs: 17, 18, and 19, respectively, or any combination thereof, or consisting of SEQ ID NOs: 17, 18, and 19; and/or LCDR1, LCDR2, LCDR3 comprising sequences as set forth in SEQ ID NOs: 21, 22, and 23, or comprising sequences containing one or more amino acid substitutions (e.g., conservative substitutions), deletions, or insertions relative to SEQ ID NOs: 21, 22, and 23, respectively, or any combination thereof, or consisting of SEQ ID NOs: 21, 22, and 23; or
4) HCDR1, HCDR2, HCDR3 comprising sequences as set forth in SEQ ID NOs: 25, 26, and 27, or comprising sequences containing one or more amino acid substitutions (e.g., conservative substitutions), deletions, or insertions relative to SEQ ID NOs: 25, 26, and 27, respectively, or any combination thereof, or consisting of SEQ ID NOs: 25, 26, and 27; and/or LCDR1, LCDR2, LCDR3 comprising sequences as set forth in SEQ ID NOs: 29, 30, and 31, or comprising sequences containing amino acid substitutions (e.g., conservative substitutions), deletions, or insertions of one or more and no more than 3 amino acids relative to SEQ ID NOs: 29, 30, and 31, respectively, or consisting of SEQ ID NOs: 29, 30, and 31.

In another embodiment, the present disclosure provides an anti-FOLR1 antibody and antigen-binding fragment thereof that specifically binds FOLR1, comprising a heavy chain variable region and/or a light chain variable region, wherein:
1) the heavy chain variable region comprises the amino acid sequence as set forth in SEQ ID NO: 4, or an amino acid sequence comprising one or more amino acid substitutions (e.g., conservative substitutions), deletions, or insertions, or any combination thereof, relative to SEQ ID NO: 4, or an amino acid sequence having at least 90%, 91%, 92%, 93%, 94%, 95%, 96%, 97%, 98%, or 99% identity to the amino acid sequence of SEQ ID NO: 4, or consists of SEQ ID NO: 4, and the light chain variable region comprises the amino acid sequence as set forth in SEQ ID NO: 8, or an amino acid sequence comprising one or more amino acid substitutions (e.g., conservative substitutions), deletions, or insertions, or any combination thereof, relative to SEQ ID NO: 8, or an amino acid sequence having at least 90%, 91%, 92%, 93%, 94%, 95%, 96%, 97%, 98%, or 99% identity to the amino acid sequence of SEQ ID NO: 8, or consists of SEQ ID NO: 8;
2) the heavy chain variable region comprises the amino acid sequence as set forth in SEQ ID NO: 12, or an amino acid sequence comprising one or more amino acid substitutions (e.g., conservative substitutions), deletions, or insertions, or any combination thereof, relative to SEQ ID NO: 12, or an amino acid sequence having at least 90%, 91%, 92%, 93%, 94%, 95%, 96%, 97%, 98%, or 99% identity to the amino acid sequence of SEQ ID NO: 12, or consists of SEQ ID NO: 12, and the light chain variable region comprises the amino acid sequence as set forth in SEQ ID NO: 16, or an amino acid sequence comprising one or more amino acid substitutions (e.g., conservative substitutions), deletions, or insertions, or any combination thereof, relative to SEQ ID NO: 16, or an amino acid sequence having at least 90%, 91%, 92%, 93%, 94%, 95%, 96%, 97%, 98%, or 99% identity to the amino acid sequence of SEQ ID NO: 16, or consists of SEQ ID NO: 16;
3) the heavy chain variable region comprises the amino acid sequence as set forth in SEQ ID NO: 20, or an amino acid sequence comprising one or more amino acid substitutions (e.g., conservative substitutions), deletions, or insertions, or any combination thereof, relative to SEQ ID NO: 20, or an amino acid sequence having at least 90%, 91%, 92%, 93%, 94%, 95%, 96%, 97%, 98%, or 99% identity to the amino acid sequence of SEQ ID NO: 20, or consists of SEQ ID NO: 20, and the light chain variable region comprises the amino acid sequence as set forth in SEQ ID NO: 24, or an amino acid sequence comprising one or more amino acid substitutions (e.g., conservative substitutions), deletions, or insertions, or any combination thereof, relative to SEQ ID NO: 24, or an amino acid sequence having at least 90%, 91%, 92%, 93%, 94%, 95%, 96%, 97%, 98%, or 99% identity to the amino acid sequence of SEQ ID NO: 24, or consists of SEQ ID NO: 24; or
4) the heavy chain variable region comprises the amino acid sequence as set forth in SEQ ID NO: 28, or an amino acid sequence comprising one or more amino acid substitutions (e.g., conservative substitutions), deletions, or insertions, or any combination thereof, relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consists of SEQ ID NO: 28, and the light chain variable region comprises the amino acid sequence as set forth in SEQ ID NO: 32, or an amino acid sequence comprising one or more amino acid substitutions (e.g., conservative substitutions), deletions, or insertions, or any combination thereof, relative to SEQ ID NO: 32, or an amino acid sequence having at least 90%, 91%, 92%, 93%, 94%, 95%, 96%, 97%, 98%, or 99% identity to the amino acid sequence of SEQ ID NO: 32, or consists of SEQ ID NO: 32;
5) the heavy chain variable region comprises the amino acid sequence as set forth in SEQ ID NO: 35, or an amino acid sequence comprising one or more amino acid substitutions (e.g., conservative substitutions), deletions, or insertions, or any combination thereof, relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consists of SEQ ID NO: 35, and the light chain variable region comprises the amino acid sequence as set forth in SEQ ID NO: 39, or an amino acid sequence comprising one or more amino acid substitutions (e.g., conservative substitutions), deletions, or insertions, or any combination thereof, relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consists of SEQ ID NO: 39;
6) the heavy chain variable region comprises the amino acid sequence as set forth in SEQ ID NO: 35, or an amino acid sequence comprising one or more amino acid substitutions (e.g., conservative substitutions), deletions, or insertions, or any combination thereof, relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consists of SEQ ID NO: 35, and the light chain variable region comprises the amino acid sequence as set forth in SEQ ID NO: 40, or an amino acid sequence comprising one or more amino acid substitutions (e.g., conservative substitutions), deletions, or insertions, or any combination thereof, relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consists of SEQ ID NO: 40;
7) the heavy chain variable region comprises the amino acid sequence as set forth in SEQ ID NO: 35, or an amino acid sequence comprising one or more amino acid substitutions (e.g., conservative substitutions), deletions, or insertions, or any combination thereof, relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consists of SEQ ID NO: 35, and the light chain variable region comprises the amino acid sequence as set forth in SEQ ID NO: 41, or an amino acid sequence comprising one or more amino acid substitutions (e.g., conservative substitutions), deletions, or insertions, or any combination thereof, relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consists of SEQ ID NO: 41;
8) the heavy chain variable region comprises the amino acid sequence as set forth in SEQ ID NO: 35, or an amino acid sequence comprising one or more amino acid substitutions (e.g., conservative substitutions), deletions, or insertions, or any combination thereof, relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consists of SEQ ID NO: 35, and the light chain variable region comprises the amino acid sequence as set forth in SEQ ID NO: 42, or an amino acid sequence comprising one or more amino acid substitutions (e.g., conservative substitutions), deletions, or insertions, or any combination thereof, relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consists of SEQ ID NO: 42;
9) the heavy chain variable region comprises the amino acid sequence as set forth in SEQ ID NO: 36, or an amino acid sequence comprising one or more amino acid substitutions (e.g., conservative substitutions), deletions, or insertions, or any combination thereof, relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consists of SEQ ID NO: 36, and the light chain variable region comprises the amino acid sequence as set forth in SEQ ID NO: 39, or an amino acid sequence comprising one or more amino acid substitutions (e.g., conservative substitutions), deletions, or insertions, or any combination thereof, relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consists of SEQ ID NO: 39;
10) the heavy chain variable region comprises the amino acid sequence as set forth in SEQ ID NO: 36, or an amino acid sequence comprising one or more amino acid substitutions (e.g., conservative substitutions), deletions, or insertions, or any combination thereof, relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consists of SEQ ID NO: 36, and the light chain variable region comprises the amino acid sequence as set forth in SEQ ID NO: 40, or an amino acid sequence comprising one or more amino acid substitutions (e.g., conservative substitutions), deletions, or insertions, or any combination thereof, relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consists of SEQ ID NO: 40;
11) the heavy chain variable region comprises the amino acid sequence as set forth in SEQ ID NO: 36, or an amino acid sequence comprising one or more amino acid substitutions (e.g., conservative substitutions), deletions, or insertions, or any combination thereof, relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consists of SEQ ID NO: 36, and the light chain variable region comprises the amino acid sequence as set forth in SEQ ID NO: 41, or an amino acid sequence comprising one or more amino acid substitutions (e.g., conservative substitutions), deletions, or insertions, or any combination thereof, relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consists of SEQ ID NO: 41;
12) the heavy chain variable region comprises the amino acid sequence as set forth in SEQ ID NO: 36, or an amino acid sequence comprising one or more amino acid substitutions (e.g., conservative substitutions), deletions, or insertions, or any combination thereof, relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consists of SEQ ID NO: 36, and the light chain variable region comprises the amino acid sequence as set forth in SEQ ID NO: 42, or an amino acid sequence comprising one or more amino acid substitutions (e.g., conservative substitutions), deletions, or insertions, or any combination thereof, relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consists of SEQ ID NO: 42;
13) the heavy chain variable region comprises the amino acid sequence as set forth in SEQ ID NO: 37, or an amino acid sequence comprising one or more amino acid substitutions (e.g., conservative substitutions), deletions, or insertions, or any combination thereof, relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consists of SEQ ID NO: 37, and the light chain variable region comprises the amino acid sequence as set forth in SEQ ID NO: 39, or an amino acid sequence comprising one or more amino acid substitutions (e.g., conservative substitutions), deletions, or insertions, or any combination thereof, relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consists of SEQ ID NO: 39;
14) the heavy chain variable region comprises the amino acid sequence as set forth in SEQ ID NO: 37, or an amino acid sequence comprising one or more amino acid substitutions (e.g., conservative substitutions), deletions, or insertions, or any combination thereof, relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consists of SEQ ID NO: 37, and the light chain variable region comprises the amino acid sequence as set forth in SEQ ID NO: 40, or an amino acid sequence comprising one or more amino acid substitutions (e.g., conservative substitutions), deletions, or insertions, or any combination thereof, relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consists of SEQ ID NO: 40;
15) the heavy chain variable region comprises the amino acid sequence as set forth in SEQ ID NO: 37, or an amino acid sequence comprising one or more amino acid substitutions (e.g., conservative substitutions), deletions, or insertions, or any combination thereof, relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consists of SEQ ID NO: 37, and the light chain variable region comprises the amino acid sequence as set forth in SEQ ID NO: 41, or an amino acid sequence comprising one or more amino acid substitutions (e.g., conservative substitutions), deletions, or insertions, or any combination thereof, relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consists of SEQ ID NO: 41;
16) the heavy chain variable region comprises the amino acid sequence as set forth in SEQ ID NO: 37, or an amino acid sequence comprising one or more amino acid substitutions (e.g., conservative substitutions), deletions, or insertions, or any combination thereof, relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consists of SEQ ID NO: 37, and the light chain variable region comprises the amino acid sequence as set forth in SEQ ID NO: 42, or an amino acid sequence comprising one or more amino acid substitutions (e.g., conservative substitutions), deletions, or insertions, or any combination thereof, relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consists of SEQ ID NO: 42;
17) the heavy chain variable region comprises the amino acid sequence as set forth in SEQ ID NO: 51, or an amino acid sequence comprising one or more amino acid substitutions (e.g., conservative substitutions), deletions, or insertions, or any combination thereof, relative to SEQ ID NO: 51, or an amino acid sequence having at least 90%, 91%, 92%, 93%, 94%, 95%, 96%, 97%, 98%, or 99% identity to the amino acid sequence of SEQ ID NO: 51, or consists of SEQ ID NO: 51, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
18) the heavy chain variable region comprises the amino acid sequence as set forth in SEQ ID NO: 51, or an amino acid sequence comprising one or more amino acid substitutions (e.g., conservative substitutions), deletions, or insertions, or any combination thereof, relative to SEQ ID NO: 51, or an amino acid sequence having at least 90%, 91%, 92%, 93%, 94%, 95%, 96%, 97%, 98%, or 99% identity to the amino acid sequence of SEQ ID NO: 51, or consists of SEQ ID NO: 51, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
19) the heavy chain variable region comprises the amino acid sequence as set forth in SEQ ID NO: 51, or an amino acid sequence comprising one or more amino acid substitutions (e.g., conservative substitutions), deletions, or insertions, or any combination thereof, relative to SEQ ID NO: 51, or an amino acid sequence having at least 90%, 91%, 92%, 93%, 94%, 95%, 96%, 97%, 98%, or 99% identity to the amino acid sequence of SEQ ID NO: 51, or consists of SEQ ID NO: 51, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
20) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
21) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
22) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
23) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
24) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
25) the heavy chain variable region comprises the amino acid sequence as set forth in SEQ ID NO: 52, or an amino acid sequence comprising one or more amino acid substitutions (e.g., conservative substitutions), deletions, or insertions, or any combination thereof, relative to SEQ ID NO: 52, or an amino acid sequence having at least 90%, 91%, 92%, 93%, 94%, 95%, 96%, 97%, 98%, or 99% identity to the amino acid sequence of SEQ ID NO: 52, or consists of SEQ ID NO: 52, and the light chain variable region comprises the amino acid sequence as set forth in SEQ ID NO: 60, or an amino acid sequence comprising one or more amino acid substitutions (e.g., conservative substitutions), deletions, or insertions, or any combination thereof, relative to SEQ ID NO: 60, or an amino acid sequence having at least 90%, 91%, 92%, 93%, 94%, 95%, 96%, 97%, 98%, or 99% identity to the amino acid sequence of SEQ ID NO: 60, or consists of SEQ ID NO: 60;
26) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
27) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
28) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
29) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
30) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
31) the heavy chain variable region comprises the amino acid sequence as set forth in SEQ ID NO: 53, or an amino acid sequence comprising one or more amino acid substitutions (e.g., conservative substitutions), deletions, or insertions, or any combination thereof, relative to SEQ ID NO: 53, or an amino acid sequence having at least 90%, 91%, 92%, 93%, 94%, 95%, 96%, 97%, 98%, or 99% identity to the amino acid sequence of SEQ ID NO: 53, or consists of SEQ ID NO: 53, and the light chain variable region comprises the amino acid sequence as set forth in SEQ ID NO: 60, or an amino acid sequence comprising one or more amino acid substitutions (e.g., conservative substitutions), deletions, or insertions, or any combination thereof, relative to SEQ ID NO: 60, or an amino acid sequence having at least 90%, 91%, 92%, 93%, 94%, 95%, 96%, 97%, 98%, or 99% identity to the amino acid sequence of SEQ ID NO: 60, or consists of SEQ ID NO: 60;
32) the heavy chain variable region comprises the amino acid sequence as set forth in SEQ ID NO: 54, or an amino acid sequence comprising one or more amino acid substitutions (e.g., conservative substitutions), deletions, or insertions, or any combination thereof, relative to SEQ ID NO: 54, or an amino acid sequence having at least 90%, 91%, 92%, 93%, 94%, 95%, 96%, 97%, 98%, or 99% identity to the amino acid sequence of SEQ ID NO: 54, or consists of SEQ ID NO: 54, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
33) the heavy chain variable region comprises the amino acid sequence as set forth in SEQ ID NO: 54, or an amino acid sequence comprising one or more amino acid substitutions (e.g., conservative substitutions), deletions, or insertions, or any combination thereof, relative to SEQ ID NO: 54, or an amino acid sequence having at least 90%, 91%, 92%, 93%, 94%, 95%, 96%, 97%, 98%, or 99% identity to the amino acid sequence of SEQ ID NO: 54, or consists of SEQ ID NO: 54, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
34) the heavy chain variable region comprises the amino acid sequence as set forth in SEQ ID NO: 54, or an amino acid sequence comprising one or more amino acid substitutions (e.g., conservative substitutions), deletions, or insertions, or any combination thereof, relative to SEQ ID NO: 54, or an amino acid sequence having at least 90%, 91%, 92%, 93%, 94%, 95%, 96%, 97%, 98%, or 99% identity to the amino acid sequence of SEQ ID NO: 54, or consists of SEQ ID NO: 54, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
35) the heavy chain variable region comprises the amino acid sequence as set forth in SEQ ID NO: 54, or an amino acid sequence comprising one or more amino acid substitutions (e.g., conservative substitutions), deletions, or insertions, or any combination thereof, relative to SEQ ID NO: 54, or an amino acid sequence having at least 90%, 91%, 92%, 93%, 94%, 95%, 96%, 97%, 98%, or 99% identity to the amino acid sequence of SEQ ID NO: 54, or consists of SEQ ID NO: 54, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
36) the heavy chain variable region comprises the amino acid sequence as set forth in SEQ ID NO: 54, or an amino acid sequence comprising one or more amino acid substitutions (e.g., conservative substitutions), deletions, or insertions, or any combination thereof, relative to SEQ ID NO: 54, or an amino acid sequence having at least 90%, 91%, 92%, 93%, 94%, 95%, 96%, 97%, 98%, or 99% identity to the amino acid sequence of SEQ ID NO: 54, or consists of SEQ ID NO: 54, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
37) the heavy chain variable region comprises the amino acid sequence as set forth in SEQ ID NO: 61, or an amino acid sequence comprising one or more amino acid substitutions (e.g., conservative substitutions), deletions, or insertions, or any combination thereof, relative to SEQ ID NO: 61, or an amino acid sequence having at least 90%, 91%, 92%, 93%, 94%, 95%, 96%, 97%, 98%, or 99% identity to the amino acid sequence of SEQ ID NO: 61, or consists of SEQ ID NO: 61, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
38) the heavy chain variable region comprises the amino acid sequence as set forth in SEQ ID NO: 61, or an amino acid sequence comprising one or more amino acid substitutions (e.g., conservative substitutions), deletions, or insertions, or any combination thereof, relative to SEQ ID NO: 61, or an amino acid sequence having at least 90%, 91%, 92%, 93%, 94%, 95%, 96%, 97%, 98%, or 99% identity to the amino acid sequence of SEQ ID NO: 61, or consists of SEQ ID NO: 61, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
39) the heavy chain variable region comprises the amino acid sequence as set forth in SEQ ID NO: 61, or an amino acid sequence comprising one or more amino acid substitutions (e.g., conservative substitutions), deletions, or insertions, or any combination thereof, relative to SEQ ID NO: 61, or an amino acid sequence having at least 90%, 91%, 92%, 93%, 94%, 95%, 96%, 97%, 98%, or 99% identity to the amino acid sequence of SEQ ID NO: 61, or consists of SEQ ID NO: 61, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
40) the heavy chain variable region comprises the amino acid sequence as set forth in SEQ ID NO: 61, or an amino acid sequence comprising one or more amino acid substitutions (e.g., conservative substitutions), deletions, or insertions, or any combination thereof, relative to SEQ ID NO: 61, or an amino acid sequence having at least 90%, 91%, 92%, 93%, 94%, 95%, 96%, 97%, 98%, or 99% identity to the amino acid sequence of SEQ ID NO: 61, or consists of SEQ ID NO: 61, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
41) the heavy chain variable region comprises the amino acid sequence as set forth in SEQ ID NO: 61, or an amino acid sequence comprising one or more amino acid substitutions (e.g., conservative substitutions), deletions, or insertions, or any combination thereof, relative to SEQ ID NO: 61, or an amino acid sequence having at least 90%, 91%, 92%, 93%, 94%, 95%, 96%, 97%, 98%, or 99% identity to the amino acid sequence of SEQ ID NO: 61, or consists of SEQ ID NO: 61, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
42) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
43) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
44) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
45) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
46) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
47) the heavy chain variable region comprises the amino acid sequence as set forth in SEQ ID NO: 62, or an amino acid sequence comprising one or more amino acid substitutions (e.g., conservative substitutions), deletions, or insertions, or any combination thereof, relative to SEQ ID NO: 62, or an amino acid sequence having at least 90%, 91%, 92%, 93%, 94%, 95%, 96%, 97%, 98%, or 99% identity to the amino acid sequence of SEQ ID NO: 62, or consists of SEQ ID NO: 62, and the light chain variable region comprises the amino acid sequence as set forth in SEQ ID NO: 60, or an amino acid sequence comprising one or more amino acid substitutions (e.g., conservative substitutions), deletions, or insertions, or any combination thereof, relative to SEQ ID NO: 60, or an amino acid sequence having at least 90%, 91%, 92%, 93%, 94%, 95%, 96%, 97%, 98%, or 99% identity to the amino acid sequence of SEQ ID NO: 60, or consists of SEQ ID NO: 60;
48) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
49) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
50) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
51) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
52) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
53) the heavy chain variable region comprises the amino acid sequence as set forth in SEQ ID NO: 63, or an amino acid sequence comprising one or more amino acid substitutions (e.g., conservative substitutions), deletions, or insertions, or any combination thereof, relative to SEQ ID NO: 63, or an amino acid sequence having at least 90%, 91%, 92%, 93%, 94%, 95%, 96%, 97%, 98%, or 99% identity to the amino acid sequence of SEQ ID NO: 63, or consists of SEQ ID NO: 63, and the light chain variable region comprises the amino acid sequence as set forth in SEQ ID NO: 60, or an amino acid sequence comprising one or more amino acid substitutions (e.g., conservative substitutions), deletions, or insertions, or any combination thereof, relative to SEQ ID NO: 60, or an amino acid sequence having at least 90%, 91%, 92%, 93%, 94%, 95%, 96%, 97%, 98%, or 99% identity to the amino acid sequence of SEQ ID NO: 60, or consists of SEQ ID NO: 60;
54) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 55, or an amino acid sequence comprising one or more amino acid substitutions (e.g., conservative substitutions), deletions, or insertions, or any combination thereof, relative to SEQ ID NO: 55, or an amino acid sequence having at least 90%, 91%, 92%, 93%, 94%, 95%, 96%, 97%, 98%, or 99% identity to the amino acid sequence of SEQ ID NO: 55, or consists of SEQ ID NO: 55;
55) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 56, or an amino acid sequence comprising one or more amino acid substitutions (e.g., conservative substitutions), deletions, or insertions, or any combination thereof, relative to SEQ ID NO: 56, or an amino acid sequence having at least 90%, 91%, 92%, 93%, 94%, 95%, 96%, 97%, 98%, or 99% identity to the amino acid sequence of SEQ ID NO: 56, or consists of SEQ ID NO: 56;
56) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 57, or an amino acid sequence comprising one or more amino acid substitutions (e.g., conservative substitutions), deletions, or insertions, or any combination thereof, relative to SEQ ID NO: 57, or an amino acid sequence having at least 90%, 91%, 92%, 93%, 94%, 95%, 96%, 97%, 98%, or 99% identity to the amino acid sequence of SEQ ID NO: 57, or consists of SEQ ID NO: 57;
57) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 58, or an amino acid sequence comprising one or more amino acid substitutions (e.g., conservative substitutions), deletions, or insertions, or any combination thereof, relative to SEQ ID NO: 58, or an amino acid sequence having at least 90%, 91%, 92%, 93%, 94%, 95%, 96%, 97%, 98%, or 99% identity to the amino acid sequence of SEQ ID NO: 58, or consists of SEQ ID NO: 58;
58) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 59, or an amino acid sequence comprising one or more amino acid substitutions (e.g., conservative substitutions), deletions, or insertions, or any combination thereof, relative to SEQ ID NO: 59, or an amino acid sequence having at least 90%, 91%, 92%, 93%, 94%, 95%, 96%, 97%, 98%, or 99% identity to the amino acid sequence of SEQ ID NO: 59, or consists of SEQ ID NO: 59;
59) the heavy chain variable region comprises the amino acid sequence as set forth in SEQ ID NO: 64, or an amino acid sequence comprising one or more amino acid substitutions (e.g., conservative substitutions), deletions, or insertions, or any combination thereof, relative to SEQ ID NO: 64, or an amino acid sequence having at least 90%, 91%, 92%, 93%, 94%, 95%, 96%, 97%, 98%, or 99% identity to the amino acid sequence of SEQ ID NO: 64, or consists of SEQ ID NO: 64, and the light chain variable region comprises the amino acid sequence as set forth in SEQ ID NO: 60, or an amino acid sequence comprising one or more amino acid substitutions (e.g., conservative substitutions), deletions, or insertions, or any combination thereof, relative to SEQ ID NO: 60, or an amino acid sequence having at least 90%, 91%, 92%, 93%, 94%, 95%, 96%, 97%, 98%, or 99% identity to the amino acid sequence of SEQ ID NO: 60, or consists of SEQ ID NO: 60;
60) the heavy chain variable region comprises the amino acid sequence as set forth in SEQ ID NO: 43, or an amino acid sequence comprising one or more amino acid substitutions (e.g., conservative substitutions), deletions, or insertions, or any combination thereof, relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consists of SEQ ID NO: 43, and the light chain variable region comprises the amino acid sequence as set forth in SEQ ID NO: 47, or an amino acid sequence comprising one or more amino acid substitutions (e.g., conservative substitutions), deletions, or insertions, or any combination thereof, relative to SEQ ID NO: 47, or an amino acid sequence having at least 90%, 91%, 92%, 93%, 94%, 95%, 96%, 97%, 98%, or 99% identity to the amino acid sequence of SEQ ID NO: 47, or consists of SEQ ID NO: 47;
61) the heavy chain variable region comprises the amino acid sequence as set forth in SEQ ID NO: 43, or an amino acid sequence comprising one or more amino acid substitutions (e.g., conservative substitutions), deletions, or insertions, or any combination thereof, relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consists of SEQ ID NO: 43, and the light chain variable region comprises the amino acid sequence as set forth in SEQ ID NO: 48, or an amino acid sequence comprising one or more amino acid substitutions (e.g., conservative substitutions), deletions, or insertions, or any combination thereof, relative to SEQ ID NO: 48, or an amino acid sequence having at least 90%, 91%, 92%, 93%, 94%, 95%, 96%, 97%, 98%, or 99% identity to the amino acid sequence of SEQ ID NO: 48, or consists of SEQ ID NO: 48;
62) the heavy chain variable region comprises the amino acid sequence as set forth in SEQ ID NO: 43, or an amino acid sequence comprising one or more amino acid substitutions (e.g., conservative substitutions), deletions, or insertions, or any combination thereof, relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consists of SEQ ID NO: 43, and the light chain variable region comprises the amino acid sequence as set forth in SEQ ID NO: 49, or an amino acid sequence comprising one or more amino acid substitutions (e.g., conservative substitutions), deletions, or insertions, or any combination thereof, relative to SEQ ID NO: 49, or an amino acid sequence having at least 90%, 91%, 92%, 93%, 94%, 95%, 96%, 97%, 98%, or 99% identity to the amino acid sequence of SEQ ID NO: 49, or consists of SEQ ID NO: 49;
63) the heavy chain variable region comprises the amino acid sequence as set forth in SEQ ID NO: 43, or an amino acid sequence comprising one or more amino acid substitutions (e.g., conservative substitutions), deletions, or insertions, or any combination thereof, relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consists of SEQ ID NO: 43, and the light chain variable region comprises the amino acid sequence as set forth in SEQ ID NO: 50, or an amino acid sequence comprising one or more amino acid substitutions (e.g., conservative substitutions), deletions, or insertions, or any combination thereof, relative to SEQ ID NO: 50, or an amino acid sequence having at least 90%, 91%, 92%, 93%, 94%, 95%, 96%, 97%, 98%, or 99% identity to the amino acid sequence of SEQ ID NO: 50, or consists of SEQ ID NO: 50;
64) the heavy chain variable region comprises the amino acid sequence as set forth in SEQ ID NO: 44, or an amino acid sequence comprising one or more amino acid substitutions (e.g., conservative substitutions), deletions, or insertions, or any combination thereof, relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consists of SEQ ID NO: 44, and the light chain variable region comprises the amino acid sequence as set forth in SEQ ID NO: 47, or an amino acid sequence comprising one or more amino acid substitutions (e.g., conservative substitutions), deletions, or insertions, or any combination thereof, relative to SEQ ID NO: 47, or an amino acid sequence having at least 90%, 91%, 92%, 93%, 94%, 95%, 96%, 97%, 98%, or 99% identity to the amino acid sequence of SEQ ID NO: 47, or consists of SEQ ID NO: 47;
65) the heavy chain variable region comprises the amino acid sequence as set forth in SEQ ID NO: 44, or an amino acid sequence comprising one or more amino acid substitutions (e.g., conservative substitutions), deletions, or insertions, or any combination thereof, relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consists of SEQ ID NO: 44, and the light chain variable region comprises the amino acid sequence as set forth in SEQ ID NO: 48, or an amino acid sequence comprising one or more amino acid substitutions (e.g., conservative substitutions), deletions, or insertions, or any combination thereof, relative to SEQ ID NO: 48, or an amino acid sequence having at least 90%, 91%, 92%, 93%, 94%, 95%, 96%, 97%, 98%, or 99% identity to the amino acid sequence of SEQ ID NO: 48, or consists of SEQ ID NO: 48;
66) the heavy chain variable region comprises the amino acid sequence as set forth in SEQ ID NO: 44, or an amino acid sequence comprising one or more amino acid substitutions (e.g., conservative substitutions), deletions, or insertions, or any combination thereof, relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consists of SEQ ID NO: 44, and the light chain variable region comprises the amino acid sequence as set forth in SEQ ID NO: 49, or an amino acid sequence comprising one or more amino acid substitutions (e.g., conservative substitutions), deletions, or insertions, or any combination thereof, relative to SEQ ID NO: 49, or an amino acid sequence having at least 90%, 91%, 92%, 93%, 94%, 95%, 96%, 97%, 98%, or 99% identity to the amino acid sequence of SEQ ID NO: 49, or consists of SEQ ID NO: 49;
67) the heavy chain variable region comprises the amino acid sequence as set forth in SEQ ID NO: 44, or an amino acid sequence comprising one or more amino acid substitutions (e.g., conservative substitutions), deletions, or insertions, or any combination thereof, relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consists of SEQ ID NO: 44, and the light chain variable region comprises the amino acid sequence as set forth in SEQ ID NO: 50, or an amino acid sequence comprising one or more amino acid substitutions (e.g., conservative substitutions), deletions, or insertions, or any combination thereof, relative to SEQ ID NO: 50, or an amino acid sequence having at least 90%, 91%, 92%, 93%, 94%, 95%, 96%, 97%, 98%, or 99% identity to the amino acid sequence of SEQ ID NO: 50, or consists of SEQ ID NO: 50;
68) the heavy chain variable region comprises the amino acid sequence as set forth in SEQ ID NO: 45, or an amino acid sequence comprising one or more amino acid substitutions (e.g., conservative substitutions), deletions, or insertions, or any combination thereof, relative to SEQ ID NO: 45, or an amino acid sequence having at least 90%, 91%, 92%, 93%, 94%, 95%, 96%, 97%, 98%, or 99% identity to the amino acid sequence of SEQ ID NO: 45, or consists of SEQ ID NO: 45, and the light chain variable region comprises the amino acid sequence as set forth in SEQ ID NO: 47, or an amino acid sequence comprising one or more amino acid substitutions (e.g., conservative substitutions), deletions, or insertions, or any combination thereof, relative to SEQ ID NO: 47, or an amino acid sequence having at least 90%, 91%, 92%, 93%, 94%, 95%, 96%, 97%, 98%, or 99% identity to the amino acid sequence of SEQ ID NO: 47, or consists of SEQ ID NO: 47;
69) the heavy chain variable region comprises the amino acid sequence as set forth in SEQ ID NO: 45, or an amino acid sequence comprising one or more amino acid substitutions (e.g., conservative substitutions), deletions, or insertions, or any combination thereof, relative to SEQ ID NO: 45, or an amino acid sequence having at least 90%, 91%, 92%, 93%, 94%, 95%, 96%, 97%, 98%, or 99% identity to the amino acid sequence of SEQ ID NO: 45, or consists of SEQ ID NO: 45, and the light chain variable region comprises the amino acid sequence as set forth in SEQ ID NO: 48, or an amino acid sequence comprising one or more amino acid substitutions (e.g., conservative substitutions), deletions, or insertions, or any combination thereof, relative to SEQ ID NO: 48, or an amino acid sequence having at least 90%, 91%, 92%, 93%, 94%, 95%, 96%, 97%, 98%, or 99% identity to the amino acid sequence of SEQ ID NO: 48, or consists of SEQ ID NO: 48;
70) the heavy chain variable region comprises the amino acid sequence as set forth in SEQ ID NO: 45, or an amino acid sequence comprising one or more amino acid substitutions (e.g., conservative substitutions), deletions, or insertions, or any combination thereof, relative to SEQ ID NO: 45, or an amino acid sequence having at least 90%, 91%, 92%, 93%, 94%, 95%, 96%, 97%, 98%, or 99% identity to the amino acid sequence of SEQ ID NO: 45, or consists of SEQ ID NO: 45, and the light chain variable region comprises the amino acid sequence as set forth in SEQ ID NO: 49, or an amino acid sequence comprising one or more amino acid substitutions (e.g., conservative substitutions), deletions, or insertions, or any combination thereof, relative to SEQ ID NO: 49, or an amino acid sequence having at least 90%, 91%, 92%, 93%, 94%, 95%, 96%, 97%, 98%, or 99% identity to the amino acid sequence of SEQ ID NO: 49, or consists of SEQ ID NO: 49;
71) the heavy chain variable region comprises the amino acid sequence as set forth in SEQ ID NO: 45, or an amino acid sequence comprising one or more amino acid substitutions (e.g., conservative substitutions), deletions, or insertions, or any combination thereof, relative to SEQ ID NO: 45, or an amino acid sequence having at least 90%, 91%, 92%, 93%, 94%, 95%, 96%, 97%, 98%, or 99% identity to the amino acid sequence of SEQ ID NO: 45, or consists of SEQ ID NO: 45, and the light chain variable region comprises the amino acid sequence as set forth in SEQ ID NO: 50, or an amino acid sequence comprising one or more amino acid substitutions (e.g., conservative substitutions), deletions, or insertions, or any combination thereof, relative to SEQ ID NO: 50, or an amino acid sequence having at least 90%, 91%, 92%, 93%, 94%, 95%, 96%, 97%, 98%, or 99% identity to the amino acid sequence of SEQ ID NO: 50, or consists of SEQ ID NO: 50;
72) the heavy chain variable region comprises the amino acid sequence as set forth in SEQ ID NO: 46, or an amino acid sequence comprising one or more amino acid substitutions (e.g., conservative substitutions), deletions, or insertions, or any combination thereof, relative to SEQ ID NO: 46, or an amino acid sequence having at least 90%, 91%, 92%, 93%, 94%, 95%, 96%, 97%, 98%, or 99% identity to the amino acid sequence of SEQ ID NO: 46, or consists of SEQ ID NO: 46, and the light chain variable region comprises the amino acid sequence as set forth in SEQ ID NO: 47, or an amino acid sequence comprising one or more amino acid substitutions (e.g., conservative substitutions), deletions, or insertions, or any combination thereof, relative to SEQ ID NO: 47, or an amino acid sequence having at least 90%, 91%, 92%, 93%, 94%, 95%, 96%, 97%, 98%, or 99% identity to the amino acid sequence of SEQ ID NO: 47, or consists of SEQ ID NO: 47;
73) the heavy chain variable region comprises the amino acid sequence as set forth in SEQ ID NO: 46, or an amino acid sequence comprising one or more amino acid substitutions (e.g., conservative substitutions), deletions, or insertions, or any combination thereof, relative to SEQ ID NO: 46, or an amino acid sequence having at least 90%, 91%, 92%, 93%, 94%, 95%, 96%, 97%, 98%, or 99% identity to the amino acid sequence of SEQ ID NO: 46, or consists of SEQ ID NO: 46, and the light chain variable region comprises the amino acid sequence as set forth in SEQ ID NO: 48, or an amino acid sequence comprising one or more amino acid substitutions (e.g., conservative substitutions), deletions, or insertions, or any combination thereof, relative to SEQ ID NO: 48, or an amino acid sequence having at least 90%, 91%, 92%, 93%, 94%, 95%, 96%, 97%, 98%, or 99% identity to the amino acid sequence of SEQ ID NO: 48, or consists of SEQ ID NO: 48;
74) the heavy chain variable region comprises the amino acid sequence as set forth in SEQ ID NO: 46, or an amino acid sequence comprising one or more amino acid substitutions (e.g., conservative substitutions), deletions, or insertions, or any combination thereof, relative to SEQ ID NO: 46, or an amino acid sequence having at least 90%, 91%, 92%, 93%, 94%, 95%, 96%, 97%, 98%, or 99% identity to the amino acid sequence of SEQ ID NO: 46, or consists of SEQ ID NO: 46, and the light chain variable region comprises the amino acid sequence as set forth in SEQ ID NO: 49, or an amino acid sequence comprising one or more amino acid substitutions (e.g., conservative substitutions), deletions, or insertions, or any combination thereof, relative to SEQ ID NO: 49, or an amino acid sequence having at least 90%, 91%, 92%, 93%, 94%, 95%, 96%, 97%, 98%, or 99% identity to the amino acid sequence of SEQ ID NO: 49, or consists of SEQ ID NO: 49; or
75) the heavy chain variable region comprises the amino acid sequence as set forth in SEQ ID NO: 46, or an amino acid sequence comprising one or more amino acid substitutions (e.g., conservative substitutions), deletions, or insertions, or any combination thereof, relative to SEQ ID NO: 46, or an amino acid sequence having at least 90%, 91%, 92%, 93%, 94%, 95%, 96%, 97%, 98%, or 99% identity to the amino acid sequence of SEQ ID NO: 46, or consists of SEQ ID NO: 46, and the light chain variable region comprises the amino acid sequence as set forth in SEQ ID NO: 50, or an amino acid sequence comprising one or more amino acid substitutions (e.g., conservative substitutions), deletions, or insertions, or any combination thereof, relative to SEQ ID NO: 50, or an amino acid sequence having at least 90%, 91%, 92%, 93%, 94%, 95%, 96%, 97%, 98%, or 99% identity to the amino acid sequence of SEQ ID NO: 50, or consists of SEQ ID NO: 50.

In some embodiments, the above antibody or antigen-binding fragment thereof further comprises heavy chain and/or light chain constant region sequences from a human antibody germline consensus sequence. The light chain constant region is preferably a human κ or λ chain constant region. The heavy chain constant region may be a γ, µ, α, δ, or ε chain. In some embodiments, the heavy chain constant region is preferably from a constant region sequence of human IgG1, IgG2, IgG3, or IgG4. In one embodiment, the light chain constant region comprises the sequence as set forth in SEQ ID NO: 34, or consists of said sequence. In another embodiment, the heavy chain constant region comprises the sequence as set forth in SEQ ID NO: 33.

It should be understood that sequence variants of these constant region domains may also be used, for example comprising one or more amino acid modifications, wherein amino acid positions are identified according to the EU index system of Kabat et al. (1991).

In a specific embodiment, the present disclosure provides an anti-FOLR1 antibody and antigen-binding fragment thereof that specifically binds FOLR1, comprising a heavy chain and/or a light chain, wherein:
1) the heavy chain comprises the amino acid sequence as set forth in SEQ ID NO: 65, or an amino acid sequence comprising one or more amino acid substitutions (e.g., conservative substitutions), deletions, or insertions, or any combination thereof, relative to SEQ ID NO: 65, or an amino acid sequence having at least 90%, 91%, 92%, 93%, 94%, 95%, 96%, 97%, 98%, or 99% identity to the amino acid sequence of SEQ ID NO: 65, or consists of SEQ ID NO: 65, and the light chain comprises the amino acid sequence as set forth in SEQ ID NO: 66, or an amino acid sequence comprising one or more amino acid substitutions (e.g., conservative substitutions), deletions, or insertions, or any combination thereof, relative to SEQ ID NO: 66, or an amino acid sequence having at least 90%, 91%, 92%, 93%, 94%, 95%, 96%, 97%, 98%, or 99% identity to the amino acid sequence of SEQ ID NO: 66, or consists of SEQ ID NO: 66;
2) the heavy chain comprises the amino acid sequence as set forth in SEQ ID NO: 67, or an amino acid sequence comprising one or more amino acid substitutions (e.g., conservative substitutions), deletions, or insertions, or any combination thereof, relative to SEQ ID NO: 67, or an amino acid sequence having at least 90%, 91%, 92%, 93%, 94%, 95%, 96%, 97%, 98%, or 99% identity to the amino acid sequence of SEQ ID NO: 67, or consists of SEQ ID NO: 67, and the light chain comprises the amino acid sequence as set forth in SEQ ID NO: 68, or an amino acid sequence comprising one or more amino acid substitutions (e.g., conservative substitutions), deletions, or insertions, or any combination thereof, relative to SEQ ID NO: 68, or an amino acid sequence having at least 90%, 91%, 92%, 93%, 94%, 95%, 96%, 97%, 98%, or 99% identity to the amino acid sequence of SEQ ID NO: 68, or consists of SEQ ID NO: 68;
3) the heavy chain comprises the amino acid sequence as set forth in SEQ ID NO: 69, or an amino acid sequence comprising one or more amino acid substitutions (e.g., conservative substitutions), deletions, or insertions, or any combination thereof, relative to SEQ ID NO: 69, or an amino acid sequence having at least 90%, 91%, 92%, 93%, 94%, 95%, 96%, 97%, 98%, or 99% identity to the amino acid sequence of SEQ ID NO: 69, or consists of SEQ ID NO: 69, and the light chain comprises the amino acid sequence as set forth in SEQ ID NO: 70, or an amino acid sequence comprising one or more amino acid substitutions (e.g., conservative substitutions), deletions, or insertions, or any combination thereof, relative to SEQ ID NO: 70, or an amino acid sequence having at least 90%, 91%, 92%, 93%, 94%, 95%, 96%, 97%, 98%, or 99% identity to the amino acid sequence of SEQ ID NO: 70, or consists of SEQ ID NO: 70;
4) the heavy chain comprises the amino acid sequence as set forth in SEQ ID NO: 71, or an amino acid sequence comprising one or more amino acid substitutions (e.g., conservative substitutions), deletions, or insertions, or any combination thereof, relative to SEQ ID NO: 71, or an amino acid sequence having at least 90%, 91%, 92%, 93%, 94%, 95%, 96%, 97%, 98%, or 99% identity to the amino acid sequence of SEQ ID NO: 71, or consists of SEQ ID NO: 71, and the light chain comprises the amino acid sequence as set forth in SEQ ID NO: 72, or an amino acid sequence comprising one or more amino acid substitutions (e.g., conservative substitutions), deletions, or insertions, or any combination thereof, relative to SEQ ID NO: 72, or an amino acid sequence having at least 90%, 91%, 92%, 93%, 94%, 95%, 96%, 97%, 98%, or 99% identity to the amino acid sequence of SEQ ID NO: 72, or consists of SEQ ID NO: 72;
5) the heavy chain comprises the amino acid sequence as set forth in SEQ ID NO: 73, or an amino acid sequence comprising one or more amino acid substitutions (e.g., conservative substitutions), deletions, or insertions, or any combination thereof, relative to SEQ ID NO: 73, or an amino acid sequence having at least 90%, 91%, 92%, 93%, 94%, 95%, 96%, 97%, 98%, or 99% identity to the amino acid sequence of SEQ ID NO: 73, or consists of SEQ ID NO: 73, and the light chain comprises the amino acid sequence as set forth in SEQ ID NO: 74, or an amino acid sequence comprising one or more amino acid substitutions (e.g., conservative substitutions), deletions, or insertions, or any combination thereof, relative to SEQ ID NO: 74, or an amino acid sequence having at least 90%, 91%, 92%, 93%, 94%, 95%, 96%, 97%, 98%, or 99% identity to the amino acid sequence of SEQ ID NO: 74, or consists of SEQ ID NO: 74;
6) the heavy chain comprises the amino acid sequence as set forth in SEQ ID NO: 75, or an amino acid sequence comprising one or more amino acid substitutions (e.g., conservative substitutions), deletions, or insertions, or any combination thereof, relative to SEQ ID NO: 75, or an amino acid sequence having at least 90%, 91%, 92%, 93%, 94%, 95%, 96%, 97%, 98%, or 99% identity to the amino acid sequence of SEQ ID NO: 75, or consists of SEQ ID NO: 75, and the light chain comprises the amino acid sequence as set forth in SEQ ID NO: 76, or an amino acid sequence comprising one or more amino acid substitutions (e.g., conservative substitutions), deletions, or insertions, or any combination thereof, relative to SEQ ID NO: 76, or an amino acid sequence having at least 90%, 91%, 92%, 93%, 94%, 95%, 96%, 97%, 98%, or 99% identity to the amino acid sequence of SEQ ID NO: 76, or consists of SEQ ID NO: 76;
7) the heavy chain comprises the amino acid sequence as set forth in SEQ ID NO: 77, or an amino acid sequence comprising one or more amino acid substitutions (e.g., conservative substitutions), deletions, or insertions, or any combination thereof, relative to SEQ ID NO: 77, or an amino acid sequence having at least 90%, 91%, 92%, 93%, 94%, 95%, 96%, 97%, 98%, or 99% identity to the amino acid sequence of SEQ ID NO: 77, or consists of SEQ ID NO: 77, and the light chain comprises the amino acid sequence as set forth in SEQ ID NO: 78, or an amino acid sequence comprising one or more amino acid substitutions (e.g., conservative substitutions), deletions, or insertions, or any combination thereof, relative to SEQ ID NO: 78, or an amino acid sequence having at least 90%, 91%, 92%, 93%, 94%, 95%, 96%, 97%, 98%, or 99% identity to the amino acid sequence of SEQ ID NO: 78, or consists of SEQ ID NO: 78;
8) the heavy chain comprises the amino acid sequence as set forth in SEQ ID NO: 79, or an amino acid sequence comprising one or more amino acid substitutions (e.g., conservative substitutions), deletions, or insertions, or any combination thereof, relative to SEQ ID NO: 79, or an amino acid sequence having at least 90%, 91%, 92%, 93%, 94%, 95%, 96%, 97%, 98%, or 99% identity to the amino acid sequence of SEQ ID NO: 79, or consists of SEQ ID NO: 79, and the light chain comprises the amino acid sequence as set forth in SEQ ID NO: 80, or an amino acid sequence comprising one or more amino acid substitutions (e.g., conservative substitutions), deletions, or insertions, or any combination thereof, relative to SEQ ID NO: 80, or an amino acid sequence having at least 90%, 91%, 92%, 93%, 94%, 95%, 96%, 97%, 98%, or 99% identity to the amino acid sequence of SEQ ID NO: 80, or consists of SEQ ID NO: 80;
9) the heavy chain comprises the amino acid sequence as set forth in SEQ ID NO: 81, or an amino acid sequence comprising one or more amino acid substitutions (e.g., conservative substitutions), deletions, or insertions, or any combination thereof, relative to SEQ ID NO: 81, or an amino acid sequence having at least 90%, 91%, 92%, 93%, 94%, 95%, 96%, 97%, 98%, or 99% identity to the amino acid sequence of SEQ ID NO: 81, or consists of SEQ ID NO: 81, and the light chain comprises the amino acid sequence as set forth in SEQ ID NO: 82, or an amino acid sequence comprising one or more amino acid substitutions (e.g., conservative substitutions), deletions, or insertions, or any combination thereof, relative to SEQ ID NO: 82, or an amino acid sequence having at least 90%, 91%, 92%, 93%, 94%, 95%, 96%, 97%, 98%, or 99% identity to the amino acid sequence of SEQ ID NO: 82, or consists of SEQ ID NO: 82;
10) the heavy chain comprises the amino acid sequence as set forth in SEQ ID NO: 83, or an amino acid sequence comprising one or more amino acid substitutions (e.g., conservative substitutions), deletions, or insertions, or any combination thereof, relative to SEQ ID NO: 83, or an amino acid sequence having at least 90%, 91%, 92%, 93%, 94%, 95%, 96%, 97%, 98%, or 99% identity to the amino acid sequence of SEQ ID NO: 83, or consists of SEQ ID NO: 83, and the light chain comprises the amino acid sequence as set forth in SEQ ID NO: 84, or an amino acid sequence comprising one or more amino acid substitutions (e.g., conservative substitutions), deletions, or insertions, or any combination thereof, relative to SEQ ID NO: 84, or an amino acid sequence having at least 90%, 91%, 92%, 93%, 94%, 95%, 96%, 97%, 98%, or 99% identity to the amino acid sequence of SEQ ID NO: 84, or consists of SEQ ID NO: 84;
11) the heavy chain comprises the amino acid sequence as set forth in SEQ ID NO: 85, or an amino acid sequence comprising one or more amino acid substitutions (e.g., conservative substitutions), deletions, or insertions, or any combination thereof, relative to SEQ ID NO: 85, or an amino acid sequence having at least 90%, 91%, 92%, 93%, 94%, 95%, 96%, 97%, 98%, or 99% identity to the amino acid sequence of SEQ ID NO: 85, or consists of SEQ ID NO: 85, and the light chain comprises the amino acid sequence as set forth in SEQ ID NO: 86, or an amino acid sequence comprising one or more amino acid substitutions (e.g., conservative substitutions), deletions, or insertions, or any combination thereof, relative to SEQ ID NO: 86, or an amino acid sequence having at least 90%, 91%, 92%, 93%, 94%, 95%, 96%, 97%, 98%, or 99% identity to the amino acid sequence of SEQ ID NO: 86, or consists of SEQ ID NO: 86;
12) the heavy chain comprises the amino acid sequence as set forth in SEQ ID NO: 87, or an amino acid sequence comprising one or more amino acid substitutions (e.g., conservative substitutions), deletions, or insertions, or any combination thereof, relative to SEQ ID NO: 87, or an amino acid sequence having at least 90%, 91%, 92%, 93%, 94%, 95%, 96%, 97%, 98%, or 99% identity to the amino acid sequence of SEQ ID NO: 87, or consists of SEQ ID NO: 87, and the light chain comprises the amino acid sequence as set forth in SEQ ID NO: 88, or an amino acid sequence comprising one or more amino acid substitutions (e.g., conservative substitutions), deletions, or insertions, or any combination thereof, relative to SEQ ID NO: 88, or an amino acid sequence having at least 90%, 91%, 92%, 93%, 94%, 95%, 96%, 97%, 98%, or 99% identity to the amino acid sequence of SEQ ID NO: 88, or consists of SEQ ID NO: 88;
13) the heavy chain comprises the amino acid sequence as set forth in SEQ ID NO: 89, or an amino acid sequence comprising one or more amino acid substitutions (e.g., conservative substitutions), deletions, or insertions, or any combination thereof, relative to SEQ ID NO: 89, or an amino acid sequence having at least 90%, 91%, 92%, 93%, 94%, 95%, 96%, 97%, 98%, or 99% identity to the amino acid sequence of SEQ ID NO: 89, or consists of SEQ ID NO: 89, and the light chain comprises the amino acid sequence as set forth in SEQ ID NO: 90, or an amino acid sequence comprising one or more amino acid substitutions (e.g., conservative substitutions), deletions, or insertions, or any combination thereof, relative to SEQ ID NO: 90, or an amino acid sequence having at least 90%, 91%, 92%, 93%, 94%, 95%, 96%, 97%, 98%, or 99% identity to the amino acid sequence of SEQ ID NO: 90, or consists of SEQ ID NO: 90; or
14) the heavy chain comprises the amino acid sequence as set forth in SEQ ID NO: 91, or an amino acid sequence comprising one or more amino acid substitutions (e.g., conservative substitutions), deletions, or insertions, or any combination thereof, relative to SEQ ID NO: 91, or an amino acid sequence having at least 90%, 91%, 92%, 93%, 94%, 95%, 96%, 97%, 98%, or 99% identity to the amino acid sequence of SEQ ID NO: 91, or consists of SEQ ID NO: 91, and the light chain comprises the amino acid sequence as set forth in SEQ ID NO: 92, or an amino acid sequence comprising one or more amino acid substitutions (e.g., conservative substitutions), deletions, or insertions, or any combination thereof, relative to SEQ ID NO: 92, or an amino acid sequence having at least 90%, 91%, 92%, 93%, 94%, 95%, 96%, 97%, 98%, or 99% identity to the amino acid sequence of SEQ ID NO: 92, or consists of SEQ ID NO: 92.

In certain embodiments of the antibody of any of the foregoing, the antibody is monoclonal.

In certain embodiments of the antibody of any of the foregoing, the antibody is a full-length antibody.

In certain embodiments of the antibody of any of the foregoing, the antibody is a chimeric antibody or a humanized antibody.

In one embodiment, the anti-FOLR1 antibody of the present disclosure is an intact antibody, such as an IgG1, IgG2, IgG3, or IgG4 antibody. In another embodiment, the anti-FOLR1 antibody of the present disclosure encompasses only antigen-binding portions thereof, for example: Fab, Fab'-SH, Fv, scFv, or (Fab')₂ fragments.

In a second aspect, the present disclosure provides antibody-drug conjugates targeting human FOLR1, wherein the antibody-drug conjugates have the following advantages:
(1) binding to target cells expressing human FOLR1 with high affinity thereto;
(2) being capable of entering cells through endocytosis to kill target cells; in some embodiments, the ADCs of the present disclosure have high internalization efficiency;
(3) treating or ameliorating conditions associated with abnormal function or expression of FOLR1 in a subject (e.g., cancers such as ovarian cancer, triple-negative breast cancer, endometrial cancer, peritoneal cancer, and lung cancer), or treating or ameliorating one or more symptoms of said diseases;
(4) reducing or inhibiting tumor growth or progression in a subject having a tumor expressing FOLR1;
(5) exerting cytotoxic activity in cells expressing FOLR1.

In one embodiment, the present application provides a conjugate comprising an anti-FOLR1 antibody as described in the first aspect. In a specific embodiment, molecules that can be conjugated to the anti-FOLR1 antibody include, for example, cytotoxic agents, immunomodulators, imaging agents, fluorescent proteins, molecular markers, therapeutic proteins, biopolymers, and oligonucleotides, and the like.

In one embodiment, the present disclosure provides an antibody-drug conjugate (ADC) comprising the anti-FOLR1 antibody or antigen-binding fragment thereof as described in the first aspect and at least one therapeutically active substance or pharmaceutically active ingredient, having the structure Ab-(L-D)n, wherein: Ab is an antibody or antigen-binding fragment thereof that binds FOLR1 as described in the first aspect of the present disclosure; L is a linker; D is a therapeutically active substance or pharmaceutically active ingredient; and n represents 1-8, for example 1, 2, 3, 4, 5, ....

In one embodiment of the present disclosure, n represents the number of drug-linker (L-D) moieties conjugated to a single antibody (Ab), and is preferably an integer from 1 to 8. In this case, the individual ADC conjugate may also be referred to as an ADC compound. In any embodiment herein, there may be 1, 2, 3, 4, 5, 6, 7, or 8 drug-linker moieties conjugated to a single antibody on an ADC compound according to the present disclosure.

In another embodiment of the present disclosure, n represents the average DAR of the prepared conjugate composition. In this case, n may be, for example, an integer or decimal within the range of 1 to 8, 1.0 to about 8.0, 1.0 to about 7.0, or 1.0 to about 6.6, 2.0 to about 8.0, 2.0 to about 7.5, 2.0 to 6.5, or 2.0 to about 6.0, for example 3.0-4.0, 4.0-5.0, 7.0-8.0. In some aspects, n represents an average DAR of about 3. In some aspects, n represents an average DAR of about 4. In some aspects, n represents an average DAR of about 5. In some aspects, n represents an average DAR of about 6. In some aspects, n represents an average DAR of about 7. In some aspects, n represents an average DAR of about 8.

IgG1 antibodies have 16 pairs of cysteine residues, which exist in the form of 12 intra-chain and 4 inter-chain disulfide bonds. The inter-chain disulfide bonds are solvent-accessible and can be reduced by reducing agents to form eight thiol groups, which then become conjugation targets. In one embodiment of the present disclosure, the linker is conjugated to the thiol group of cysteine on the anti-FOLR1 antibody.

In a specific embodiment, the therapeutically active substance or pharmaceutically active ingredient is a cytotoxin, plant toxin, small molecule toxin, radioactive isotope, maytansinoid, or the like. In a specific embodiment, the cytotoxin is dolastatin and auristatin derivatives thereof, such as monomethyl auristatin E (MMAE). In another specific embodiment, the cytotoxin is a topoisomerase I inhibitor, such as a camptothecin derivative. In a preferred embodiment, the cytotoxin is DXD. In another embodiment, the therapeutically active substance or pharmaceutically active ingredient is MMAE or DXD.

In one embodiment, the cytotoxin is covalently linked to the anti-FOLR1 antibody or antigen-binding fragment thereof directly or by means of a linker in a non-site-specific manner or a site-specific manner.

In one embodiment, the linker is a protease-cleavable peptide linker. In a specific embodiment, the peptide linker comprises a protease-cleavable peptide fragment, such as glycine-glycine-phenylalanine-glycine (GGFG). In a specific embodiment, the peptide linker may also comprise, for example, a maleimide conjugated to a cysteine residue of the antibody.

In one embodiment, the linker L and the active substance D constitute Deruxtecan.

In a specific embodiment, the antibody-drug conjugate of the present disclosure has the following structure: 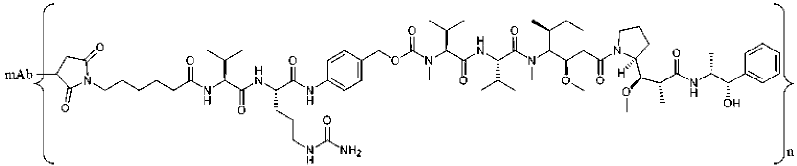 or

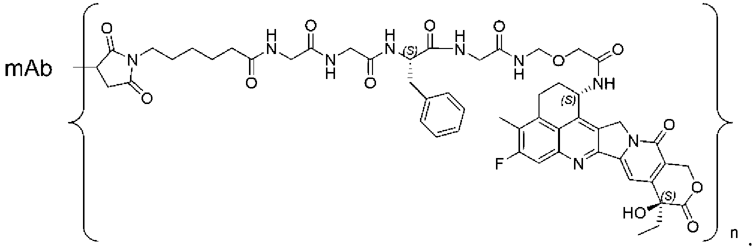

In a third aspect, the present disclosure provides an isolated polynucleotide molecule encoding any of the antibodies or antigen-binding fragments thereof as described in the first aspect.

In a fourth aspect, the present disclosure provides a vector comprising the nucleic acid molecule of the third aspect. In one embodiment, the vector is an expression vector.

In a fifth aspect, the present disclosure provides a host cell comprising the vector of the fourth aspect or the nucleic acid molecule of the third aspect. In some embodiments, the host cell is prokaryotic, such as Escherichia coli. In other embodiments, the host cell is eukaryotic, such as 293 cells, CHO cells, or yeast cells.

In a sixth aspect, the present disclosure provides a pharmaceutical composition comprising the antibody or antigen-binding fragment thereof of the present disclosure, or the antibody-drug conjugate of the present disclosure, polynucleotide molecule, vector, host cell, and a pharmaceutically acceptable carrier.

In a seventh aspect, the present disclosure provides a method for preparing the anti-FOLR1 antibody or antigen-binding fragment thereof described herein, the method comprising expressing the antibody or antigen-binding fragment thereof in a host cell described herein under conditions suitable for expression of the antibody or antigen-binding fragment thereof, and recovering the expressed antibody or antigen-binding fragment thereof from the host cell.

In an eighth aspect, the present disclosure provides a method of treating a disease associated with abnormal expression of FOLR1 in a subject in need thereof, comprising administering to the subject a therapeutically effective amount of the antibody or antigen-binding fragment thereof of the present disclosure, the antibody-drug conjugate of the present disclosure, the polynucleotide molecule, the vector, the host cell, and the pharmaceutical composition of the present disclosure.

In one embodiment, the disease associated with abnormal expression of FOLR1 is a cancer with high expression of FOLR1, such as ovarian cancer, triple-negative breast cancer, endometrial cancer, peritoneal cancer, and lung cancer.

In some embodiments, the antibody or antigen-binding fragment thereof of the present disclosure, the antibody-drug conjugate of the present disclosure, the polynucleotide molecule, the vector, the host cell, and the pharmaceutical composition of the present disclosure can also be administered in combination with one or more other therapies, such as treatment modalities and/or other therapeutic agents, for uses described herein, for example, for treating the related diseases or conditions mentioned herein.

In a specific embodiment, the present disclosure provides a method of killing cells expressing FOLR1 or inhibiting the growth of cells expressing FOLR1, comprising contacting the cells with an effective amount of the antibody or antigen-binding fragment thereof of the present disclosure, the antibody-drug conjugate of the present disclosure, the polynucleotide molecule, the vector, the host cell, and the pharmaceutical composition of the present disclosure.

In a ninth aspect, the present disclosure provides a use of the anti-FOLR1 antibody or antigen-binding fragment thereof in the preparation of an antibody-drug conjugate for treating cancer.

In a tenth aspect, the present disclosure provides a use of the anti-FOLR1 antibody or antigen-binding fragment thereof of the present disclosure, the antibody-drug conjugate, the vector, the host cell, or the pharmaceutical composition of the present disclosure in the preparation of a medicament for treating cancer.

In some embodiments, the cancer is a cancer with high expression of FOLR1, such as ovarian cancer, triple-negative breast cancer, endometrial cancer, peritoneal cancer, and lung cancer.

### BRIEF DESCRIPTION OF THE DRAWINGS

Figure 1 shows a schematic structural diagram of the conjugate of an anti-FOLR1 chimeric antibody with MMAE.
Figure 2 shows the killing activity of the anti-FOLR1 chimeric antibody MMAE conjugate against KB cells.
Figure 3 shows the non-specific binding activity and the binding activity to mouse FOLR1 of anti-FOLR1 humanized antibodies. Figure 3A shows binding curves of anti-FOLR1 humanized antibodies to FOLR2; Figure 3B shows binding curves of anti-FOLR1 humanized antibodies to FOLR3; Figure 3C shows binding curves of anti-FOLR1 humanized antibodies to FOLR4; Figure 3D shows binding curves of anti-FOLR1 humanized antibodies to mouse FOLR1.
Figure 4 shows the results of blocking experiments of anti-FOLR1 humanized antibodies on the binding of FA-BSA to FOLR1.
Figure 5 shows the cell killing activity of anti-FOLR1 humanized antibody MMAE conjugates. Figures A-C show the killing curves of anti-FOLR1 humanized antibody MMAE conjugates against KB cells;
Figures D-F show the killing curves of anti-FOLR1 humanized antibody MMAE conjugates against SK-OV-3 cells.
Figure 6 shows a schematic structural diagram of the conjugate of an anti-FOLR1 humanized antibody with DXD.
Figure 7 shows that ADCs prepared by conjugating anti-FOLR1 humanized antibodies with DXD inhibit the growth of human oral epidermoid carcinoma cells. Figure 7A shows that anti-FOLR1 humanized antibody DXD conjugates inhibit KB tumor growth. Figure 7B shows that treatment with anti-FOLR1 humanized antibody DXD conjugates does not affect changes in mouse body weight.
Figure 8 shows that ADCs prepared by conjugating anti-FOLR1 humanized antibodies with DXD inhibit the growth of human lung adenocarcinoma cells. Figure 8A shows that ADCs of anti-FOLR1 humanized antibodies inhibit tumor growth. Figure 8B shows that treatment with ADCs of anti-FOLR1 humanized antibodies does not affect changes in mouse body weight.

### DETAILED DESCRIPTION OF THE INVENTION

All publications, patent applications, patents, and other references mentioned herein are incorporated by reference in their entirety. In addition, the materials, methods, and examples described herein are illustrative only and are not intended to be limiting. Other features, objects, and advantages of the present disclosure will be apparent from this specification and the drawings, as well as from the appended claims. Unless otherwise defined, all technical and scientific terms used herein have the same meaning as commonly understood by one of ordinary skill in the art. For the purposes of the present disclosure, the following terms are defined below.

### I. Definitions

The term "about" when used in conjunction with a numerical value is meant to encompass numerical values within a range having a lower limit that is 5% less than the indicated numerical value and an upper limit that is 5% greater than the indicated numerical value.

The term "and/or" should be understood to mean any one of the alternatives or a combination of any two or more of the alternatives.

As used herein, the term "comprising" or "including" means including the stated elements, integers, or steps, but not excluding any other elements, integers, or steps. When the term "comprising" or "including" is used herein, unless otherwise indicated, the situation consisting of the stated elements, integers, or steps is also encompassed.

The term "antibody" is used herein in the broadest sense and refers to a protein comprising an antigen-binding site.

The term "immunoglobulin" refers to a protein having the structure of a naturally occurring antibody, and in the present application can generally be used interchangeably with the term "antibody". IgG-class immunoglobulins are heterotetrameric glycoproteins composed of two light chains and two heavy chains linked by disulfide bonds. From N-terminus to C-terminus, each immunoglobulin heavy chain has one heavy chain variable region (VH), also called the heavy chain variable domain, followed by three heavy chain constant domains (CH1, CH2, and CH3). Similarly, from N-terminus to C-terminus, each immunoglobulin light chain has one light chain variable region (VL), also called the light chain variable domain, followed by one light chain constant domain (CL). In an IgG molecule, typically the VH-CH1 of the heavy chain pairs with the VL-CL of the light chain to form a Fab fragment that specifically binds antigen. Thus, an IgG immunoglobulin essentially consists of two Fab molecules connected by an immunoglobulin hinge region and two dimerized Fc regions. The heavy chains of immunoglobulins can be classified into one of five classes based on the type of their constant regions, designated α (IgA), δ (IgD), ε (IgE), γ (IgG), or µ (IgM), some of which can be further divided into subclasses, such as γ1 (IgG1), γ2 (IgG2), γ3 (IgG3), γ4 (IgG4), α1 (IgA1), and α2 (IgA2). The light chains of immunoglobulins can also be classified into one of two types based on the amino acid sequence of their constant domains, designated κ and λ.

The term "antibody fragment" refers to a molecule different from an intact antibody, which comprises a portion of an intact antibody and is capable of binding the antigen to which the intact antibody binds. Examples of antibody fragments include, but are not limited to, Fv, Fab, Fab', Fab'-SH, F(ab')₂; diabodies; linear antibodies; single-chain antibodies (e.g., scFv); single-domain antibodies; bivalent or bispecific antibodies or fragments thereof; camelid antibodies (heavy chain antibodies); and multispecific antibodies formed from antibody fragments.

The terms "antigen-binding site" and "antigen-binding domain" are used interchangeably and refer to the region of an antibody molecule that actually binds to the antigen. Antigen-binding sites include, but are not limited to, forms such as Fv, Fab fragment, Fab', Fab'-SH, F(ab')₂, single-chain antibody molecules (e.g., scFv), VHH, and the like.

The term "variable region" or "variable domain" of an antibody refers to the domain of the antibody heavy or light chain that is involved in binding of the antibody to the antigen. The variable region of an antibody can be further subdivided into hypervariable regions (i.e., complementarity determining regions (CDRs)) and more conserved regions interspersed between the hypervariable regions (i.e., framework regions (FRs)). In the case of IgG-class immunoglobulins, the heavy chain variable region or light chain variable region from N-terminus to C-terminus respectively comprises FR1-CDR1-FR2-CDR2-FR3-CDR3-FR4. In the case of heavy chain antibodies (also referred to herein as nanobodies), such as those from camelid heavy chain antibodies, the antigen-binding site consists of a single VH domain (i.e., a "VHH" domain). The VHH of a natural heavy chain antibody has a similar structure to the heavy chain variable region of a natural IgG antibody, i.e., it comprises four conserved framework regions (FRs) and three complementarity determining regions (CDRs).

"Complementarity determining region" or "CDR region" or "CDR" or "hypervariable region" refers to a region of an antibody variable domain that is hypervariable in sequence and forms structurally defined loops ("hypervariable loops") and/or contains antigen-contacting residues ("antigen contacts"). CDRs are primarily responsible for binding to antigen epitopes. CDRs in a variable domain are generally referred to as CDR1, CDR2, and CDR3, numbered sequentially starting from the N-terminus.

Various schemes for determining CDR sequences within a given VH or VL amino acid sequence are well known in the art: Kabat complementarity determining regions (CDRs) are determined based on sequence variability and are the most commonly used (Kabat et al., Sequences of Proteins of Immunological Interest, 5th ed., Public Health Service, National Institutes of Health, Bethesda, Md. (1991)), while Chothia refers to the location of structural loops (Chothia et al., (1987) J. Mol. Biol. 196:901-917; Chothia et al. (1989) Nature 342:877-883), AbM CDRs are a compromise between Kabat CDRs and Chothia structural loops and are used by Oxford Molecular's AbM antibody modeling software, and "Contact" CDRs are based on analysis of available complex crystal structures. According to the different CDR determination schemes, the residues of each of these CDRs are as described below.

| CDR | Kabat Scheme | AbM Scheme | Chothia Scheme | Contact Scheme |
|---|---|---|---|---|
| CDR1 | L24-L34 | L24-L34 | L26-L32 | L30-L36 |
| CDR2 | L50-L56 | L50-L56 | L50-L52 | L46-L55 |
| CDR3 | L89-L97 | L89-L97 | L91-L96 | L89-L96 |
| CDR1 | H31-H35B | H26-H35B | H26-H32 | H30-H35B |
| (Kabat num bering system) | | | | |
| CDR1 | H31-H35 | H26-H35 | H26-H32 | H30-H35 |
| (Chothia numbering system) | | | | |
| CDR2 | H50-H65 | H50-H58 | H53-H55 | H47-H58 |
| CDR3 | H95-H102 | H95-H102 | H96-H101 | H93-H101 |
| (Kabat numbering system) | | | | |

When defining an antibody using specific CDR sequences defined by the present disclosure, the scope of the antibody also encompasses antibodies whose variable region sequences contain the stated specific CDR sequences, but whose claimed CDR boundaries differ from the specific CDR boundaries defined by the present disclosure due to the application of different schemes (e.g., different assignment scheme rules or combinations).

The CDRs of the antibodies of the present disclosure can be manually assessed and boundaries determined according to any scheme in the art or combinations thereof. Unless otherwise stated, in the present disclosure, the term "CDR" or "CDR sequence" encompasses CDR sequences determined in any of the above-described manners.

"Heavy chain constant region domain" or "heavy chain constant region" refers to the constant region domain derived from or obtained from or derived from the immunoglobulin heavy chain, including the heavy chain constant regions CH1, CH2, CH3, and optionally the heavy chain constant region CH4, covalently connected in order from N-terminus to C-terminus. In most cases, the heavy chain constant regions CH1 and CH2 are connected through a heavy chain hinge region, but where appropriate, may also be connected through a flexible linker.

The term "EC₅₀", also known as the "half maximal effective concentration", refers to the concentration of a drug, antibody, or toxicant that induces a response halfway between the baseline and the maximum after a specified exposure time.

As used herein, the term "binding" or "specific binding" means that the binding is selective for the antigen and can be distinguished from unwanted or non-specific interactions. The ability of an antigen-binding site to bind a particular antigen can be determined by enzyme-linked immunosorbent assay (ELISA) or conventional binding assays known in the art.

"Affinity" or "binding affinity" refers to the intrinsic binding capability reflecting the interaction between members of a binding pair. The affinity of molecule X for its binding partner Y can be represented by the equilibrium dissociation constant (K_{D}), which is the ratio of the dissociation rate constant to the association rate constant (k_{dis} and kₒₙ, respectively). Binding affinity can be measured by common methods known in the art.

Amino acid mutations can be amino acid substitutions, deletions, insertions, and/or additions. In some embodiments, amino acid mutations are substitutions of one or more amino acids, such as single amino acid substitutions or combinations of multiple amino acid substitutions. Amino acid deletions and insertions include deletions and insertions at the amino and/or carboxyl termini of polypeptide sequences, as well as internal deletions and insertions within polypeptide sequences. The amino acid substitutions of the present disclosure optionally include conservative substitutions of amino acids.

"Percent (%) identity" of an amino acid sequence refers to the percentage of amino acid residues in a candidate sequence that are identical to the amino acid residues of a specific amino acid sequence set forth in this specification, after aligning the candidate sequence with the specific amino acid sequence set forth in this specification and introducing gaps, if necessary, to achieve maximum percent sequence identity, and not considering any conservative substitutions as part of the sequence identity. In some embodiments, the present disclosure contemplates variants of the antibody molecules of the present disclosure that have a considerable degree of identity relative to the antibody molecules and sequences thereof specifically disclosed herein, for example, an identity of at least 80%, 85%, 90%, 95%, 97%, 98%, or 99% or higher. Such variants may contain conservative modifications.

For polypeptide sequences, "conservative modifications" include substitutions, deletions, or additions to the polypeptide sequence that result in the substitution of an amino acid with a chemically similar amino acid. Conservative substitution tables providing functionally similar amino acids are well known in the art. Such conservatively modified variants are additional to and do not exclude the polymorphic variants, interspecies homologs, and alleles of the present disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In some embodiments, the term "conservative sequence modifications" is used to refer to amino acid modifications that do not significantly affect or alter the binding characteristics of an antibody containing the amino acid sequence.

The term "therapeutic agent" as used herein encompasses any substance effective in the prevention or treatment of tumors, such as cancer, including chemotherapeutic agents, cytokines, angiogenesis inhibitors, cytotoxic agents, other antibodies, small molecule drugs, or immunomodulators (e.g., immunosuppressants).

The term "antibody-drug conjugate" or "ADC" refers to an antibody or antibody fragment covalently conjugated to a therapeutically active substance or active pharmaceutical ingredient, such that the therapeutically active substance or active pharmaceutical ingredient is targeted to the binding target of the antibody to exert its pharmacological function. The therapeutically active substance or active pharmaceutical ingredient can be a cytotoxin capable of killing cells targeted by the ADC (preferably cancer cells). Covalent attachment of the therapeutically active substance, active pharmaceutical ingredient, or cytotoxin can be performed in a non-site-specific manner using a linker, or in a site-specific manner.

The term "site-specific conjugation" refers to a connection method in which a therapeutically active substance or active pharmaceutical ingredient is specifically attached to a specific site of an antibody. In one embodiment, the conjugation is accomplished by means of a linker.

The term "cytotoxic agent" can be used interchangeably with "cytotoxin" and refers in the present disclosure to a substance that inhibits or disrupts cell function and/or causes cell death or destruction. In one embodiment, cytotoxic agents can include, but are not limited to, bacterial toxins, plant toxins, small molecule toxins, radioactive isotopes, and the like.

Any antibody-drug conjugate of the present disclosure can be prepared by conjugating dolastatin and its auristatin derivatives with antibodies. Dolastatin and its auristatin derivatives are important cytotoxins used in antibody-drug conjugates (ADCs), which interfere with microtubule dynamics, cell division, and the like, and have anti-tumor and anti-fungal activities. In one aspect, dolastatin and its auristatin derivatives can be, for example, monomethyl auristatin D (MMAD), monomethyl auristatin E (MMAE), monomethyl auristatin F (MMAF), and other auristatins (such as auristatins described in US No. 20130129753), and the like.

Monomethyl auristatin E (MMAE), i.e., desmethyl-auristatin E, is a well-known member of the auristatin compound family, with the following structural formula:

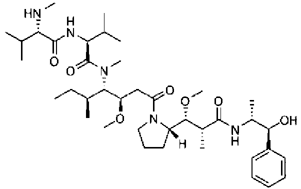

MMAE is conjugated to a monoclonal antibody through a linker to form an ADC. In general, the linker is cleaved after the ADC enters tumor cells, thereby releasing MMAE to exert its cytotoxic effect and kill tumor cells.

DXD, as a derivative of Exatecan, is an effective DNA topoisomerase I inhibitor and is now widely used in the ADCs, where it is connected to cysteine residues of the antibody via a maleimide moiety through a cleavable tetrapeptide linker (GGFG). Currently, Deruxtecan, composed of maleimide-GGFG-DXD, is commercially available as an ADC drug linker and has the following structure:

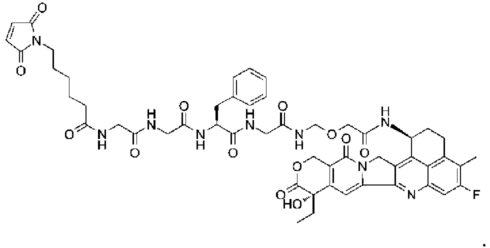

The terms "linker" and "connector" are used interchangeably in the present application and refer to a chemical moiety that covalently links an antibody to a therapeutically active substance or active pharmaceutical ingredient in an ADC. In one embodiment, the linker can include amino acid residues that connect the antigen to the payload. The amino acid residues can form dipeptide, tripeptide, tetrapeptide, pentapeptide, hexapeptide, heptapeptide, octapeptide, nonapeptide, decapeptide, undecapeptide, or dodecapeptide units. Amino acid residues include naturally occurring ones as well as non-naturally occurring amino acid analogs, such as citrulline or β-amino acids, such as β-alanine, or ω-amino acids such as 4-amino-butyric acid.

Classified by properties, linkers suitable for the present disclosure can be protease-degradable linkers, non-cleavable linkers, acid-sensitive linkers, silicone-structured linkers, disulfide-carbamate linkers, MC-GGFG linkers, TRX linkers, galactoside-containing linkers, pyrophosphate linkers, near-infrared sensitive linkers, ultraviolet-sensitive linkers, and the like.

The linkers of the present disclosure can also be a combination of one or more linkers; for example, a cathepsin-degradable linker can be combined with other types of linkers to form a new linker. Therefore, the "linker" described in the present disclosure encompasses a single type of linker, or a combination of different types of linkers, as long as it is capable of conjugating the antibody of the present disclosure with the drug.

The term "payload" or "drug load" or "effective payload" refers to the average number of effective payloads per antibody within an ADC molecule (where "effective payload" is used interchangeably with "therapeutically active substance or active pharmaceutical ingredient" herein). The drug load can range from 1 to 20 therapeutically active substances or active pharmaceutical ingredients per antibody.

The term "drug-to-antibody ratio" or "DAR" refers to the ratio of therapeutically active substance or active pharmaceutical ingredient (D) conjugated to the antibody to the antibody. ADCs described herein generally have a DAR of 1-8, and in certain specific embodiments have a DAR of 1-8, 2-8, 2-6, 2-5, 3-8, 4-6, and 2-4. Representative DAR values are, for example, 1, 2, 3, 4, 5, 6, 7, 8, typically expressed as a combination of the letter D or DAR and a number, where the number indicates the DAR value, for example, D2/DAR2 indicates a drug-to-antibody ratio with a DAR value of 2. In some embodiments, the DAR is an average DAR, i.e., the overall ratio of small molecule drug moieties (D) conjugated to the Ab moieties described herein to the Ab moieties in the product as determined by detection methods (e.g., by conventional methods such as UV/visible spectroscopy, mass spectrometry, ELISA assay, electrophoresis, and/or HPLC).

DAR may be limited by the number of attachment sites of the antibody. For example, in the case where the attachment sites are cysteine thiols, the antibody may have only one or a few cysteine thiol groups or may have only one or a few thiol groups with sufficient reactivity (through which the linking unit can be connected).

In some embodiments, the average DAR value of the conjugate of the present disclosure is 0 to 8, for example, 3.0-4.0, 4.0-5.0, 7.0-8.0, for example, a range with two of these values as endpoints.

The terms "individual" or "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the individual is a human.

The term "treatment" refers to clinical intervention intended to alter the natural course of a disease in an individual being treated. Desired therapeutic effects include, but are not limited to, preventing the occurrence or recurrence of disease, alleviating symptoms, diminishing any direct or indirect pathological consequences of the disease, preventing metastasis, reducing the rate of disease progression, ameliorating or palliating the disease state, and remission or improvement of prognosis. In some embodiments, the antibody molecules of the present disclosure are used to delay disease development or to slow the progression of the disease.

The term "effective amount" refers to such an amount or dosage of an antibody or composition of the present disclosure that produces a desired effect in a patient in need of treatment or prevention when administered in single or multiple doses. An effective amount can be readily determined by a treating physician, as one skilled in the art, by considering various factors such as: species of mammal; body weight, age, and general health; the specific disease involved; degree or severity of the disease; the individual patient's response; the specific antibody administered; the mode of administration; the bioavailability characteristics of the formulation administered; the dosing regimen selected; and the use of any concomitant therapy.

The term "therapeutically effective amount" refers to an amount effective to achieve a desired therapeutic result at a required dosage and for a required period of time. A therapeutically effective amount of an antibody or antibody fragment or conjugate or composition thereof can vary depending on various factors such as the disease state, the age, sex, and weight of the individual, and the ability of the antibody or antibody portion to elicit a desired response in the individual. A therapeutically effective amount is also one in which any toxic or detrimental effects of the antibody or antibody fragment or conjugate or composition thereof are outweighed by the therapeutically beneficial effects. A "therapeutically effective amount" preferably inhibits a measurable parameter (e.g., tumor growth rate, tumor volume, etc.) by at least about 20%, more preferably by at least about 40%, even more preferably by at least about 50%, 60%, or 70%, and still more preferably by at least about 80% or 90%, relative to an untreated subject. The ability of a compound to inhibit a measurable parameter (e.g., cancer) can be evaluated in animal model systems predictive of efficacy in human tumors.

The term "pharmaceutical composition" refers to a composition that is present in a form that allows the biological activity of the active ingredient contained therein to be effective, and that does not contain additional components that are unacceptably toxic to a subject to whom the composition is administered.

### II. Compositions of the Present Disclosure

In some embodiments, the present disclosure provides compositions comprising any of the anti-FOLR1 antibodies described herein, or ADC molecules thereof, preferably the compositions being pharmaceutical compositions. In one embodiment, the composition further comprises pharmaceutical excipients, such as pharmaceutically acceptable carriers and pharmaceutically acceptable excipients known in the art. In one embodiment, the composition (e.g., pharmaceutical composition) comprises an anti-FOLR1 antibody of the present disclosure, or an ADC molecule thereof, in combination with one or more other therapeutic agents.

As used herein, "pharmaceutically acceptable carrier" includes any and all physiologically compatible solvents, dispersion media, isotonic agents, and absorption delaying agents, and the like.

For the use of pharmaceutical excipients and their uses, see also "Handbook of Pharmaceutical Excipients," 8th edition, R.C. Rowe, P.J. Seskey, and S.C. Owen, Pharmaceutical Press, London, Chicago.

The compositions of the present disclosure can be in a variety of forms. These forms include, for example, liquid, semi-solid, and solid dosage forms, such as powders or suspensions, liquid solutions (e.g., injectable solutions and infusible solutions), liposomal formulations, and suppositories. The preferred form depends on the intended mode of administration and therapeutic application.

The routes of administration of the compositions of the present disclosure are according to known methods, for example, oral, intravenous, intraperitoneal, intracerebral, intramuscular, intraocular, intra-arterial, or intralesional routes; by sustained release systems or by implanted devices. In certain embodiments, the compositions can be administered by bolus injection or by continuous infusion or by implanted devices.

The subject can be a mammal, for example, a primate, for example, a human (e.g., an individual having or at risk of having a disease described herein). In one embodiment, the subject has a disease (e.g., cancer) described herein or is at risk of having a disease described herein. In certain embodiments, the subject has received or has previously received other treatments, such as chemotherapy and/or radiation therapy. In some embodiments, the subject has previously received or is receiving immunotherapy.

Pharmaceuticals comprising the antibodies described herein can be prepared by mixing the anti-FOLR1 antibody of the present disclosure, or an ADC molecule thereof, having the desired purity with one or more optional pharmaceutical excipients, preferably in the form of a lyophilized formulation or aqueous solution.

The pharmaceutical compositions or formulations of the present disclosure may also include more than one active ingredient required for the particular indication being treated, preferably those active ingredients that have complementary activities that do not adversely affect each other. For example, it is desirable to additionally provide other therapeutic agents, including chemotherapeutic agents, angiogenesis inhibitors, cytokines, cytotoxic agents, other antibodies, small molecule drugs, or immunomodulators (e.g., immune checkpoint inhibitors or agonists), and the like. The active ingredients are suitably combined and present in amounts effective for the intended purpose.

Sustained release formulations can be prepared. Suitable examples of sustained release formulations include semi-permeable matrices of solid hydrophobic polymers containing the antibody, the matrices being in the form of shaped articles, such as films or microcapsules.

### III. Preparation of Antibodies and Antibody-Drug Conjugates of the Present Disclosure

In one embodiment, the present disclosure provides a method for preparing an anti-FOLR1 antibody, wherein the method comprises culturing a host cell comprising a nucleic acid encoding the anti-FOLR1 antibody or an expression vector comprising the nucleic acid under conditions suitable for expression of the nucleic acid encoding the anti-FOLR1 antibody, and optionally isolating the anti-FOLR1 antibody. In a certain embodiment, the method further comprises recovering the anti-FOLR1 antibody from the host cell (or host cell culture medium).

For recombinant production of the anti-FOLR1 antibody of the present disclosure, a nucleic acid encoding the anti-FOLR1 antibody of the present disclosure is first isolated and the nucleic acid is inserted into a vector for further cloning and/or expression in a host cell. Such nucleic acids are readily isolated and sequenced using conventional procedures, for example, by using oligonucleotide probes that can specifically bind to nucleic acids encoding the anti-FOLR1 antibody of the present disclosure.

The anti-FOLR1 antibody of the present disclosure prepared as described herein can be purified by known prior art techniques such as high performance liquid chromatography, ion exchange chromatography, gel electrophoresis, affinity chromatography, size exclusion chromatography, and the like. The actual conditions used to purify a particular protein also depend on factors such as net charge, hydrophobicity, hydrophilicity, and the like, and these will be apparent to those skilled in the art. The purity of the anti-FOLR1 antibody of the present disclosure can be determined by any of a variety of well-known analytical methods, including size exclusion chromatography, gel electrophoresis, high performance liquid chromatography, and the like.

The generation of antibody-drug conjugates can be accomplished by any technique known to those skilled in the art. In some aspects, conjugation of the drug-linker to the antibody is accomplished by reaction with amino acid residues of the antibody. In some embodiments, a heteroaryl linker L bearing a leaving group is applied to conjugate drug D to cysteine residues of the antibody to prepare the conjugate of Formula I of the present disclosure. In some embodiments, the interchain disulfide bonds of the antibody can be disrupted and free thiols exposed for conjugation with the heteroaryl linker-drug by controlling the conditions under which the antibody is treated with a reducing agent such as tris(2-carboxyethyl)phosphine (TCEP). For IgG1-type antibodies, up to four interchain disulfide bonds can be reduced, thereby generating up to 8 reactive thiol groups for conjugation. Conjugates prepared by this method can contain zero, one, two, three, four, five, six, seven, or eight drugs per antibody molecule.

When the prepared conjugate is a composition of conjugates having different drug conjugation sites and/or numbers, the drug loading of the conjugate is represented by the average DAR, which is the average number of drug molecules per antibody. The average number of drugs per antibody of the produced antibody-drug conjugate can be characterized by conventional means, such as mass spectrometry, ELISA assay, and HPLC. In other embodiments, the quantitative distribution of the antibody-drug conjugate expressed as n can also be determined. Separation, purification, and characterization of homogeneous antibody-drug conjugates where n is a certain value from antibody-drug conjugates with other drug loadings can be achieved by means such as reverse phase HPLC or electrophoresis.

### EXAMPLES

The following examples further illustrate the present disclosure; however, it should be understood that the examples are described by way of illustration rather than limitation, and various modifications can be made by those skilled in the art.

Unless expressly indicated to the contrary, the practice of the present disclosure will employ conventional methods of chemistry, biochemistry, organic chemistry, molecular biology, microbiology, recombinant DNA technology, genetics, immunology, and cell biology within the art. The experimental methods mentioned, unless otherwise specified, are conventional methods in the art using default parameters, procedures, and the like; the experimental materials used, unless otherwise specified, are commercially available products. Where specific techniques or conditions are not specified in the examples, they are carried out according to techniques or conditions described in the literature in the art, or according to the corresponding product instructions. Reagents or instruments for which the manufacturer is not specified are conventional products that can be obtained through regular commercial channels.

**Example 1. Preparation of mouse hybridomas producing anti-human FOLR1 antibodies** This example utilized FOLR1 protein to immunize mice, SP2/0 cell fusion, and flow cytometry to screen for positive clones. The specific methods were as follows:
**Animal immunization:** FOLR1-Fc protein (FO1-H5253, Acro) at 1 mg/mL was used as immunogen and mixed with an equal volume of Gerbu MM (Gerbu, 3001), Balb/c female mice were immunized at a dose of 10 µg/mouse. The protein immunization was performed four times in total at 4-day intervals. Two days after the final immunization, the mouse spleens were harvested for cell fusion.

FOLR1-Fc protein was used as immunogen and diluted to 1 mg/mL. Then it was mixed with an equal volume of CFA (Sigma, F5881) or IFA (Sigma, F5506). CFA was used for the primary immunization, and IFA for all subsequent immunizations. Female Balb/c mice were immunized by protein. The dose was 50 µg/mouse for the primary immunization and 25 µg/mouse for the subsequent immunizations. The immunizations were repeated at least 3 times at 2-week intervals. Fourteen days after the final immunization, a booster immunization was administered via intraperitoneal injection of FOLR1 protein. Three days later, the mouse spleens were harvested for cell fusion.

**Cell fusion:** Mouse splenocytes were electrofused with SP2/0 cells (ATCC No. CRL-1581) at at a splenocyte-to-SP2/0 cell ratio of 2:1 (BTX electrofusion instrument: ECM2001+), cultured in 96-well culture plates with HAT (Sigma-Aldrich, Cat. No.: H0262) medium, and antibody screening was performed using hybridoma cell supernatants 10 days later.

**Screening of hybridoma positive clones:** The cultured cell supernatants were collected and subjected to ELISA or FACS binding experiments. Positive wells from the cell culture supernatants were selected and cloned in 96-well culture plates using HT (Sigma-Aldrich, Cat. No.: H0137) medium. After 7-10 days, visible clones appeared and the supernatants were collected for antibody detection. Under an inverted microscope, positive wells with only a single clone growing were selected, expanded in culture and cryopreserved, and cultured in serum-free medium (SFM, Thermo, 12045076), after which supernatants were collected and purified for subsequent detection.

**ELISA binding experiment:** Hybridoma supernatants, positive controls, and negative controls were each added at 50 µL per well to 96-well flat-bottom assay plates (Corning, 9018) coated with human FOLR1 protein (hFOLR1-his, Acro, FO1-H52H1) or cynomolgus monkey FOLR1 protein (cynoFOLR1-his, Acro, FO1-C52H8). After incubation at 37°C for 60 minutes, the plates were washed three times with PBST, and secondary antibody (Anti-Mouse IgG, Sigma, A0168) was added, followed by incubation at 37°C for 30 minutes. After three washes with PBST, 100 µL of TMB (Yingchuang Bio, EL0009) was added to each well, color development was carried out for 15 minutes, and then 50 µL of sulfuric acid was added to each well to stop the reaction. OD values were read on a microplate reader, and the results are shown in Table 1.

**FACS binding experiment:** CHOK1 cells expressing human FOLR1 (CHOK1-hFOLR1, Kyinno Biotechnology, KC-1236), ovarian cancer cells SK-OV-3 (Cobioer, CBP60291), human oral epithelial carcinoma cells KB (Cobioer, CBP60259), and CHOK1 cells expressing cynomolgus monkey FOLR1 (CHOK1-cyno FOLR1, Kyinno Biotechnology, KC-1238) were added to 96-well plates (Corning, 3799), and then hybridoma cell supernatants, positive controls, and negative controls were respectively added to the 96-well plates containing the above four types of cells and incubated at 4°C for approximately 1 hour. After three washes with FACS buffer, 100 µL of diluted secondary antibody (Invitrogen, B118301) was added to each well to resuspend the cells, followed by incubation at 4°C for approximately 1 hour. After three washes with FACS buffer, 100 µL of FACS buffer was added to each well to resuspend the cells, and the Median Fluorescence Intensity (MFI) was measured by a flow cytometer (BECKMAN COULTER cytoFLEX or BD FACSCelesta^{™}). The results are shown in Table 1. A total of four positive hybridoma clones were screened: 115E3C9, 148B9H3, 182C2D11, and 186D9A7.

**Table 1. Ability of supernatants of positive hybridoma clones to bind hFOLR1 and cynoFOLR1 proteins**

| Experimental Method | ELISA (OD450) | | FACS (Median Fluorescence Intensity) | | | |
|---|---|---|---|---|---|---|
| Clone No | hFOLR1 Protein | cynoFOLR1 Protein | SK-OV-3 Cells | KB Cells | CHOK1-hFOLR1Cells | CHOK1-cynoFOLR1Cells |
| Positive control^{*} | 2.6894 | 2.8858 | 17671 | 83478 | 187825 | 73892.5 |
| Negative control^{#} | 0.0642 | 0.0605 | 66 | 41 | 18 | 24.5 |
| 115E3C9 | 1.6939 | 0.9875 | 13651 | 25440 | 14701 | 13777 |
| 148B9H3 | 3.1864 | 1.1299 | 13453 | 39439 | 31805 | 26154 |
| 182C2D11 | 2.9455 | 2.763 | 17323 | 42479 | 72630 | 52824 |
| 186D9A7 | 3.2659 | 2.7092 | 17899 | 47487 | 73459 | 66064 |

| | | | | | | |
|---|---|---|---|---|---|---|
| Note: *In the ELISA experiment, the positive control was mouse antibody serum after immunization and before cell fusion; in the FACS experiment, the positive control was 1µg/mL Farletuzumab-mIgG (Farletuzumab variable region sequences constructed onto murine constant regions); #In the ELISA experiment, the negative control was HAT medium; in the FACS experiment, the negative control was FACS Buffer. | | | | | | |

**Example 2. Preparation and characterization of murine monoclonal antibodies against human FOLR1**

The hybridoma cells from Table 1 were cultured in serum-free medium for 10 days, the supernatants were collected, and murine monoclonal antibodies were purified using a Protein A column (Bestchrom (Shanghai) Biosciences Ltd., Cat. No.: AA0272). The binding activity of anti-human FOLR1 antibodies and ADC killing activity were detected by flow cytometry.

### 1. Binding activity of anti-human FOLR1 antibodies to human ovarian cancer cells SK-OV-3, human oral epithelial carcinoma cells KB, and engineered cell lines expressing human and cynomolgus monkey FOLR1 (CHOK1-hFOLR1 and CHOK1-cynoFOLR1)

CHOK1 cell lines expressing human FOLR1 (CHOK1-hFOLR1, Kyinno Biotechnology, KC-1236), SK-OV-3 cells (Cobioer, CBP60291), KB cells (Cobioer, CBP60259), or CHOK1 cell lines expressing cynomolgus monkey FOLR1 (CHOK1-cyno FOLR1, Kyinno Biotechnology, KC-1238) were added to 96-well U-bottom plates at 100 µL/well, and the supernatants were discarded after centrifugation. 100 µL serially diluted antibody samples prepared with FACS buffer (PBS solution containing 1% FBS) were added to each well, followed by incubation at 4°C for approximately 1 hour. After three washes with FACS buffer, 100 µL of secondary antibody diluted in FACS buffer (Invitrogen, Cat. No.: B118301) was added to each well, followed by incubation at 4°C for approximately 1 hour. After three washes with FACS buffer, 100 µL of FACS buffer was added to each well to resuspend the cells. MFI was measured on a flow cytometer, and the data were analyzed using GraphPad Prism 8.0 software, with the logarithm of antibody concentration as the x-axis and the corresponding MFI value as the y-axis, four-parameter logistic (4PL) regression model to fit the antibody dose-response curve and calculate EC₅₀. The results are shown in Table 2.

As shown in Table 2, anti-FOLR1 antibody clones 115E3C9, 148B9H3, 182C2D11, and 186D9A7 all exhibited strong binding activity to human ovarian cancer cells SK-OV-3, human oral epithelial carcinoma cells KB, and engineered cell lines expressing human and cynomolgus monkey FOLR1 (CHOK1-hFOLR1 and CHOK1-cynoFOLR1), with binding potency comparable to or superior to Farletuzumab-mIgG.

**Table 2. Binding activity of anti-FOLR1 antibodies to cells expressing human and cynomolgus monkey FOLR1**

| Sample | SK-OV-3 EC₅₀, nM | KB EC₅₀, nM | CHOK1-cynoFOLR1 EC₅₀, nM | CHOK1-hFOLR1 EC₅₀, nM |
|---|---|---|---|---|
| Farletuzumab-mIgG | 0.206 | 2.19 | 6.67 | 3.15 |
| 115E3C9 | 0.114 | 1.71 | 1.32 | 2.80 |
| 148B9H3 | 0.147 | 2.27 | 2.25 | 2.71 |
| 182C2D11 | 0.169 | 1.42 | 1.50 | 1.23 |
| 186D9A7 | 0.140 | 1.17 | 1.07 | 1.08 |
| mIgG | N/A | N/A | N/A | N/A |

| | | | | |
|---|---|---|---|---|
| N/A: not applicable. | | | | |

### 2. Cytotoxicity induced by αMFc-CL-MMAE and anti-human FOLR1 antibodies

αMFc-CL-MMAE (Moradec, AM-202AE-50) is an anti-mouse IgG Fc-specific antibody conjugated with MMAE via a cleavable linker. αMFc-CL-MMAE recognizes the Fc region of anti-human FOLR1 murine antibodies, causing internalization in FOLR1-expressing cells and intracellular release of MMAE to kill cells. KB cells were obtained by trypsin digestion, and the cell density was adjusted to 4×10⁴ cells/mL by centrifugation. To each well of a white clear-bottom 96-well plate, 25 µL of serially diluted antibody solutions prepared in culture medium (starting final concentration of 20 nM, 4-fold dilution) and 50 µL of cell suspension were added. After 10 minutes of incubation, 25 µL of αMFc-CL-MMAE working solution (Moradec, AM-202AE-50) prepared in culture medium was added to each well, and the 96-well plate was placed in an incubator at 37°C, 5% CO₂. After 3 days, 50 µL of CTG (Adamas Life, RA-GL11-A) was added to each well, and relative light unit (RLU) values were read on a microplate reader. EC50 values for each antibody were calculated by fitting RLU data using a four-parameter logistic (4PL) model. The results are shown in Table 3. Anti-FOLR1 antibody clones 115E3C9, 148B9H3, 182C2D11, and 186D9A7 all exhibited strong killing activity, with killing potency comparable to or superior to Farletuzumab-mIgG.

**Table 3. Cytotoxicity induced by αMFc-CL-MMAE and anti-human FOLR1 antibodies**

| Sample | EC₅₀, nM |
|---|---|
| Farletuzumab-mIgG | 0.098 |
| 115E3C9 | 0.059 |
| 148B9H3 | 0.060 |
| 186D9A7 | 0.039 |
| 182C2D11 | 0.055 |
| mIgG | N/A |

| | |
|---|---|
| N/A: no binding activity or poor binding activity; no valid EC₅₀ value was obtained. | |

### Example 3. Sequencing of anti-FOLR1 antibodies, preparation of chimeric antibodies, and functional characterization

### 1. Sequencing, expression, and purification of anti-FOLR1 antibodies

The above positive hybridoma clones were sequenced, and the sequences are shown in Table 4. The light and heavy chain variable regions obtained by sequencing (Table 4) were constructed onto human constant regions (IgG1/κ, Table 5) to construct the corresponding chimeric antibodies, and the sequences were verified by sequencing. Chimeric antibodies were named by adding the prefix "ch" to the corresponding hybridoma clone number. For example, the chimeric antibody obtained from hybridoma clone 115E3C9 through this example was named ch115E3C9, and used for in vitro functional characterization or in vivo efficacy studies.

The corresponding nucleic acids encoding the antibodies were transfected into Expi293 cells for antibody expression, and purified using Protein A columns, as follows:
Expi293 cells at 1.5×10⁶ cells/mL were placed on a shaker at 37°C, 8% CO₂, 120 rpm for culture. The next day, cell density and viability were measured; the density should be 3×10⁶ cells/mL with viability greater than 95%. Plasmids containing the encoding nucleic acids were diluted with OPM-293 CD05 Medium (OPM biosciences Co., Ltd., Cat. No.: 81075-001), diluted to a final total plasmid concentration of 1 µg/mL; The medium volume used for plasmid dilution accounted for 1/20 of the total transfection volume, and the heavy chain to light chain molar ratio was 1:1.5. PEI (1 mg/mL, Polysciences, Cat. No.: 24765-1) was mixed by inversion, diluted with OPM-293 CD05 Medium; the volume of medium for diluting PEI was 1/20 of the transfection volume, and incubated at room temperature for 5 minutes. The diluted PEI was added to the diluted plasmid and mixed, incubated at room temperature for 15 minutes. The PEI/plasmid complex was then added to the shake flask containing Expi293 cells and cultured at 37°C, 8% CO₂, 120 rpm for 24 hours, after which 10% OPM-293 ProFeed (OPM biosciences Co., Ltd., Cat. No.: F081918) was added, and culture was continued for 5-7 days before the supernatant was collected.

A gasket was placed at the bottom of a gravity chromatography column and pressed tightly. The required volume of Protein A resin slurry (Cytiva, Cat. No.: 17549801) was calculated as target resin volume divided by resin slurry concentration. The packing was thoroughly vortexed, added to the bottom of the gravity chromatography column, and PBS was flushed through the column until the pH of the effluent reached the target value. The sample was loaded onto the column, at least 10 column volumes (CV) of wash buffer were added, followed by 5 CV of elution buffer (20 mM NaAc, pH 3.0), incubated for 3-5 minutes, and then the eluate was collected. The pH was adjusted to 7.2 with neutralization buffer (1 M Tris), protein concentration was determined by Nanodrop, and buffer was exchanged to PBS using Millipore ultrafiltration spin columns (50 kDa).

**Table 4. CDR sequences and variable region sequences of anti-FOLR1 hybridoma antibodies (Defined According to the Kabat Numbering Scheme)**

| Clone | | Amino acid sequence | SEQ ID NO: |
|---|---|---|---|
| 115E3C9 | HCDR1 | NYGVN | 1 |
| | HCDR2 | WMNTNTGEPTYAEDFKG | 2 |
| | HCDR3 | MAMITGYFDY | 3 |
| | **VH** | QIQLVQSGPELKKPGETVKISCKASGSPFTNYGVNWVKQAPGKGL KWMGWMNTNTGEPTYAEDFKGRFAFSLETSGSTAHLQISNLKNED TATYFCARMAMITGYFDYWGQGTALTVSS | 4 |
| | LCDR1 | RASQVFSNYLN | 5 |
| | LCDR2 | FTSRLHS | 6 |
| | LCDR3 | QQGDTFPYT | 7 |
| | **VL** | DIQMTQTTSSLSASLGDRVTINCRASQVFSNYLNWYQQKPNGTVK LLILFTSRLHSGVPSRFSGSGSGTDYSLTISNLEQEDIATYFCQQGDT FPYTFGGGTKLEIK | 8 |
| 148B9H3 | HCDR1 | DYIIH | 9 |
| | HCDR2 | WFYPGSGTIRFNEKFKD | 10 |
| | HCDR3 | HEHRFTTAMDY | 11 |
| | **VH** | KVQLQQSGAGLVKPGASVKLSCKASGYTFTDYIIHWVKQRSGQGL EWIGWFYPGSGTIRFNEKFKDKATLTADKSSSTVYMELSGLTSEDS AVYFCARHEHRFTTAMDYWGQGTSVTVSS | 12 |
| | LCDR1 | KASEDIYNRLA | 13 |
| | LCDR2 | GATSLET | 14 |
| | LCDR3 | QQYWSIPFT | 15 |
| | **VL** | DIQMTQSSSSFSVSPGDRVTITCKASEDIYNRLAWYQQKPGNAPRL LISGATSLETGVPSRFSGSGSGKDYTLSITSLQTEDVVTYYCQQYW SIPFTFGAGTKLELK | 16 |
| 186D9A7 | HCDR1 | SYWMH | 17 |
| | HCDR2 | TIYPGNSDTKYSQKFRD | 18 |
| | HCDR3 | RDGSWFAY | 19 |
| | **VH** | EVQLQQSGTVLARPGASVKMSCKASGYSFSSYWMHWIKQRPGQG LEWIATIYPGNSDTKYSQKFRDKAKLTAVTSASTAYMELSSLTNEDS AVYYCTRRDGSWFAYWGQGTLVTVSA | 20 |
| | LCDR1 | RASENIYDNLA | 21 |
| | LCDR2 | DATNLAD | 22 |
| | LCDR3 | QHFWNTPPT | 23 |
| | **VL** | DIQMTQSPASLSVSVGETVTITCRASENIYDNLAWYRQKQGKSPQL LVYDATNLADGVPSRFSGSGSGTQYSLKINSLQSEDCGSYYCQHF WNTPPTFGGGTKLEIK | 24 |
| 182C2D11 | HCDR1 | NYWMH | 25 |
| | HCDR2 | AIYPGNSDTNYNQKFRD | 26 |
| | HCDR3 | RDGSWFAY | 27 |
| | **VH** | EAQLQQSGTVLARPGASVKMSCKASGYSFSNYWMHWVKQRPGQ GLEWIAAIYPGNSDTNYNQKFRDKAKLTAVTSASTAYMELSSLTNE DSAVYYCTRRDGSWFAYWGQGTLVTVSA | 28 |
| | LCDR1 | RASENIYDNLA | 29 |
| | LCDR2 | DATNLAD | 30 |
| | LCDR3 | QHFWNTPPT | 31 |
| | **VL** | DIQMTQSPASLSVSVGETVTITCRASENIYDNLAWYRQKQGKSPQL LVYDATNLADGVPSRFGGSGSGTQYSLKINSLQSEDFGSYYCQHF WNTPPTFGGGTKLEIK | 32 |

**Table 5. Constant region sequences of chimeric antibodies and humanized antibodies**

| Name | Amino acid sequence | SEQ ID NO: |
|---|---|---|
| IgG1/κ heavy chain constant region | | 33 |
| IgG1/κ light chain constant region | | 34 |

### 2. Binding activity of anti-FOLR1 chimeric antibodies to human ovarian cancer cells SK-OV-3, human oral epithelial carcinoma cells KB, and engineered cell lines expressing human and cynomolgus monkey FOLR1 (CHOK1-hFOLR1 and CHOK1-cynoFOLR1)

Human ovarian cancer cells SK-OV-3, human oral epithelial carcinoma cells KB, and engineered cell lines expressing human and cynomolgus monkey FOLR1 (CHOK1-hFOLR1 and CHOK1-cynoFOLR1) were seeded into 96-well U-bottom plates at 100 µL per well. Plates were centrifuged and the supernatant was discarded. 100 µL serially diluted antibody samples prepared with FACS buffer (PBS solution containing 1% FBS) were added to each well, followed by incubation at 4°C for approximately 1 hour. After three washes with FACS buffer, 100 µL of secondary antibody diluted in FACS buffer (Invitrogen, Cat. No.: A21445) was added to each well, followed by incubation at 4°C for approximately 1 hour. After three washes with FACS buffer, 100 µL of FACS buffer was added to each well to resuspend the cells. MFI were measured by a flow cytometer, and the data were analyzed using GraphPad Prism 8.0 software, with the logarithm of antibody concentration as the x-axis and the corresponding MFI value as the y-axis, using a four-parameter logistic (4PL) regression model to fit the antibody dose-response curve and calculate EC₅₀.

The results are shown in Table 6. Chimeric antibodies ch115E3C9, ch148B9H3, ch186D9A7, and ch182C2D11 exhibited strong binding activity to human ovarian cancer cells SK-OV-3, human oral epithelial carcinoma cells KB, and engineered cell lines expressing human and cynomolgus monkey FOLR1 (CHOK1-hFOLR1 and CHOK1-cynoFOLR1), with binding potency comparable to or superior to Farletuzumab.

**Table 6. Binding activity of anti-FOLR1 chimeric antibodies to human FOLR1 and cynomolgus monkey FOLR1 (EC₅₀, nM)**

| Sample | SK-OV-3 | KB | CHOK1-hFOLR1 | CHOK1-cynoFOLR1 |
|---|---|---|---|---|
| Farletuzumab | 0.0318 | 0.293 | 0.593 | 0.797 |
| ch115E3C9 | 0.0333 | 0.320 | 0.448 | 0.416 |
| ch148B9H3 | 0.0314 | 0.256 | 0.701 | 0.588 |
| ch186D9A7 | 0.0172 | NT | 0.281 | 0.283 |
| ch182C2D11 | 0.0190 | NT | 0.312 | 0.297 |
| hIgG1 | N/A | NT | N/A | N/A |

| | | | | |
|---|---|---|---|---|
| N/A: no binding activity or poor binding activity; no valid EC₅₀ value was obtained. NT: not tested. | | | | |

### 3. Cytotoxicity of conjugates of anti-human FOLR1 chimeric antibody and MMAE

### 3.1 Preparation of conjugates of anti-FOLR1 antibody and MMAE

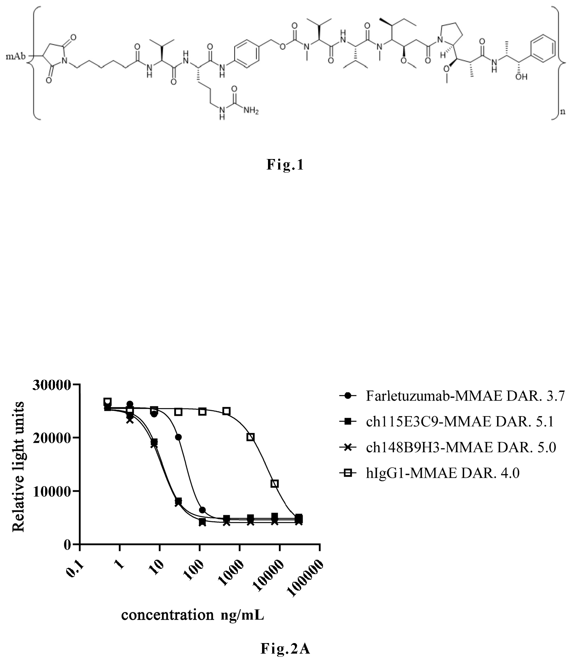

Antibody ch115E3C9, ch148B9H3, ch182C2D11, ch186D9A7, or control antibody Farletuzumab and PBS buffer were added to bring the antibody concentration to approximately 1 mg/mL; EDTA solution (Hushi, 10009617) was added to bring the EDTA concentration to approximately 2 mM; 1 M Tris salt solution was added to bring the pH of the reaction system between 6.6-7.5; 2-6 equivalents of TCEP (Thermo, 20490) were added for reduction, and the mixture was gently shaken on a mixer at 37°C for 1-3 hours. The mixer temperature was then adjusted to approximately 4°C, the reaction solution was cooled, and 5-10 equivalents of VcMMAE dissolved in DMSO (MCE, HY-15575, Figure 1) were added, followed by reaction at 4°C for 1-2 hours. Finally, the reaction solution was loaded into a 30K ultrafiltration spin column, buffer was exchanged 6-8 times with 10 mM histidine hydrochloride solution at pH 6.0, concentrated to an appropriate volume, loaded onto a pre-equilibrated desalting column, and centrifuged to obtain the purified ADC product. The concentration was measured by Nanodrop, and the DAR value was determined by hydrophobic interaction chromatography HPLC. Conjugates of FOLR1 antibody and MMAE ch115E3C9-MMAE, ch148B9H3-MMAE, ch182C2D11-MMAE, ch186D9A7-MMAE, and Farletuzumab-MMAE were obtained. The structure of conjugate of the FOLR1 antibody and MMAE is shown in Figure 1.

### 3.2 Cytotoxicity assay of conjugates of anti-FOLR1 antibody and MMAE

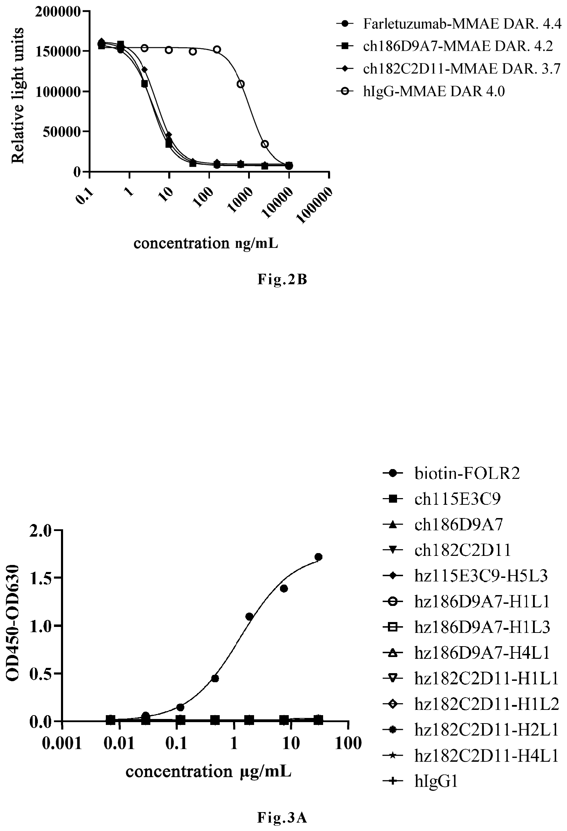

KB cell concentration was adjusted to 4×10⁴ cells/mL and seeded at 50 µL/well in white clear-bottom 96-well plates. 50 µL serially diluted ADC solutions prepared in MEM complete medium were added to each well, and plates were cultured in a 37°C, 5% CO₂ incubator. After 3 days, the number of viable cells and cell viability were quantified by the CTG method. Specifically, 50 µL of CTG reagent (Adamas Life, RA-GL11) was added to each well, and the reaction was carried out for 5 minutes at room temperature in the dark. Relative light units (RLU) were measured on a microplate reader. Experimental data were analyzed using GraphPad Prism 8.0 software, with the logarithm of antibody concentration as the x-axis and the corresponding RLU value as the y-axis, using a four-parameter equation regression model to fit the antibody dose-response curve. The results are shown in Figure 2 and Table 7. ch115E3C9-MMAE, ch148B9H3-MMAE, ch182C2D11-MMAE, and ch186D9A7-MMAE exhibited strong cytotoxicity, comparable to or better than the cytotoxicity of Farletuzumab-MMAE.

**Table 7. Cytotoxicity of conjugates of anti-FOLR1 chimeric antibody and MMAE**

| ADC | EC50 ng/mL |
|---|---|
| Farletuzumab-MMAE Dar. 3.7 | 42.6 |
| ch115E3C9-MMAE Dar. 5.1 | 11.6 |
| ch148B9H3-MMAE Dar. 5.0 | 11.4 |
| Farletuzumab-MMAE Dar. 4.4 | 3.91 |
| ch182C2D11-MMAE Dar. 3.7 | 4.91 |
| ch186D9A7-MMAE Dar. 4.2 | 3.68 |
| hIgG1-MMAE Dar. 4.0 | N/A |

| | |
|---|---|
| N/A: not applicable. | |

### Example 4. Humanization of Anti-FOLR1 antibodies and expression and purification of humanized antibodies

CDRs were defined according to the Kabat numbering scheme. Human germline sequences sharing the highest homology with the murine variable regions were selected as human acceptor frameworks, and the CDRs from the murine sequences were grafted into the human frameworks. Based on the functional importance of framework amino acid residues, back mutations were introduced, i.e., certain key amino acids in the grafted framework regions were back-mutated to the corresponding murine amino acids. Several variants were designed for the heavy and light chains, respectively.

### 1. Humanization of anti-FOLR1 antibody 115E3C9

The HCDRs and LCDRs of the 115E3C9 murine antibody were grafted onto human germline genes IGHV7-4-1*01 and IGKV1-39*01, respectively, and the obtained sequences are described below:

**Table 8. Humanized sequences of anti-FOLR1 antibody 115E3C9**

| | **Name** | **Sequence** | SEQ ID NO |
|---|---|---|---|
| Heavy chain variable region | 061-115E3C9-hH4 | QVQLVQSGSELKKPGASVKVSCKASGYTFTNYGVNWVRQAPGQGL EWMGWMNTNTGEPTYAEDFKGRFVFSLDTSVSTAYLQISSLKAEDT AVYYCARMAMITGYFDYWGQGTMVTVSS | 35 |
| | 061-115E3C9-hH5 | QVQLVQSGSELKKPGASVKVSCKASGSTFTNYGVNWVRQAPGQGL EWMGWMNTNTGEPTYAEDFKGRFVFSLDTSVSTAYLQISSLKAEDT AVYYCARMAMITGYFDYWGQGTMVTVSS | 36 |
| | 061-115E3C9-hH6 | QIQLVQSGSELKKPGASVKVSCKASGSTFTNYGVNWVRQAPGQGLE WMGWMNTNTGEPTYAEDFKGRFVFSLDTSVSTAYLQISSLKAEDTA VYFCARMAMITGYFDYWGQGTMVTVSS | 37 |
| | 061-115E3C9-hH7 | QIQLVQSGSELKKPGASVKVSCKASGSPFTNYGVNWVRQAPGQGLE WMGWMNTNTGEPTYAEDFKGRFVFSLDTSVSTAYLQISSLKAEDTA VYFCARMAMITGYFDYWGQGTMVTVSS | 38 |
| Light chain variable region | 061-115E3C9-hL1 | DIQMTQSPSSLSASVGDRVTITCRASQVFSNYLNWYQQKPGKAPKLL IYFTSRLHSGVPSRFSGSGSGTDFTLTISSLQPEDFATYYCQQGDTFPY TFGGGTKVEIK | 39 |
| | 061-115E3C9-hL2 | DIQMTQSPSSLSASVGDRVTITCRASQVFSNYLNWYQQKPGKAPKLL ILFTSRLHSGVPSRFSGSGSGTDFTLTISSLQPEDFATYYCQQGDTFPY TFGGGTKVEIK | 40 |
| | 061-115E3C9-hL3 | DIQMTQSPSSLSASVGDRVTITCRASQVFSNYLNWYQQKPGKAPKLL ILFTSRLHSGVPSRFSGSGSGTDYTLTISSLQPEDFATYFCQQGDTFPY TFGGGTKVEIK | 41 |
| | 061-115E3C9-hL4 | DIQMTQSPSSLSASVGDRVTITCRASQVFSNYLNWYQQKPGKTVKL LILFTSRLHSGVPSRFSGSGSGTDYTLTISSLQPEDFATYFCQQGDTFP YTFGGGTKVEIK | 42 |

### 2. Humanization of anti-FOLR1 antibody 148B9H3

The HCDRs and LCDRs of murine antibody 148B9H3 were grafted onto human germline genes IGHV1-3*01 and IGKV1-33*01, respectively, and the resulting sequences are listed below:

**Table 9. Humanized sequences of anti-FOLR1 antibody 148B9H3**

| | Name | Sequence | SEQ ID NO |
|---|---|---|---|
| Heavy chain variable region | 066-148B9H3-hH1 | QVQLVQSGAEVKKPGASVKVSCKASGYTFTDYIIHWVRQAPGQRLEW MGWFYPGSGTIRFNEKFKDRVTITADKSASTAYMELSSLRSEDTAVYY CARHEHRFTTAMDYWGQGTMVTVSS | 43 |
| | 066-148B9H3-hH2 | QVQLVQSGAEVKKPGASVKVSCKASGYTFTDYIIHWVRQAPGQRLEWI GWFYPGSGTIRFNEKFKDRVTITADKSASTAYMELSSLRSEDTAVYYCA RHEHRFTTAMDYWGQGTMVTVSS | 44 |
| | 066-148B9H3-hH3 | KVQLVQSGAEVKKPGASVKVSCKASGYTFTDYIIHWVRQAPGQRLEWI GWFYPGSGTIRFNEKFKDRATITADKSASTVYMELSSLRSEDTAVYYCA RHEHRFTTAMDYWGQGTMVTVSS | 45 |
| | 066-148B9H3-hH4 | KVQLVQSGAEVKKPGASVKVSCKASGYTFTDYIIHWVRQAPGQGLEWI GWFYPGSGTIRFNEKFKDRATLTADKSASTVYMELSSLRSEDTAVYYC ARHEHRFTTAMDYWGQGTMVTVSS | 46 |
| Light chain variable region | 066-148B9H3-hL1 | DIQMTQSPSSLSASVGDRVTITCKASEDIYNRLAWYQQKPGKAPKLLIY GATSLETGVPSRFSGSGSGTDFTFTISSLQPEDIATYYCQQYWSIPFTFGG GTKVEIK | 47 |
| | 066-148B9H3-hL2 | DIQMTQSPSSLSASVGDRVTITCKASEDIYNRLAWYQQKPGKAPKLLIS GATSLETGVPSRFSGSGSGTDFTFTISSLQPEDIATYYCQQYWSIPFTFGG GTKVEIK | 48 |
| | 066-148B9H3-hL3 | DIQMTQSPSSLSASVGDRVTITCKASEDIYNRLAWYQQKPGKAPKLLIS GATSLETGVPSRFSGSGSGKDFTFTISSLQPEDIATYYCQQYWSIPFTFGG GTKVEIK | 49 |
| | 066-148B9H3-hL4 | DIQMTQSPSSLSASVGDRVTITCKASEDIYNRLAWYQQKPGKAPKLLIS GATSLETGVPSRFSGSGSGKDYTFTISSLQPEDIATYYCQQYWSIPFTFG GGTKVEIK | 50 |

### 3. Humanization of anti-FOLR1 antibody 186D9A7

The HCDRs and LCDRs of murine antibody 186D9A7 were grafted onto human germline genes IGHV1-69, KV1-NL1, and IGKV1-5, respectively, and the following humanized sequences were obtained.

**Table 10. Humanized sequences of anti-FOLR1 antibody 186D9A7**

| | **Name** | **Sequence** | SEQ ID NO |
|---|---|---|---|
| Heavy chain variable region | 076-186D9A7-hH1 | QVQLVQSGAEVKKPGSSVKVSCKASGGTFSSYWMHWVRQAPGQGLE WMGTIYPGNSDTKYSQKFRDRVTITADTSTSTAYMELSSLRSEDTAVY YCTRRDGSWFAYWGQGTLVTVSS | 51 |
| | 076-186D9A7-hH2 | QVQLVQSGAEVKKPGSSVKVSCKASGGTFSSYWMHWVRQAPGQGLE WMATIYPGNSDTKYSQKFRDRATITADTSTSTAYMELSSLRSEDTAVY YCTRRDGSWFAYWGQGTLVTVSS | 52 |
| | 076-186D9A7-hH3 | QVQLVQSGAEVKKPGSSVKVSCKASGGTFSSYWMHWVRQAPGQGLE WIATIYPGNSDTKYSQKFRDRATLTADTSTSTAYMELSSLRSEDTAVY YCTRRDGSWFAYWGQGTLVTVSS | 53 |
| | 076-186D9A7-hH4 | QVQLVQSGAEVKKPGSSVKVSCKASGGSFSSYWMHWIRQAPGQGLE WIATIYPGNSDTKYSQKFRDRATLTADTSTSTAYMELSSLRSEDTAVY YCTRRDGSWFAYWGQGTLVTVSS | 54 |
| Light chain variable region | 076-186D9A7-hL1 | DIQMTQSPSSLSASVGDRVTITCRASENIYDNLAWYQQKPGKAPKLLL YDATNLADGVPSRFSGSGSGTDYTLTISSLQPEDFATYYCQHFWNTPPT FGGGTKVEIK | 55 |
| | 076-186D9A7-hL2 | DIQMTQSPSSLSASVGDRVTITCRASENIYDNLAWYQQKPGKAPKLLV YDATNLADGVPSRFSGSGSGTDYTLTISSLQPEDFATYYCQHFWNTPPT FGGGTKVEIK | 56 |
| | 076-186D9A7-hL3 | DIQMTQSPSSLSASVGDRVTITCRASENIYDNLAWYQQKPGKSPKLLV YDATNLADGVPSRFSGSGSGTDYTLTISSLQPEDFATYYCQHFWNTPPT FGGGTKVEIK | 57 |
| | 076-186D9A7-hL4 | DIQMTQSPSTLSASVGDRVTITCRASENIYDNLAWYQQKPGKAPKLLIY DATNLADGVPSRFSGSGSGTEFTLTISSLQPDDFATYYCQHFWNTPPTF GGGTKVEIK | 58 |
| | 076-186D9A7-hL5 | DIQMTQSPSTLSASVGDRVTITCRASENIYDNLAWYQQKPGKAPKLLV YDATNLADGVPSRFSGSGSGTEFTLTISSLQPDDFATYYCQHFWNTPPT FGGGTKVEIK | 59 |
| | 076-186D9A7-hL6 | DIQMTQSPSTLSASVGDRVTITCRASENIYDNLAWYQQKPGKSPKLLV YDATNLADGVPSRFSGSGSGTEFTLTISSLQPDDFATYYCQHFWNTPPT FGGGTKVEIK | 60 |

### 4. Humanization of anti-FOLR1 antibody 182C2D11

The HCDRs and LCDRs of the 182C2D11 murine antibody were grafted onto human germline genes IGHV1-69, KV1-NL1, and IGKV1-5, respectively. The obtained heavy chain variable region sequences are shown in Table 11 below, and the obtained corresponding light chain variable region sequences were identical to the light chain variable region sequences of the humanized 186D9A7 antibody, i.e., the light chain variable region sequences of the obtained humanized 182C2D11 antibody are shown in any one of SEQ ID NOs: 55-60. The specific combinations of heavy and light chain variable region sequences of the obtained humanized 182C2D11 antibodies are shown in Table 12.

**Table 11. Humanized heavy chain variable region sequences of anti-FOLR1 antibody 182C2D11**

| | **Name** | **Sequence** | SEQ ID NO |
|---|---|---|---|
| Heavy chain variable region | 083-182C2D11 -hH1 | QVQLVQSGAEVKKPGSSVKVSCKASGGTFSNYWMHWVRQAPGQGL EWMGAIYPGNSDTNYNQKFRDRVTITADTSTSTAYMELSSLRSEDTA VYYCTRRDGSWFAYWGQGTLVTVSS | 61 |
| | 083-182C2D11 -hH2 | QVQLVQSGAEVKKPGSSVKVSCKASGGTFSNYWMHWVRQAPGQGL EWMAAIYPGNSDTNYNQKFRDRATITADTSTSTAYMELSSLRSEDTA VYYCTRRDGSWFAYWGQGTLVTVSS | 62 |
| | 083-182C2D11 -hH3 | QVQLVQSGAEVKKPGSSVKVSCKASGGTFSNYWMHWVRQAPGQGL EWIAAIYPGNSDTNYNQKFRDRATLTADTSTSTAYMELSSLRSEDTA VYYCTRRDGSWFAYWGQGTLVTVSS | 63 |
| | 083-182C2D11 -hH4 | QAQLVQSGAEVKKPGSSVKVSCKASGGSFSNYWMHWVRQAPGQGL EWIAAIYPGNSDTNYNQKFRDRATLTADTSTSTAYMELSSLRSEDTA VYYCTRRDGSWFAYWGQGTLVTVSS | 64 |

**Table 12. Heavy chain variable region sequences and light chain variable region sequences corresponding to the constructed humanized antibodies**

| Name of humanized antibodies | Heavy chain variable region sequences | Light chain variable region sequences |
|---|---|---|
| hz115E3C9-H4L1 | 061-115E3C9-hH4 | 061-115E3C9-hL1 |
| hz115E3C9-H4L2 | 061-115E3C9-hH4 | 061-115E3C9-hL2 |
| hz115E3C9-H4L3 | 061-115E3C9-hH4 | 061-115E3C9-hL3 |
| hz115E3C9-H4L4 | 061-115E3C9-hH4 | 061-115E3C9-hL4 |
| hz115E3C9-H5L1 | 061-115E3C9-hH5 | 061-115E3C9-hL1 |
| hz115E3C9-H5L2 | 061-115E3C9-hH5 | 061-115E3C9-hL2 |
| hz115E3C9-H5L3 | 061-115E3C9-hH5 | 061-115E3C9-hL3 |
| hz115E3C9-H5L4 | 061-115E3C9-hH5 | 061-115E3C9-hL4 |
| hz115E3C9-H6L1 | 061-115E3C9-hH6 | 061-115E3C9-hL1 |
| hz115E3C9-H6L2 | 061-115E3C9-hH6 | 061-115E3C9-hL2 |
| hz115E3C9-H6L3 | 061-115E3C9-hH6 | 061-115E3C9-hL3 |
| hz115E3C9-H6L4 | 061-115E3C9-hH6 | 061-115E3C9-hL4 |
| hz186D9A7-H1L1 | 076-186D9A7-hH1 | 076-186D9A7-hL1 |
| hz186D9A7-H1L2 | 076-186D9A7-hH1 | 076-186D9A7-hL2 |
| hz186D9A7-H1L3 | 076-186D9A7-hH1 | 076-186D9A7-hL3 |
| hz186D9A7-H2L1 | 076-186D9A7-hH2 | 076-186D9A7-hL1 |
| hz186D9A7-H2L2 | 076-186D9A7-hH2 | 076-186D9A7-hL2 |
| hz186D9A7-H2L3 | 076-186D9A7-hH2 | 076-186D9A7-hL3 |
| hz186D9A7-H2L4 | 076-186D9A7-hH2 | 076-186D9A7-hL4 |
| hz186D9A7-H2L5 | 076-186D9A7-hH2 | 076-186D9A7-hL5 |
| hz186D9A7-H2L6 | 076-186D9A7-hH2 | 076-186D9A7-hL6 |
| hz186D9A7-H3L1 | 076-186D9A7-hH3 | 076-186D9A7-hL1 |
| hz186D9A7-H3L2 | 076-186D9A7-hH3 | 076-186D9A7-hL2 |
| hz186D9A7-H3L3 | 076-186D9A7-hH3 | 076-186D9A7-hL3 |
| hz186D9A7-H3L4 | 076-186D9A7-hH3 | 076-186D9A7-hL4 |
| hz186D9A7-H3L5 | 076-186D9A7-hH3 | 076-186D9A7-hL5 |
| hz186D9A7-H3L6 | 076-186D9A7-hH3 | 076-186D9A7-hL6 |
| hz186D9A7-H4L1 | 076-186D9A7-hH4 | 076-186D9A7-hL1 |
| hz186D9A7-H4L2 | 076-186D9A7-hH4 | 076-186D9A7-hL2 |
| hz186D9A7-H4L3 | 076-186D9A7-hH4 | 076-186D9A7-hL3 |
| hz186D9A7-H4L4 | 076-186D9A7-hH4 | 076-186D9A7-hL4 |
| hz186D9A7-H4L5 | 076-186D9A7-hH4 | 076-186D9A7-hL5 |
| hz182C2D11-H1L1 | 083-182C2D11-hH1 | 076-186D9A7-hL1 |
| hz182C2D11-H1L2 | 083-182C2D11-hH1 | 076-186D9A7-hL2 |
| hz182C2D11-H1L3 | 083-182C2D11-hH1 | 076-186D9A7-hL3 |
| hz182C2D11-H1L4 | 083-182C2D11-hH1 | 076-186D9A7-hL4 |
| hz182C2D11-H1L5 | 083-182C2D11-hH1 | 076-186D9A7-hL5 |
| hz182C2D11-H2L1 | 083-182C2D11-hH2 | 076-186D9A7-hL1 |
| hz182C2D11-H2L2 | 083-182C2D11-hH2 | 076-186D9A7-hL2 |
| hz182C2D11-H2L3 | 083-182C2D11-hH2 | 076-186D9A7-hL3 |
| hz182C2D11-H2L4 | 083-182C2D11-hH2 | 076-186D9A7-hL4 |
| hz182C2D11-H2L5 | 083-182C2D11-hH2 | 076-186D9A7-hL5 |
| hz182C2D11-H2L6 | 083-182C2D11-hH2 | 076-186D9A7-hL6 |
| hz182C2D11-H3L1 | 083-182C2D11-hH3 | 076-186D9A7-hL1 |
| hz182C2D11-H3L2 | 083-182C2D11-hH3 | 076-186D9A7-hL2 |
| hz182C2D11-H3L3 | 083-182C2D11-hH3 | 076-186D9A7-hL3 |
| hz182C2D11-H3L4 | 083-182C2D11-hH3 | 076-186D9A7-hL4 |
| hz182C2D11-H3L5 | 083-182C2D11-hH3 | 076-186D9A7-hL5 |
| hz182C2D11-H3L6 | 083-182C2D11-hH3 | 076-186D9A7-hL6 |
| hz182C2D11-H4L1 | 083-182C2D11-hH4 | 076-186D9A7-hL1 |
| hz182C2D11-H4L2 | 083-182C2D11-hH4 | 076-186D9A7-hL2 |
| hz182C2D11-H4L3 | 083-182C2D11-hH4 | 076-186D9A7-hL3 |
| hz182C2D11-H4L4 | 083-182C2D11-hH4 | 076-186D9A7-hL4 |
| hz182C2D11-H4L5 | 083-182C2D11-hH4 | 076-186D9A7-hL5 |
| hz182C2D11-H4L6 | 083-182C2D11-hH4 | 076-186D9A7-hL6 |
| hz148B9H3-H1L1 | 066-148B9H3-hH1 | 066-148B9H3-hL1 |
| hz148B9H3-H1L2 | 066-148B9H3-hH1 | 066-148B9H3-hL2 |
| hz148B9H3-H1L3 | 066-148B9H3-hH1 | 066-148B9H3-hL3 |
| hz148B9H3-H1L4 | 066-148B9H3-hH1 | 066-148B9H3-hL4 |
| hz148B9H3-H2L1 | 066-148B9H3-hH2 | 066-148B9H3-hL1 |
| hz148B9H3-H2L2 | 066-148B9H3-hH2 | 066-148B9H3-hL2 |
| hz148B9H3-H2L3 | 066-148B9H3-hH2 | 066-148B9H3-hL3 |
| hz148B9H3-H2L4 | 066-148B9H3-hH2 | 066-148B9H3-hL4 |
| hz148B9H3-H3L1 | 066-148B9H3-hH3 | 066-148B9H3-hL1 |
| hz148B9H3-H3L2 | 066-148B9H3-hH3 | 066-148B9H3-hL2 |
| hz148B9H3-H3L3 | 066-148B9H3-hH3 | 066-148B9H3-hL3 |
| hz148B9H3-H3L4 | 066-148B9H3-hH3 | 066-148B9H3-hL4 |
| hz148B9H3-H4L1 | 066-148B9H3-hH4 | 066-148B9H3-hL1 |
| hz148B9H3-H4L2 | 066-148B9H3-hH4 | 066-148B9H3-hL2 |
| hz148B9H3-H4L3 | 066-148B9H3-hH4 | 066-148B9H3-hL3 |
| hz148B9H3-H4L4 | 066-148B9H3-hH4 | 066-148B9H3-hL4 |

### 5. Expression and purification of anti-FOLR1 humanized antibodies

The light and heavy chain variable regions of the humanized antibodies in Tables 8-11 were constructed onto human constant regions (hIgG1/κ, Table 5) according to the combinations in Table 12. Gene synthesis was performed, and sequencing confirmed consistency with the designed sequences. Expression was carried out in Expi293 cells using the method described in Example 3, and purification was performed using Protein A columns.

### Example 5. Functional characterization of anti-FOLR1 humanized antibodies

The humanized antibodies were respectively tested for their binding activity to human FOLR1 (SK-OV-3, KB), mouse FOLR1, and cynomolgus monkey FOLR1 (CHOK1-cynoFOLR1), their binding activity to human FOLR2, human FOLR3, and human FOLR4, and their activity in blocking the binding of FOLR1 to Folic acid-BSA conjugate (FA-BSA).

### 1. Saturation binding experiments with anti-FOLR1 humanized antibodies

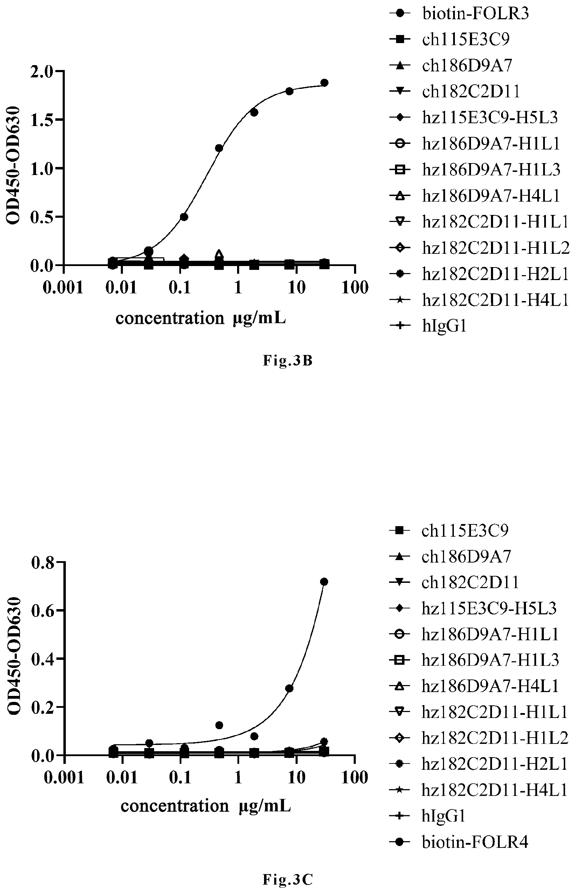

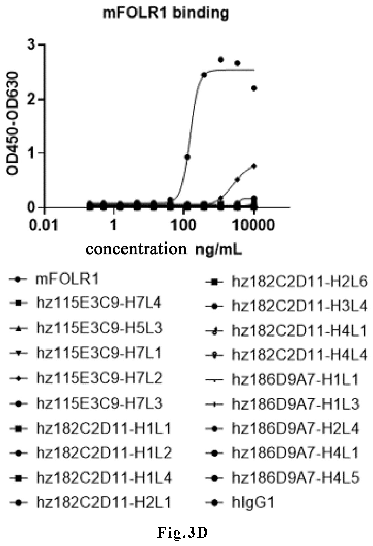

The binding activity of anti-FOLR1 humanized antibodies to human FOLR1, mouse FOLR1, and cynomolgus monkey FOLR1 was detected using the method disclosed in Example 3. The results of antibody binding to human and cynomolgus monkey FOLR1 are shown in Table 13. The results of antibody binding to mouse FOLR1 are shown in Figure 3D, except for hz115E3C9-H7L2, all other tested anti-FOLR1 humanized antibodies showed no significant binding to mouse FOLR1.

**Table 13. EC₅₀ values for binding of anti-FOLR1 humanized antibodies to human FOLR1 and cynomolgus monkey FOLR1**

| 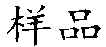 | SK-OV-3 cells, nM | KB cells, nM | CHOK1-cynoFOLR1 cells, nM |
|---|---|---|---|
| Farletuzumab | 0.391 | 2.62 | 8.33 |
| ch115E3C9 | 0.227 | 1.15 | 7.31 |
| hz115E3C9-H4L1 | N/A | 1.92 | 9.64 |
| hz115E3C9-H4L2 | 0.241 | 4.65 | 7.22 |
| hz115E3C9-H4L3 | 0.258 | 8.96 | 9.94 |
| hz115E3C9-H4L4 | 0.211 | 3.96 | 8.28 |
| hz115E3C9-H5L1 | 0.398 | 1.75 | 7.65 |
| hz115E3C9-H5L2 | 0.208 | 3.17 | 5.71 |
| hz115E3C9-H5L3 | 0.188 | 3.91 | 6.64 |
| hz115E3C9-H5L4 | 0.239 | 4.63 | 8.87 |
| hz115E3C9-H6L1 | 0.357 | 3.28 | 8.10 |
| hz115E3C9-H6L2 | 0.155 | 3.36 | 6.16 |
| hz115E3C9-H6L3 | 0.209 | 3.11 | 6.94 |
| hz115E3C9-H6L4 | 0.199 | 3.86 | 7.20 |
| ch148B9H3 | 0.390 | NT | NT |
| hz148B9H3-H1L1 | 0.520 | NT | NT |
| hz148B9H3-H1L2 | 0.566 | NT | NT |
| hz148B9H3-H1L3 | 0.529 | NT | NT |
| hz148B9H3-H1L4 | 0.844 | NT | NT |
| hz148B9H3-H2L1 | 0.501 | NT | NT |
| hz148B9H3-H2L2 | 0.459 | NT | NT |
| hz148B9H3-H2L3 | 0.523 | NT | NT |
| hz148B9H3-H2L4 | 0.577 | NT | NT |
| hz148B9H3-H3L1 | 0.402 | NT | NT |
| hz148B9H3-H3L2 | 0.414 | NT | NT |
| hz148B9H3-H3L3 | 0.519 | NT | NT |
| hz148B9H3-H3L4 | 0.634 | NT | NT |
| hz148B9H3-H4L1 | 0.471 | NT | NT |
| hz148B9H3-H4L2 | 0.876 | NT | NT |
| hz148B9H3-H4L3 | 0.587 | NT | NT |
| hz148B9H3-H4L4 | 0.738 | NT | NT |
| ch186D9A7 | 0.262 | 2.00 | 4.18 |
| hz186D9A7-H1L1 | 0.163 | 4.81 | 5.93 |
| hz186D9A7-H1L2 | 0.245 | 3.78 | 6.24 |
| hz186D9A7-H1L3 | 0.218 | 4.22 | 5.81 |
| hz186D9A7-H2L1 | 0.223 | 3.87 | 6.73 |
| hz186D9A7-H2L2 | 0.286 | 3.97 | 6.87 |
| hz186D9A7-H2L3 | 0.280 | 3.83 | 7.78 |
| hz186D9A7-H3L1 | 0.200 | 3.41 | 6.62 |
| hz186D9A7-H3L2 | 0.348 | 3.87 | 11.2 |
| hz186D9A7-H3L3 | 0.197 | 3.64 | 6.78 |
| hz186D9A7-H4L1 | 0.162 | 3.39 | 6.72 |
| hz186D9A7-H4L2 | 0.148 | 3.23 | 5.47 |
| hz186D9A7-H4L3 | 0.186 | 4.18 | 6.06 |
| hz186D9A7-H2L4 | 0.524 | NT | NT |
| hz186D9A7-H2L5 | 1.41 | NT | NT |
| hz186D9A7-H2L6 | 1.33 | NT | NT |
| hz186D9A7-H3L4 | 0.803 | NT | NT |
| hz186D9A7-H3L5 | 0.816 | NT | NT |
| hz186D9A7-H3L6 | 1.11 | NT | NT |
| hz186D9A7-H4L4 | 0.929 | NT | NT |
| hz186D9A7-H4L5 | 0.687 | NT | 3.06 |
| ch182C2D11 | 0.316 | 2.81 | 4.08 |
| hz182C2D11-H1L1 | 0.210 | 4.23 | 6.06 |
| hz182C2D11-H1L2 | 0.229 | 4.35 | 5.38 |
| hz182C2D11-H1L3 | 0.227 | 4.15 | 5.87 |
| hz182C2D11-H2L1 | 0.216 | 3.91 | 6.32 |
| hz182C2D11-H2L2 | 0.214 | 3.74 | 6.74 |
| hz182C2D11-H2L3 | 0.189 | 3.06 | 5.58 |
| hz182C2D11-H3L1 | 0.213 | 4.58 | 5.87 |
| hz182C2D11-H3L2 | 0.230 | 3.30 | 5.80 |
| hz182C2D11-H3L3 | 0.231 | 3.81 | 5.69 |
| hz182C2D11-H4L1 | 0.200 | 1.80 | 5.48 |
| hz182C2D11-H4L2 | 0.194 | 5.01 | 6.35 |
| hz182C2D11-H4L3 | 0.207 | 4.01 | 5.22 |
| hz182C2D11-H1L4 | 0.395 | NT | 14.2 |
| hz182C2D11-H1L5 | 0.654 | NT | NT |
| hz182C2D11-H2L4 | 0.677 | NT | NT |
| hz182C2D11-H2L5 | 0.941 | NT | NT |
| hz182C2D11-H2L6 | 0.517 | NT | 24.1 |
| hz182C2D11-H3L4 | 0.403 | NT | 12.0 |
| hz182C2D11-H3L5 | 0.967 | NT | NT |
| hz182C2D11-H3L6 | 0.693 | NT | NT |
| hz182C2D11-H4L4 | 0.585 | NT | 16.5 |
| hz182C2D11-H4L5 | 1.11 | NT | NT |
| hz182C2D11-H4L6 | 1.13 | NT | NT |
| hIgG1 | N/A | N/A | N/A |

| | | | |
|---|---|---|---|
| NT: not tested; N/A: not applicable. | | | |

### 2. Binding activity of anti-FOLR1 humanized antibodies to human FOLR2, human FOLR3, and human FOLR4

The non-specific binding activity of anti-human FOLR1 humanized antibodies to human FOLR2, human FOLR3, and human FOLR4 (IZUMO1) was detected by enzyme-linked immunosorbent assay (ELISA). High-adsorption 96-well microplates were coated with human FOLR2 protein (Acro, FO2-H5223), human FOLR3 protein (Acro, FO3-H52H3), or human FOLR4/IZUMO1 protein (Acro, FO4-H52H3), while the positive controls biotin-FOLR2, biotin-FOLR3, and biotin-FOLR4 were coated on high-adsorption 96-well microplates with FA-BSA (Cusabio, CSB-MC00271b0105), and incubated at 4°C overnight. The next day, blocking was performed with 1% BSA-PBS blocking solution at 37°C for 1 hour. 100 µL serially diluted antibody solutions or serially diluted biotin-labeled FOLR2, FOLR3, or FOLR4 solutions prepared in 1% BSA-PBST were added to each well, followed by incubation at 37°C for 1 hour. After three washes with 0.05% PBST, 100 µL of secondary antibody prepared in 1% BSA-PBST (Sigma, A8667 and BD, 554066) was added to each well; the secondary antibody used for the positive control was SA-HRP, and the secondary antibody used for other test antibodies was anti-human IgG HRP, followed by incubation at 37°C for 1 hour. After three washes with PBST, TMB substrate was added for color development (BD, 555214), and after the reaction was stopped with stop solution (Solarbio, C1058), absorbance values at 450 nm and 630 nm were measured on a microplate reader, and the OD450-OD630 values were fitted using a four-parameter logistic (4PL) model.

As shown in Figures 3A to 3C, all tested anti-FOLR1 humanized antibodies exhibited negligible non-specific cross-reactivity to FOLR2, FOLR3, or human FOLR4.

### 3. Blocking experiments of anti-FOLR1 humanized antibodies on the binding of FA-BSA to FOLR1

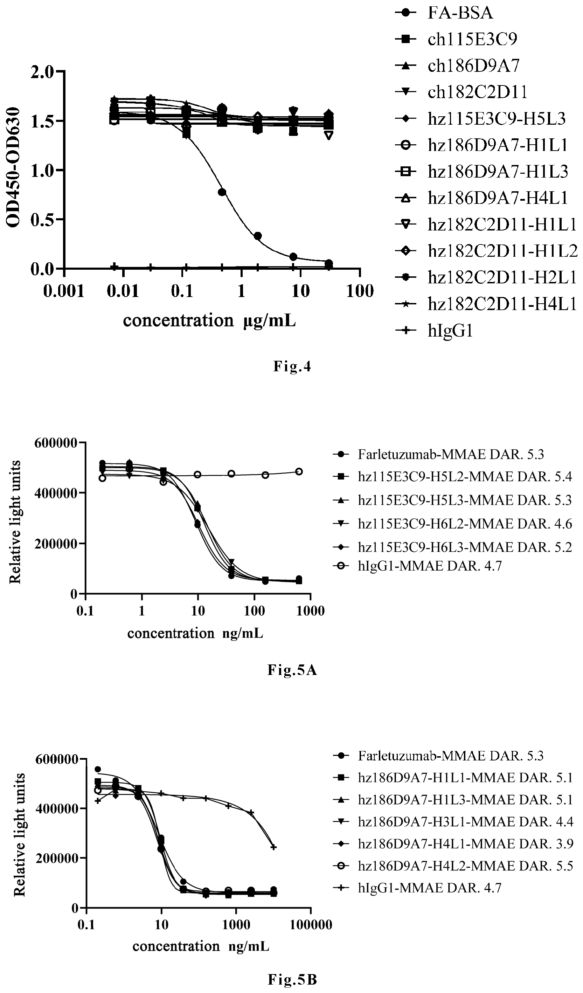

High-adsorption 96-well microplates were coated with FA-BSA protein and incubated at 4°C overnight. The next day, blocking was performed with 1% BSA-PBS at 37°C for 1 hour. Serially diluted FA-BSA solutions or serially diluted antibody solutions prepared in 1% BSA-PBST were respectively mixed 1:1 with biotin-labeled FOLR1 protein, and 100 µL of the antibody-protein mixture was added to each well of the blocked 96-well microplates, followed by incubation at 37°C for 1 hour. After four washes with PBST, 100 µL of Strep-HRP (BD, 554066, 1:5000) was added to each well, followed by incubation at 37°C for 1 hour. After four washes with PBST, color was developed with TMB, and after termination, absorbance values at 450 nm and 630 nm were measured on a microplate reader to calculate OD450-OD630, and the OD values were fitted using a four-parameter logistic (4PL) model. The results are shown in Figure 4, indicating that none of the tested antibodies blocked the binding of FA-BSA to FOLR1.

### 4. Cytotoxicity induced by αHFc-CL-MMAE and anti-human FOLR1 humanized antibodies

αHFc-CL-MMAE (Moradec, AH-102-AE) is an anti-human IgG Fc-specific antibody conjugated with MMAE via a cleavable linker. αHFc-CL-MMAE recognizes the Fc region of chimeric or humanized anti-human FOLR1 antibodies, causing internalization on FOLR1-expressing cells and intracellular release of MMAE to kill cells. KB cells or SK-OV-3 cells were obtained by trypsin digestion, and the cell density was adjusted to 4×10⁴ cells/mL. To each well of a white clear-bottom 96-well plate, 25 µL of serially diluted antibody solutions prepared in culture medium and 50 µL of cell suspension were added. After 10 minutes of incubation, 25 µL of αHFc-CL-MMAE working solution (Moradec, AH-202AE-50) prepared in culture medium was added to each well, and the 96-well plate was placed in a cell culture incubator at 37°C, 5% CO₂ for 3-7 days. Then, 50 µL of CTG was added to each well, and relative light units (RLU) were read on a microplate reader. EC₅₀ values for each antibody were calculated by fitting the RLU using a four-parameter logistic (4PL) model. The results are shown in Table 14. All tested anti-FOLR1 humanized antibodies exhibited strong killing activity against KB and/or SK-OV-3 cells, comparable to or better than the killing activity of Farletuzumab antibody.

**Table 14. cytotoxicity induced by αHFc-CL-MMAE and anti-FOLR1 humanized antibodies**

| Sample | KB cells EC₅₀, nM | SK-OV-3 cells EC₅₀, nM |
|---|---|---|
| Farletuzumab | 0.11 | 0.17 |
| ch115E3C9 | 0.019 | 0.075 |
| hz115E3C9-H5L2 | 0.027 | 0.088 |
| hz115E3C9-H5L3 | 0.022 | 0.078 |
| hz115E3C9-H6L2 | 0.022 | 0.094 |
| hz115E3C9-H6L3 | 0.025 | 0.083 |
| hz115E3C9-H6L4 | 0.032 | NT |
| ch186D9A7 | 0.049 | 0.034 |
| hz186D9A7-H1L1 | 0.020 | 0.052 |
| hz186D9A7-H1L3 | 0.021 | 0.054 |
| hz186D9A7-H3L1 | 0.030 | 0.070 |
| hz186D9A7-H3L3 | 0.032 | NT |
| hz186D9A7-H4L1 | 0.021 | 0.051 |
| hz186D9A7-H4L2 | 0.020 | 0.044 |
| hz186D9A7-H4L3 | 0.028 | NT |
| hz186D9A7-H2L4 | 0.079 | NT |
| hz186D9A7-H4L5 | 0.19 | NT |
| ch182C2D11 | 0.094 | 0.050 |
| hz182C2D11-H1L1 | 0.027 | 0.065 |
| hz182C2D11-H1L2 | 0.027 | 0.068 |
| hz182C2D11-H2L1 | 0.024 | 0.062 |
| hz182C2D11-H2L2 | 0.029 | NT |
| hz182C2D11-H2L3 | 0.029 | 0.059 |
| hz182C2D11-H4L1 | 0.024 | 0.059 |
| hz182C2D11-H4L2 | 0.042 | NT |
| hz182C2D11-H1L4 | 0.27 | NT |
| hz182C2D11-H2L6 | 0.14 | NT |
| hz182C2D11-H3L4 | 0.19 | NT |
| hz182C2D11-H4L4 | 0.22 | NT |
| ch148B9H3 | 0.060 | NT |
| hz148B9H3-H1L1 | 0.044 | NT |
| hz148B9H3-H1L2 | 0.040 | NT |
| hz148B9H3-H2L1 | 0.012 | NT |
| hz148B9H3-H2L2 | 0.075 | NT |
| hz148B9H3-H3L1 | 0.087 | NT |
| hz148B9H3-H3L2 | 0.048 | NT |
| hz148B9H3-H4L1 | 0.071 | NT |
| hIgG1 | N/A | N/A |

| | | |
|---|---|---|
| NT: not tested; N/A: no binding activity or poor binding activity; no valid EC₅₀ value was obtained. | | |

### Example 6. Preparation of conjugates of anti-human FOLR1 humanized antibodies with MMAE

Anti-human FOLR1 humanized antibody-MMAE conjugates were prepared following the procedure described in Section 3.1 of Example 3. Each conjugate (ADC) was named based on its parent antibody. Protein concentration and DAR value were measured, and subsequent biological functional characterization was performed.

### 6.1 Cytotoxicity assay of ADCs of anti-human FOLR1 humanized antibodies

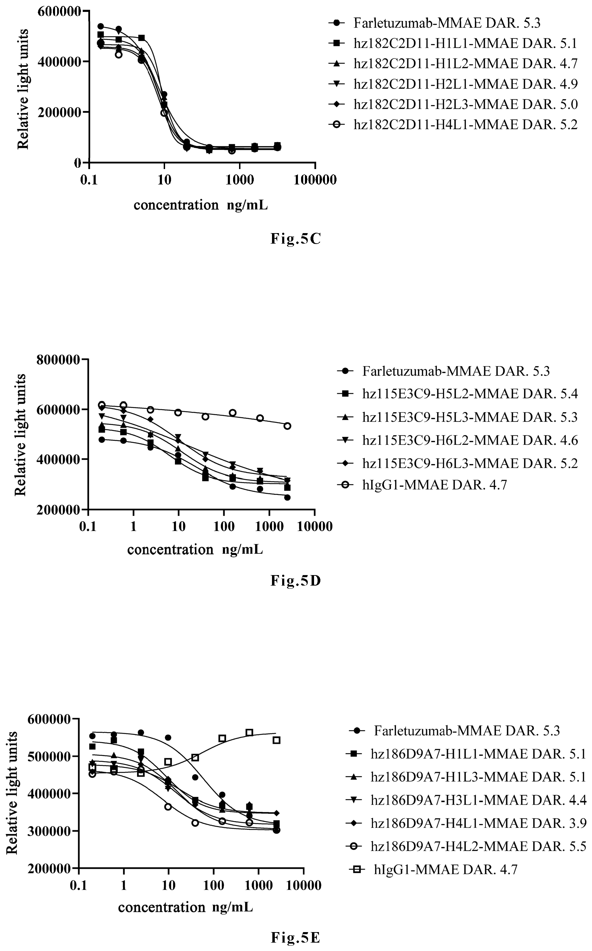

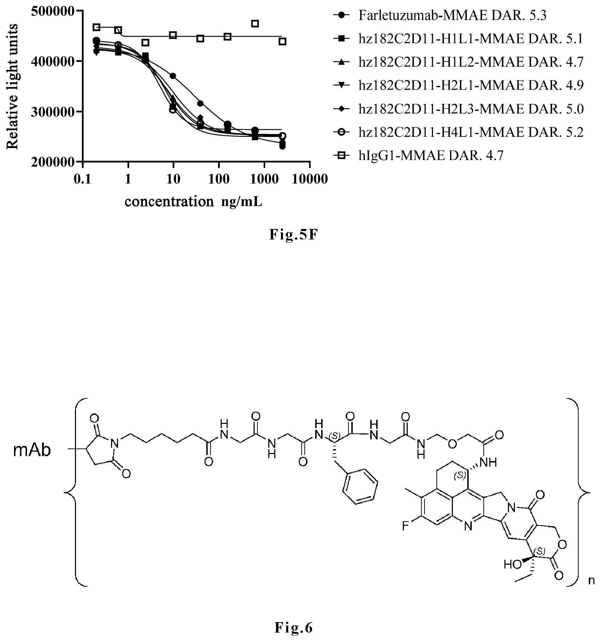

KB cells or SK-OV-3 cells were obtained by trypsin digestion, the cell concentration was adjusted to 4×10⁴ cells/mL, and cells were seeded at 50 µL/well in white clear-bottom 96-well plates. 50 µL serially diluted antibody solutions prepared in culture medium were added to each well of the 96-well plates, which were then placed in a 37°C, 5% CO₂ incubator for 3 days. Then, 50 µL of CTG reagent was added to each well, incubated at room temperature in the dark for 5 minutes, and relative light units were read on a microplate reader. Experimental data were analyzed using GraphPad Prism 8.0 software, with the logarithm of antibody concentration as the x-axis and the corresponding RLU value as the y-axis, using a four-parameter logistic (4PL) regression model to fit the antibody dose-response curve. The results are shown in Figure 5 and Table 15. All ADCs of the test antibodies exhibited strong killing activity against SK-OV-3 cells and KB cells, with cytotoxic potency comparable to or superior to the control Farletuzumab-MMAE

**Table 15. Cytotoxicity of conjugates of anti-FOLR1 humanized antibody and MMAE**

| Sample | SK-OV-3 EC50 ng/mL | KB EC50 ng/mL |
|---|---|---|
| Farletuzumab-MMAE DAR. 5.3 | 28.4 | 8.05 |
| hz115E3C9-H5L2-MMAE DAR. 5.4 | 6.28 | 12.9 |
| hz115E3C9-H5L3-MMAE DAR. 5.3 | 12.0 | 14.4 |
| hz115E3C9-H6L2-MMAE DAR. 4.6 | 17.4 | 15.5 |
| hz115E3C9-H6L3-MMAE DAR. 5.2 | 10.7 | 10.5 |
| hz186D9A7-H1L1-MMAE DAR. 5.1 | 6.75 | 9.25 |
| hz186D9A7-H1L3-MMAE DAR. 5.1 | 9.58 | 8.47 |
| hz186D9A7-H3L1-MMAE DAR. 4.4 | 7.92 | 7.62 |
| hz186D9A7-H4L1-MMAE DAR. 3.9 | 6.95 | 9.10 |
| hz186D9A7-H4L2-MMAE DAR. 5.5 | 4.94 | 8.25 |
| hz182C2D11-H1L1-MMAE DAR. 5.1 | 6.75 | 8.45 |
| hz182C2D11-H1L2-MMAE DAR. 4.7 | 9.58 | 9.66 |
| hz182C2D11-H2L1-MMAE DAR. 4.9 | 7.92 | 7.03 |
| hz182C2D11-H2L3-MMAE DAR. 5.0 | 6.95 | 7.93 |
| hz182C2D11-H4L1-MMAE DAR. 5.2 | 4.94 | 7.04 |
| hIgG1-MMAE DAR. 4.7 | N/A | N/A |

| | | |
|---|---|---|
| N/A: no binding activity or poor binding activity; no valid EC₅₀ value was obtained. | | |

### Example 7. Anti-human FOLR1 antibody ADCs inhibit tumor growth in mouse models

### 7.1 Preparation of conjugates of anti-human FOLR1 antibody and DXD

The antibody was added to PBS buffer to bring the antibody reaction concentration to 1.0 mg/mL. 500 mM EDTA (Hushi, 10009617) was added to bring the final EDTA concentration to 2 mM. TCEP solution (Thermo, 20490) was added to the reaction solution and mixed thoroughly (molar ratio TCEP:mAb = 6:1), followed by reaction at 37°C, 600 rpm for 2 hours to reduce the interchain disulfide bonds of the antibody. After the reaction was completed, the reaction solution was cooled to 4°C. Deruxtecan solution dissolved in DMSO (MCE, HY-1363E) was added to the reaction solution and mixed thoroughly (molar ratio Drug:mAb = 15:1), followed by reaction at 4°C, 600 rpm for 1 hour. The positive control Farletuzumab-DXD and negative control hIgG1-DXD were prepared using the same method. After the reaction was completed, the reaction solution was loaded into a 30 kDa ultrafiltration spin column, the solution was exchanged 6-8 column volumes with 10 mM histidine hydrochloride solution at pH 6.0, concentrated to an appropriate volume, loaded onto a pre-equilibrated desalting column, and centrifuged to obtain the purified ADC product. The final concentration of the ADC was determined using a Nanodrop microspectrophotometer, the purity of the ADC was determined by SEC-HPLC, and the DAR value of the ADC was determined by RP-HPLC/HIC-HPLC. The results are shown in Table 16. The structural schematic of anti-human FOLR1 antibody-DXD conjugate is shown in Figure 6.

**Table 16. Characterization Results of Anti-Human FOLR1 Humanized Antibody-DXD ADCs**

| Sample | Concentration (mg/mL) | Total amount (mg) | DAR value | Purity % |
|---|---|---|---|---|
| Farletuzumab-DXD | 1.6 | 13.6 | 7.2 | 97.93 |
| hz115E3C9-H5L3-DXD | 1.7 | 14.5 | 7.1 | 97.78 |
| hz186D9A7-H1L1-DXD | 0.90 | 8.60 | 7.5 | 99.33 |
| hz182C2D11-H2L1-DXD | 0.80 | 6.80 | 7.5 | 99.49 |
| hIgG1-DXD | 1.8 | 7.20 | 7.2 | 97.11 |

Full reduction of antibody interchain disulfide bonds theoretically produces a DAR of approximately 8, which represents the maximum drug-to-antibody ratio achievable via conventional cysteine interchain conjugation. The ADCs generated herein exhibit high DAR values, confirming successful production of site-specifically conjugated ADCs.

### 7.2 ADCs prepared by conjugating anti-human FOLR1 humanized antibodies and DXD inhibit the growth of KB (human oral epidermoid carcinoma) cells

Immunodeficient mice (SCID Beige, CB17.Cg-PrkdcscidLystbg-J/Crl, Beijing Vital River Laboratory Animal Technology Co., Ltd., strain code: 405) were used to establish a KB (human oral epidermoid carcinoma) animal model, and the anti-tumor efficacy of different ADC molecules prepared by conjugating anti-human FOLR1 antibodies with DXD was examined in this mouse tumor model. KB cells were diluted with serum-free medium (RPMI 1640), and a corresponding volume of Matrigel (Cat. No.: 354234, BD) was added and mixed with the cell suspension (RPMI 1640 medium:Matrigel = 7:3), and then 5×10⁶ cells were inoculated subcutaneously into the right flank of SCID Beige mice. When the average tumor volume reached approximately 150 mm³, mice were grouped at 5 mice per group, and the following test samples were administered by tail vein injection (i.v.) at a dosing volume of 10 mL/kg: vehicle (His-HCl buffer), hIgG1-DXD 5 mg/kg (Isotype), Farletuzumab-DXD 5 mg/kg (positive control), hz115E3C9-H5L3-DXD 5 mg/kg, hz186D9A7-H1L1-DXD 5 mg/kg, and hz182C2D11-H2L1-DXD 5 mg/kg, administered once. After dosing, the tumor-bearing mice were maintained normally, and the survival status of the tumor-bearing mice was observed. Body weight and tumor volume of the tumor-bearing mice were recorded every Tuesday and Friday, continuing for approximately 7 weeks. Mouse tumor volume measurement: Tumor length and width were measured using digital vernier calipers (500-151-30, Mitutoyo), and calculated according to the tumor volume formula: TV = 1/2 × L × W² mm³ (TV: tumor volume; L: tumor length; W: tumor width).

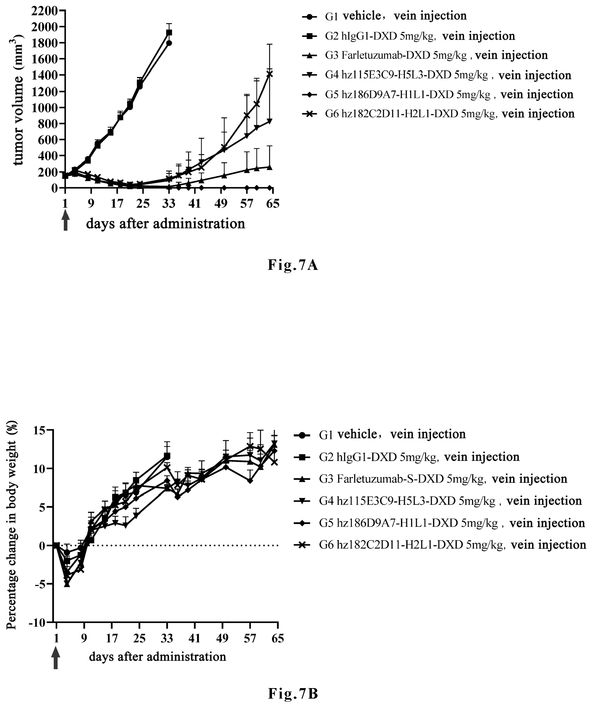

As shown in Figure 7, during the experimental observation period, the body weight of the tumor-bearing mice generally increased, indicating that the tumor-bearing mice had good tolerability to the anti-human FOLR1 antibody-DXD ADCs at this dose level. The test articles hz115E3C9-H5L3-DXD, hz186D9A7-H1L1-DXD, and hz182C2D11-H2L1-DXD all demonstrated significant anti-tumor effects after a single tail vein administration at a dose of 5 mg/kg. As the tumors continued to grow (up to day 64 post-dosing), the hz186D9A7-H1L1-DXD molecule exhibited the best efficacy (tumor regression rate 100%), followed by hz115E3C9-H5L3-DXD (tumor regression rate 80%), and then hz182C2D11-H2L1-DXD (tumor regression rate 60%).

### 7.3 Anti-Human FOLR1 Humanized Antibody-DXD ADCs Suppress Growth of NCI-H441 (Human Lung Adenocarcinoma) Tumors in Vivo

Immunodeficient mice (SCID Beige, CB17.Cg-PrkdcscidLystbg-J/Crl, Beijing Vital River Laboratory Animal Technology Co., Ltd., strain code: 405) were used to establish an NCI-H441 human lung adenocarcinoma animal model, and the anti-tumor efficacy of different ADC molecules prepared by conjugating anti-human FOLR1 antibodies and DXD was examined in this mouse model. NCI-H441 cells were diluted with serum-free medium (RPMI 1640), and a corresponding volume of Matrigel was added and mixed with the cell suspension (RPMI 1640 medium:Matrigel = 7:3), and then 5×10⁶ cells were inoculated subcutaneously into the right flank of SCID Beige mice. When the average tumor volume reached approximately 150 mm³, mice were randomly grouped at 5 mice per group, and the following were administered by tail vein injection at a dosing volume of 10 mL/kg: vehicle (His-HCl buffer), IgG1-DXD 5 mg/kg (Isotype), Farletuzumab-DXD 5 mg/kg (positive control), hz115E3C9-H5L3-DXD 1 mg/kg, hz115E3C9-H5L3-DXD 5 mg/kg, hz186D9A7-H1L1-DXD 1 mg/kg, hz186D9A7-H1L1-DXD 5 mg/kg, hz182C2D11-H2L1-DXD 1 mg/kg, and hz182C2D11-H2L1-DXD 5 mg/kg test samples, administered once. After dosing, the tumor-bearing mice were maintained normally, and the survival status of the tumor-bearing mice was observed. Body weight and tumor volume of the tumor-bearing mice were recorded every Tuesday and Friday. Mouse tumor volume measurement: Tumor length and width were measured using digital vernier calipers, and calculated according to the tumor volume formula: TV = 1/2 × L × W² mm³ (TV: tumor volume; L: tumor length; W: tumor width).

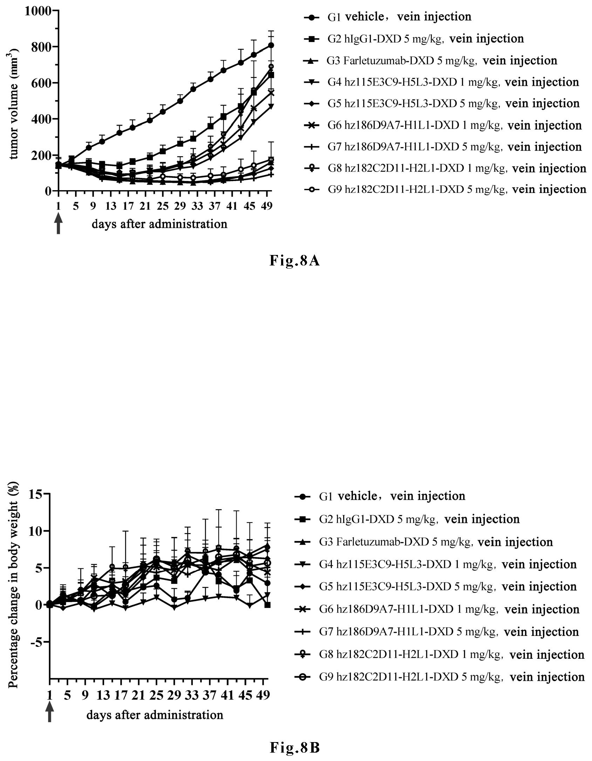

As shown in Figure 8, during the experimental observation period, the body weight of the tumor-bearing mice generally increased, indicating that the tumor-bearing mice had good tolerability to the conjugates of anti-human FOLR1 antibody and DXD at these dose levels. The test articles hz115E3C9-H5L3-DXD, hz186D9A7-H1L1-DXD, and hz182C2D11-H2L1-DXD all demonstrated significant anti-tumor effects after a single tail vein administration at doses of both 1 mg/kg and 5 mg/kg; meanwhile, the 5 mg/kg dose group was superior to the 1 mg/kg dose group, displaying evident dose-dependent anti-tumor activity.

### Sequences

| Ab name | description | sequences | SEQ ID NO: |
|---|---|---|---|
| hz115E 3C9-H5L2 | heavy chain | QVQLVQSGSELKKPGASVKVSCKASGSTFTNYGVNWVRQAPGQGLEWMGWM NTNTGEPTYAEDFKGRFVFSLDTSVSTAYLQISSLKAEDTAVYYCARMAMITGY FDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK | 65 |
| | light chain | DIQMTQSPSSLSASVGDRVTITCRASQVFSNYLNWYQQKPGKAPKLLILFTSRLH SGVPSRFSGSGSGTDFTLTISSLQPEDFATYYCQQGDTFPYTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 66 |
| hz115E 3C9-H5L3 | heavy chain | QVQLVQSGSELKKPGASVKVSCKASGSTFTNYGVNWVRQAPGQGLEWMGWM NTNTGEPTYAEDFKGRFVFSLDTSVSTAYLQISSLKAEDTAVYYCARMAMITGYF DYWGQGTMVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEAL HNHYTQKSLSLSPGK | 67 |
| | light chain | DIQMTQSPSSLSASVGDRVTITCRASQVFSNYLNWYQQKPGKAPKLLILFTSRLH SGVPSRFSGSGSGTDYTLTISSLQPEDFATYFCQQGDTFPYTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 68 |
| hz115E 3C9-H6L2 | heavy chain | QIQLVQSGSELKKPGASVKVSCKASGSTFTNYGVNWVRQAPGQGLEWMGWM NTNTGEPTYAEDFKGRFVFSLDTSVSTAYLQISSLKAEDTAVYFCARMAMITGY FDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK | 69 |
| | light chain | DIQMTQSPSSLSASVGDRVTITCRASQVFSNYLNWYQQKPGKAPKLLILFTSRLH SGVPSRFSGSGSGTDFTLTISSLQPEDFATYYCQQGDTFPYTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 70 |
| hz115E 3C9-H6L3 | heavy chain | QIQLVQSGSELKKPGASVKVSCKASGSTFTNYGVNWVRQAPGQGLEWMGWM NTNTGEPTYAEDFKGRFVFSLDTSVSTAYLQISSLKAEDTAVYFCARMAMITGY FDYWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK | 71 |
| | light chain | DIQMTQSPSSLSASVGDRVTITCRASQVFSNYLNWYQQKPGKAPKLLILFTSRLH SGVPSRFSGSGSGTDYTLTISSLQPEDFATYFCQQGDTFPYTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC | 72 |
| hz186 D9A7-H1L1 | heavy chain | QVQLVQSGAEVKKPGSSVKVSCKASGGTFSSYWMHWVRQAPGQGLEWMGTIY PGNSDTKYSQKFRDRVTITADTSTSTAYMELSSLRSEDTAVYYCTRRDGSWFAY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA | 73 |
| | | LHNHYTQKSLSLSPGK | |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 74 |
| hz186 D9A7-H1L3 | heavy chain | QVQLVQSGAEVKKPGSSVKVSCKASGGTFSSYWMHWVRQAPGQGLEWMGTIY PGNSDTKYSQKFRDRVTITADTSTSTAYMELSSLRSEDTAVYYCTRRDGSWFAY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA LHNHYTQKSLSLSPGK | 75 |
| | light chain | DIQMTQSPSSLSASVGDRVTITCRASENIYDNLAWYQQKPGKSPKLLVYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 76 |
| hz186 D9A7-H3L1 | heavy chain | QVQLVQSGAEVKKPGSSVKVSCKASGGTFSSYWMHWVRQAPGQGLEWIATIYP GNSDTKYSQKFRDRATLTADTSTSTAYMELSSLRSEDTAVYYCTRRDGSWFAY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA LHNHYTQKSLSLSPGK | 77 |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 78 |
| hz186 D9A7-H4L1 | heavy chain | QVQLVQSGAEVKKPGSSVKVSCKASGGSFSSYWMHWIRQAPGQGLEWIATIYP GNSDTKYSQKFRDRATLTADTSTSTAYMELSSLRSEDTAVYYCTRRDGSWFAY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA LHNHYTQKSLSLSPGK | 79 |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 80 |
| hz186 D9A7-H4L2 | heavy chain | QVQLVQSGAEVKKPGSSVKVSCKASGGSFSSYWMHWIRQAPGQGLEWIATIYP GNSDTKYSQKFRDRATLTADTSTSTAYMELSSLRSEDTAVYYCTRRDGSWFAY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA LHNHYTQKSLSLSPGK | 81 |
| | light chain | DIQMTQSPSSLSASVGDRVTITCRASENIYDNLAWYQQKPGKAPKLLVYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 82 |
| hz182C 2D11-H1L1 | heavy chain | QVQLVQSGAEVKKPGSSVKVSCKASGGTFSNYWMHWVRQAPGQGLEWMGAI YPGNSDTNYNQKFRDRVTITADTSTSTAYMELSSLRSEDTAVYYCTRRDGSWFA YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM | 83 |
| | | HEALHNHYTQKSLSLSPGK | |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 84 |
| hz182C 2D11-H1L2 | heavy chain | QVQLVQSGAEVKKPGSSVKVSCKASGGTFSNYWMHWVRQAPGQGLEWMGAI YPGNSDTNYNQKFRDRVTITADTSTSTAYMELSSLRSEDTAVYYCTRRDGSWFA YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPGK | 85 |
| | light chain | DIQMTQSPSSLSASVGDRVTITCRASENIYDNLAWYQQKPGKAPKLLVYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 86 |
| hz182C 2D11-H2L1 | heavy chain | QVQLVQSGAEVKKPGSSVKVSCKASGGTFSNYWMHWVRQAPGQGLEWMAAI YPGNSDTNYNQKFRDRATITADTSTSTAYMELSSLRSEDTAVYYCTRRDGSWFA YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPGK | 87 |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 88 |
| hz182C 2D11-H2L3 | heavy chain | QVQLVQSGAEVKKPGSSVKVSCKASGGTFSNYWMHWVRQAPGQGLEWMAAI YPGNSDTNYNQKFRDRATITADTSTSTAYMELSSLRSEDTAVYYCTRRDGSWFA YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPGK | 89 |
| | light chain | DIQMTQSPSSLSASVGDRVTITCRASENIYDNLAWYQQKPGKSPKLLVYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 90 |
| hz182C 2D11-H4L1 | heavy chain | QAQLVQSGAEVKKPGSSVKVSCKASGGSFSNYWMHWVRQAPGQGLEWIAAIY PGNSDTNYNQKFRDRATLTADTSTSTAYMELSSLRSEDTAVYYCTRRDGSWFA YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPGK | 91 |
| | light chain | DIQMTQSPSSLSASVGDRVTITCRASENIYDNLAWYQQKPGKAPKLLLYDATNL ADGVPSRFSGSGSGTDYTLTISSLQPEDFATYYCQHFWNTPPTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | 92 |
| Farletu zumab | heavy chain | EVQLVESGGGVVQPGRSLRLSCSASGFTFSGYGLSWVRQAPGKGLEWVAMI SSGGSYTYYADSVKGRFAISRDNAKNTLFLQMDSLRPEDTGVYFCARHGDD PAWFAYWGQGTPVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVD | 93 |
| | | KSRWQQGNVFSCSVMHEALHNHYTQKSLSLSPGK | |
| | light chain | DIQLTQSPSSLSASVGDRVTITCSVSSSISSNNLHWYQQKPGKAPKPWIYGTS NLASGVPSRFSGSGSGTDYTFTISSLQPEDIATYYCQQWSSYPYMYTFGQGT KVEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVT KSFNRGEC | 94 |
| Farletu zumab-mIgG | heavy chain | EVQLVESGGGVVQPGRSLRLSCSASGFTFSGYGLSWVRQAPGKGLEWVAMI SSGGSYTYYADSVKGRFAISRDNAKNTLFLQMDSLRPEDTGVYFCARHGDD PAWFAYWGQGTPVTVSSAKTTAPSVYPLAPVCGDTTGSSVTLGCLVKGYFPE PVTLTWNSGSLSSGVHTFPAVLQSDLYTLSSSVTVTSSTWPSQSITCNVAHPAS STKVDKKIEPRGPTIKPCPPCKCPAPNLLGGPSVFIFPPKIKDVLMISLSPIVTC VVVDVSEDDPDVQISWFVNNVEVHTAQTQTHREDYNSTLRVVSALPIQHQD WMSGKEFKCKVNNKDLPAPIERTISKPKGSVRAPQVYVLPPPEEEMTKKQV TLTCMVTDFMPEDIYVEWTNNGKTELNYKNTEPVLDSDGSYFMYSKLRVEK KNWVERNSYSCSVVHEGLHNHHTTKSFSRTPGK | 95 |
| | light chain | DIQLTQSPSSLSASVGDRVTITCSVSSSISSNNLHWYQQKPGKAPKPWIYGTS NLASGVPSRFSGSGSGTDYTFTISSLQPEDIATYYCQQWSSYPYMYTFGQGT KVEIKRADAAPTVSIFPPSSEQLTSGGASVVCFLNNFYPKDINVKWKIDGSER QNGVLNSWTDQDSKDSTYSMSSTLTLTKDEYERHNSYTCEATHKTSTSPIVK SFNRNEC | 96 |

## Claims

1. An anti-FOLR1 antibody or antigen-binding fragment thereof that specifically binds FOLR1, comprising:
1) three heavy chain CDRs (HCDR1, HCDR2, HCDR3) comprised in the heavy chain variable region as set forth in SEQ ID NO: 4 and/or three light chain CDRs (LCDR1, LCDR2, LCDR3) comprised in the light chain variable region as set forth in SEQ ID NO: 8;
2) three heavy chain CDRs (HCDR1, HCDR2, HCDR3) comprised in the heavy chain variable region as set forth in SEQ ID NO: 12 and/or three light chain CDRs (LCDR1, LCDR2, LCDR3) comprised in the light chain variable region as set forth in SEQ ID NO: 16;
3) three heavy chain CDRs (HCDR1, HCDR2, HCDR3) comprised in the heavy chain variable region as set forth in SEQ ID NO: 20 and/or three light chain CDRs (LCDR1, LCDR2, LCDR3) comprised in the light chain variable region as set forth in SEQ ID NO: 24; or
4) three heavy chain CDRs (HCDR1, HCDR2, HCDR3) comprised in the heavy chain variable region as set forth in SEQ ID NO: 28 and/or three light chain CDRs (LCDR1, LCDR2, LCDR3) comprised in the light chain variable region as set forth in SEQ ID NO: 32.

2. An anti-FOLR1 antibody or antigen-binding fragment thereof that specifically binds FOLR1, comprising:
1) HCDR1, HCDR2, HCDR3 comprising the sequences as set forth in SEQ ID NOs: 1, 2 and 3; and/or LCDR1, LCDR2, LCDR3 comprising the sequences as set forth in SEQ ID NOs: 5, 6 and 7;
2) HCDR1, HCDR2, HCDR3 comprising the sequences as set forth in SEQ ID NOs: 9, 10 and 11; and/or LCDR1, LCDR2, LCDR3 comprising the sequences as set forth in SEQ ID NOs: 13, 14 and 15;
3) HCDR1, HCDR2, HCDR3 comprising the sequences as set forth in SEQ ID NOs: 17, 18 and 19; and/or LCDR1, LCDR2, LCDR3 comprising the sequences as set forth in SEQ ID NOs: 21, 22 and 23; or
4) HCDR1, HCDR2, HCDR3 comprising the sequences as set forth in SEQ ID NOs: 25, 26 and 27; and/or LCDR1, LCDR2, LCDR3 comprising the sequences as set forth in SEQ ID NOs: 29, 30 and 31.

3. The anti-FOLR1 antibody or antigen-binding fragment thereof according to claim 1 or 2, comprising a heavy chain variable region and/or a light chain variable region, wherein:
1) the heavy chain variable region comprises the amino acid sequence as set forth in SEQ ID NO: 4, or an amino acid sequence comprising one or more amino acid substitutions (e.g., conservative substitutions), deletions or insertions, or any combination thereof, relative to SEQ ID NO: 4, or an amino acid sequence having at least 90%, 91%, 92%, 93%, 94%, 95%, 96%, 97%, 98% or 99% identity to the amino acid sequence of SEQ ID NO: 4, or consists of SEQ ID NO: 4, and the light chain variable region comprises the amino acid sequence as set forth in SEQ ID NO: 8, or an amino acid sequence comprising one or more amino acid substitutions (e.g., conservative substitutions), deletions or insertions, or any combination thereof, relative to SEQ ID NO: 8, or an amino acid sequence having at least 90%, 91%, 92%, 93%, 94%, 95%, 96%, 97%, 98% or 99% identity to the amino acid sequence of SEQ ID NO: 8, or consists of SEQ ID NO: 8;
2) the heavy chain variable region comprises the amino acid sequence as set forth in SEQ ID NO: 12, or an amino acid sequence comprising one or more amino acid substitutions (e.g., conservative substitutions), deletions or insertions, or any combination thereof, relative to SEQ ID NO: 12, or an amino acid sequence having at least 90%, 91%, 92%, 93%, 94%, 95%, 96%, 97%, 98% or 99% identity to the amino acid sequence of SEQ ID NO: 12, or consists of SEQ ID NO: 12, and the light chain variable region comprises the amino acid sequence as set forth in SEQ ID NO: 16, or an amino acid sequence comprising one or more amino acid substitutions (e.g., conservative substitutions), deletions or insertions, or any combination thereof, relative to SEQ ID NO: 16, or an amino acid sequence having at least 90%, 91%, 92%, 93%, 94%, 95%, 96%, 97%, 98% or 99% identity to the amino acid sequence of SEQ ID NO: 16, or consists of SEQ ID NO: 16;
3) the heavy chain variable region comprises the amino acid sequence as set forth in SEQ ID NO: 20, or an amino acid sequence comprising one or more amino acid substitutions (e.g., conservative substitutions), deletions or insertions, or any combination thereof, relative to SEQ ID NO: 20, or an amino acid sequence having at least 90%, 91%, 92%, 93%, 94%, 95%, 96%, 97%, 98% or 99% identity to the amino acid sequence of SEQ ID NO: 20, or consists of SEQ ID NO: 20, and the light chain variable region comprises the amino acid sequence as set forth in SEQ ID NO: 24, or an amino acid sequence comprising one or more amino acid substitutions (e.g., conservative substitutions), deletions or insertions, or any combination thereof, relative to SEQ ID NO: 24, or an amino acid sequence having at least 90%, 91%, 92%, 93%, 94%, 95%, 96%, 97%, 98% or 99% identity to the amino acid sequence of SEQ ID NO: 24, or consists of SEQ ID NO: 24; or
4) the heavy chain variable region comprises the amino acid sequence as set forth in SEQ ID NO: 28, or an amino acid sequence comprising one or more amino acid substitutions (e.g., conservative substitutions), deletions or insertions, or any combination thereof, relative to SEQ ID NO: 28, or an amino acid sequence having at least 90%, 91%, 92%, 93%, 94%, 95%, 96%, 97%, 98% or 99% identity to the amino acid sequence of SEQ ID NO: 28, or consists of SEQ ID NO: 28, and the light chain variable region comprises the amino acid sequence as set forth in SEQ ID NO: 32, or an amino acid sequence comprising one or more amino acid substitutions (e.g., conservative substitutions), deletions or insertions, or any combination thereof, relative to SEQ ID NO: 32, or an amino acid sequence having at least 90%, 91%, 92%, 93%, 94%, 95%, 96%, 97%, 98% or 99% identity to the amino acid sequence of SEQ ID NO: 32, or consists of SEQ ID NO: 32;
5) the heavy chain variable region comprises the amino acid sequence as set forth in SEQ ID NO: 35, or an amino acid sequence comprising one or more amino acid substitutions (e.g., conservative substitutions), deletions or insertions, or any combination thereof, relative to SEQ ID NO: 35, or an amino acid sequence having at least 90%, 91%, 92%, 93%, 94%, 95%, 96%, 97%, 98% or 99% identity to the amino acid sequence of SEQ ID NO: 35, or consists of SEQ ID NO: 35, and the light chain variable region comprises the amino acid sequence as set forth in SEQ ID NO: 39, or an amino acid sequence comprising one or more amino acid substitutions (e.g., conservative substitutions), deletions or insertions, or any combination thereof, relative to SEQ ID NO: 39, or an amino acid sequence having at least 90%, 91%, 92%, 93%, 94%, 95%, 96%, 97%, 98% or 99% identity to the amino acid sequence of SEQ ID NO: 39, or consists of SEQ ID NO: 39;
6) the heavy chain variable region comprises the amino acid sequence as set forth in SEQ ID NO: 35, or an amino acid sequence comprising one or more amino acid substitutions (e.g., conservative substitutions), deletions or insertions, or any combination thereof, relative to SEQ ID NO: 35, or an amino acid sequence having at least 90%, 91%, 92%, 93%, 94%, 95%, 96%, 97%, 98% or 99% identity to the amino acid sequence of SEQ ID NO: 35, or consists of SEQ ID NO: 35, and the light chain variable region comprises the amino acid sequence as set forth in SEQ ID NO: 40, or an amino acid sequence comprising one or more amino acid substitutions (e.g., conservative substitutions), deletions or insertions, or any combination thereof, relative to SEQ ID NO: 40, or an amino acid sequence having at least 90%, 91%, 92%, 93%, 94%, 95%, 96%, 97%, 98% or 99% identity to the amino acid sequence of SEQ ID NO: 40, or consists of SEQ ID NO: 40;
7) the heavy chain variable region comprises the amino acid sequence as set forth in SEQ ID NO: 35, or an amino acid sequence comprising one or more amino acid substitutions (e.g., conservative substitutions), deletions or insertions, or any combination thereof, relative to SEQ ID NO: 35, or an amino acid sequence having at least 90%, 91%, 92%, 93%, 94%, 95%, 96%, 97%, 98% or 99% identity to the amino acid sequence of SEQ ID NO: 35, or consists of SEQ ID NO: 35, and the light chain variable region comprises the amino acid sequence as set forth in SEQ ID NO: 41, or an amino acid sequence comprising one or more amino acid substitutions (e.g., conservative substitutions), deletions or insertions, or any combination thereof, relative to SEQ ID NO: 41, or an amino acid sequence having at least 90%, 91%, 92%, 93%, 94%, 95%, 96%, 97%, 98% or 99% identity to the amino acid sequence of SEQ ID NO: 41, or consists of SEQ ID NO: 41;
8) the heavy chain variable region comprises the amino acid sequence as set forth in SEQ ID NO: 35, or an amino acid sequence comprising one or more amino acid substitutions (e.g., conservative substitutions), deletions or insertions, or any combination thereof, relative to SEQ ID NO: 35, or an amino acid sequence having at least 90%, 91%, 92%, 93%, 94%, 95%, 96%, 97%, 98% or 99% identity to the amino acid sequence of SEQ ID NO: 35, or consists of SEQ ID NO: 35, and the light chain variable region comprises the amino acid sequence as set forth in SEQ ID NO: 42, or an amino acid sequence comprising one or more amino acid substitutions (e.g., conservative substitutions), deletions or insertions, or any combination thereof, relative to SEQ ID NO: 42, or an amino acid sequence having at least 90%, 91%, 92%, 93%, 94%, 95%, 96%, 97%, 98% or 99% identity to the amino acid sequence of SEQ ID NO: 42, or consists of SEQ ID NO: 42;
9) the heavy chain variable region comprises the amino acid sequence as set forth in SEQ ID NO: 36, or an amino acid sequence comprising one or more amino acid substitutions (e.g., conservative substitutions), deletions or insertions, or any combination thereof, relative to SEQ ID NO: 36, or an amino acid sequence having at least 90%, 91%, 92%, 93%, 94%, 95%, 96%, 97%, 98% or 99% identity to the amino acid sequence of SEQ ID NO: 36, or consists of SEQ ID NO: 36, and the light chain variable region comprises the amino acid sequence as set forth in SEQ ID NO: 39, or an amino acid sequence comprising one or more amino acid substitutions (e.g., conservative substitutions), deletions or insertions, or any combination thereof, relative to SEQ ID NO: 39, or an amino acid sequence having at least 90%, 91%, 92%, 93%, 94%, 95%, 96%, 97%, 98% or 99% identity to the amino acid sequence of SEQ ID NO: 39, or consists of SEQ ID NO: 39;
10) the heavy chain variable region comprises the amino acid sequence as set forth in SEQ ID NO: 36, or an amino acid sequence comprising one or more amino acid substitutions (e.g., conservative substitutions), deletions or insertions, or any combination thereof, relative to SEQ ID NO: 36, or an amino acid sequence having at least 90%, 91%, 92%, 93%, 94%, 95%, 96%, 97%, 98% or 99% identity to the amino acid sequence of SEQ ID NO: 36, or consists of SEQ ID NO: 36, and the light chain variable region comprises the amino acid sequence as set forth in SEQ ID NO: 40, or an amino acid sequence comprising one or more amino acid substitutions (e.g., conservative substitutions), deletions or insertions, or any combination thereof, relative to SEQ ID NO: 40, or an amino acid sequence having at least 90%, 91%, 92%, 93%, 94%, 95%, 96%, 97%, 98% or 99% identity to the amino acid sequence of SEQ ID NO: 40, or consists of SEQ ID NO: 40;
11) the heavy chain variable region comprises the amino acid sequence as set forth in SEQ ID NO: 36, or an amino acid sequence comprising one or more amino acid substitutions (e.g., conservative substitutions), deletions or insertions, or any combination thereof, relative to SEQ ID NO: 36, or an amino acid sequence having at least 90%, 91%, 92%, 93%, 94%, 95%, 96%, 97%, 98% or 99% identity to the amino acid sequence of SEQ ID NO: 36, or consists of SEQ ID NO: 36, and the light chain variable region comprises the amino acid sequence as set forth in SEQ ID NO: 41, or an amino acid sequence comprising one or more amino acid substitutions (e.g., conservative substitutions), deletions or insertions, or any combination thereof, relative to SEQ ID NO: 41, or an amino acid sequence having at least 90%, 91%, 92%, 93%, 94%, 95%, 96%, 97%, 98% or 99% identity to the amino acid sequence of SEQ ID NO: 41, or consists of SEQ ID NO: 41;
12) the heavy chain variable region comprises the amino acid sequence as set forth in SEQ ID NO: 36, or an amino acid sequence comprising one or more amino acid substitutions (e.g., conservative substitutions), deletions or insertions, or any combination thereof, relative to SEQ ID NO: 36, or an amino acid sequence having at least 90%, 91%, 92%, 93%, 94%, 95%, 96%, 97%, 98% or 99% identity to the amino acid sequence of SEQ ID NO: 36, or consists of SEQ ID NO: 36, and the light chain variable region comprises the amino acid sequence as set forth in SEQ ID NO: 42, or an amino acid sequence comprising one or more amino acid substitutions (e.g., conservative substitutions), deletions or insertions, or any combination thereof, relative to SEQ ID NO: 42, or an amino acid sequence having at least 90%, 91%, 92%, 93%, 94%, 95%, 96%, 97%, 98% or 99% identity to the amino acid sequence of SEQ ID NO: 42, or consists of SEQ ID NO: 42;
13) the heavy chain variable region comprises the amino acid sequence as set forth in SEQ ID NO: 37, or an amino acid sequence comprising one or more amino acid substitutions (e.g., conservative substitutions), deletions or insertions, or any combination thereof, relative to SEQ ID NO: 37, or an amino acid sequence having at least 90%, 91%, 92%, 93%, 94%, 95%, 96%, 97%, 98% or 99% identity to the amino acid sequence of SEQ ID NO: 37, or consists of SEQ ID NO: 37, and the light chain variable region comprises the amino acid sequence as set forth in SEQ ID NO: 39, or an amino acid sequence comprising one or more amino acid substitutions (e.g., conservative substitutions), deletions or insertions, or any combination thereof, relative to SEQ ID NO: 39, or an amino acid sequence having at least 90%, 91%, 92%, 93%, 94%, 95%, 96%, 97%, 98% or 99% identity to the amino acid sequence of SEQ ID NO: 39, or consists of SEQ ID NO: 39;
14) the heavy chain variable region comprises the amino acid sequence as set forth in SEQ ID NO: 37, or an amino acid sequence comprising one or more amino acid substitutions (e.g., conservative substitutions), deletions or insertions, or any combination thereof, relative to SEQ ID NO: 37, or an amino acid sequence having at least 90%, 91%, 92%, 93%, 94%, 95%, 96%, 97%, 98% or 99% identity to the amino acid sequence of SEQ ID NO: 37, or consists of SEQ ID NO: 37, and the light chain variable region comprises the amino acid sequence as set forth in SEQ ID NO: 40, or an amino acid sequence comprising one or more amino acid substitutions (e.g., conservative substitutions), deletions or insertions, or any combination thereof, relative to SEQ ID NO: 40, or an amino acid sequence having at least 90%, 91%, 92%, 93%, 94%, 95%, 96%, 97%, 98% or 99% identity to the amino acid sequence of SEQ ID NO: 40, or consists of SEQ ID NO: 40;
15) the heavy chain variable region comprises the amino acid sequence as set forth in SEQ ID NO: 37, or an amino acid sequence comprising one or more amino acid substitutions (e.g., conservative substitutions), deletions or insertions, or any combination thereof, relative to SEQ ID NO: 37, or an amino acid sequence having at least 90%, 91%, 92%, 93%, 94%, 95%, 96%, 97%, 98% or 99% identity to the amino acid sequence of SEQ ID NO: 37, or consists of SEQ ID NO: 37, and the light chain variable region comprises the amino acid sequence as set forth in SEQ ID NO: 41, or an amino acid sequence comprising one or more amino acid substitutions (e.g., conservative substitutions), deletions or insertions, or any combination thereof, relative to SEQ ID NO: 41, or an amino acid sequence having at least 90%, 91%, 92%, 93%, 94%, 95%, 96%, 97%, 98% or 99% identity to the amino acid sequence of SEQ ID NO: 41, or consists of SEQ ID NO: 41;
16) the heavy chain variable region comprises the amino acid sequence as set forth in SEQ ID NO: 37, or an amino acid sequence comprising one or more amino acid substitutions (e.g., conservative substitutions), deletions or insertions, or any combination thereof, relative to SEQ ID NO: 37, or an amino acid sequence having at least 90%, 91%, 92%, 93%, 94%, 95%, 96%, 97%, 98% or 99% identity to the amino acid sequence of SEQ ID NO: 37, or consists of SEQ ID NO: 37, and the light chain variable region comprises the amino acid sequence as set forth in SEQ ID NO: 42, or an amino acid sequence comprising one or more amino acid substitutions (e.g., conservative substitutions), deletions or insertions, or any combination thereof, relative to SEQ ID NO: 42, or an amino acid sequence having at least 90%, 91%, 92%, 93%, 94%, 95%, 96%, 97%, 98% or 99% identity to the amino acid sequence of SEQ ID NO: 42, or consists of SEQ ID NO: 42;
17) the heavy chain variable region comprises the amino acid sequence as set forth in SEQ ID NO: 51, or an amino acid sequence comprising one or more amino acid substitutions (e.g., conservative substitutions), deletions or insertions, or any combination thereof, relative to SEQ ID NO: 51, or an amino acid sequence having at least 90%, 91%, 92%, 93%, 94%, 95%, 96%, 97%, 98% or 99% identity to the amino acid sequence of SEQ ID NO: 51, or consists of SEQ ID NO: 51, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
18) the heavy chain variable region comprises the amino acid sequence as set forth in SEQ ID NO: 51, or an amino acid sequence comprising one or more amino acid substitutions (e.g., conservative substitutions), deletions or insertions, or any combination thereof, relative to SEQ ID NO: 51, or an amino acid sequence having at least 90%, 91%, 92%, 93%, 94%, 95%, 96%, 97%, 98% or 99% identity to the amino acid sequence of SEQ ID NO: 51, or consists of SEQ ID NO: 51, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
19) the heavy chain variable region comprises the amino acid sequence as set forth in SEQ ID NO: 51, or an amino acid sequence comprising one or more amino acid substitutions (e.g., conservative substitutions), deletions or insertions, or any combination thereof, relative to SEQ ID NO: 51, or an amino acid sequence having at least 90%, 91%, 92%, 93%, 94%, 95%, 96%, 97%, 98% or 99% identity to the amino acid sequence of SEQ ID NO: 51, or consists of SEQ ID NO: 51, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
20) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
21) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
22) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
23) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
24) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
25) the heavy chain variable region comprises the amino acid sequence as set forth in SEQ ID NO: 52, or an amino acid sequence comprising one or more amino acid substitutions (e.g., conservative substitutions), deletions or insertions, or any combination thereof, relative to SEQ ID NO: 52, or an amino acid sequence having at least 90%, 91%, 92%, 93%, 94%, 95%, 96%, 97%, 98% or 99% identity to the amino acid sequence of SEQ ID NO: 52, or consists of SEQ ID NO: 52, and the light chain variable region comprises the amino acid sequence as set forth in SEQ ID NO: 60, or an amino acid sequence comprising one or more amino acid substitutions (e.g., conservative substitutions), deletions or insertions, or any combination thereof, relative to SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;
26) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
27) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
28) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
29) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
30) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
31) the heavy chain variable region comprises the amino acid sequence as set forth in SEQ ID NO: 53, or an amino acid sequence comprising one or more amino acid substitutions (e.g., conservative substitutions), deletions or insertions, or any combination thereof, relative to SEQ ID NO: 53, or an amino acid sequence having at least 90%, 91%, 92%, 93%, 94%, 95%, 96%, 97%, 98% or 99% identity to the amino acid sequence of SEQ ID NO: 53, or consists of SEQ ID NO: 53, and the light chain variable region comprises the amino acid sequence as set forth in SEQ ID NO: 60, or an amino acid sequence comprising one or more amino acid substitutions (e.g., conservative substitutions), deletions or insertions, or any combination thereof, relative to SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;
32) the heavy chain variable region comprises the amino acid sequence as set forth in SEQ ID NO: 54, or an amino acid sequence comprising one or more amino acid substitutions (e.g., conservative substitutions), deletions or insertions, or any combination thereof, relative to SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
33) the heavy chain variable region comprises the amino acid sequence as set forth in SEQ ID NO: 54, or an amino acid sequence comprising one or more amino acid substitutions (e.g., conservative substitutions), deletions or insertions, or any combination thereof, relative to SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
34) the heavy chain variable region comprises the amino acid sequence as set forth in SEQ ID NO: 54, or an amino acid sequence comprising one or more amino acid substitutions (e.g., conservative substitutions), deletions or insertions, or any combination thereof, relative to SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
35) the heavy chain variable region comprises the amino acid sequence as set forth in SEQ ID NO: 54, or an amino acid sequence comprising one or more amino acid substitutions (e.g., conservative substitutions), deletions or insertions, or any combination thereof, relative to SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
36) the heavy chain variable region comprises the amino acid sequence as set forth in SEQ ID NO: 54, or an amino acid sequence comprising one or more amino acid substitutions (e.g., conservative substitutions), deletions or insertions, or any combination thereof, relative to SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
37) the heavy chain variable region comprises the amino acid sequence as set forth in SEQ ID NO: 61, or an amino acid sequence comprising one or more amino acid substitutions (e.g., conservative substitutions), deletions or insertions, or any combination thereof, relative to SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
38) the heavy chain variable region comprises the amino acid sequence as set forth in SEQ ID NO: 61, or an amino acid sequence comprising one or more amino acid substitutions (e.g., conservative substitutions), deletions or insertions, or any combination thereof, relative to SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
39) the heavy chain variable region comprises the amino acid sequence as set forth in SEQ ID NO: 61, or an amino acid sequence comprising one or more amino acid substitutions (e.g., conservative substitutions), deletions or insertions, or any combination thereof, relative to SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
40) the heavy chain variable region comprises the amino acid sequence as set forth in SEQ ID NO: 61, or an amino acid sequence comprising one or more amino acid substitutions (e.g., conservative substitutions), deletions or insertions, or any combination thereof, relative to SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
41) the heavy chain variable region comprises the amino acid sequence as set forth in SEQ ID NO: 61, or an amino acid sequence comprising one or more amino acid substitutions (e.g., conservative substitutions), deletions or insertions, or any combination thereof, relative to SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
42) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
43) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
44) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
45) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
46) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
47) the heavy chain variable region comprises the amino acid sequence as set forth in SEQ ID NO: 62, or an amino acid sequence comprising one or more amino acid substitutions (e.g., conservative substitutions), deletions or insertions, or any combination thereof, relative to SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62, and the light chain variable region comprises the amino acid sequence as set forth in SEQ ID NO: 60, or an amino acid sequence comprising one or more amino acid substitutions (e.g., conservative substitutions), deletions or insertions, or any combination thereof, relative to SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;
48) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
49) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
50) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
51) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
52) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
53) the heavy chain variable region comprises the amino acid sequence as set forth in SEQ ID NO: 63, or an amino acid sequence comprising one or more amino acid substitutions (e.g., conservative substitutions), deletions or insertions, or any combination thereof, relative to SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino acid sequence as set forth in SEQ ID NO: 60, or an amino acid sequence comprising one or more amino acid substitutions (e.g., conservative substitutions), deletions or insertions, or any combination thereof, relative to SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;
54) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 55, or an amino acid sequence comprising one or more amino acid substitutions (e.g., conservative substitutions), deletions or insertions, or any combination thereof, relative to SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;
55) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 56, or an amino acid sequence comprising one or more amino acid substitutions (e.g., conservative substitutions), deletions or insertions, or any combination thereof, relative to SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;
56) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 57, or an amino acid sequence comprising one or more amino acid substitutions (e.g., conservative substitutions), deletions or insertions, or any combination thereof, relative to SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;
57) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 58, or an amino acid sequence comprising one or more amino acid substitutions (e.g., conservative substitutions), deletions or insertions, or any combination thereof, relative to SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;
58) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 59, or an amino acid sequence comprising one or more amino acid substitutions (e.g., conservative substitutions), deletions or insertions, or any combination thereof, relative to SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;
59) the heavy chain variable region comprises the amino acid sequence as set forth in SEQ ID NO: 64, or an amino acid sequence comprising one or more amino acid substitutions (e.g., conservative substitutions), deletions or insertions, or any combination thereof, relative to SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64, and the light chain variable region comprises the amino acid sequence as set forth in SEQ ID NO: 60, or an amino acid sequence comprising one or more amino acid substitutions (e.g., conservative substitutions), deletions or insertions, or any combination thereof, relative to SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;
60) the heavy chain variable region comprises the amino acid sequence as set forth in SEQ ID NO: 43, or an amino acid sequence comprising one or more amino acid substitutions (e.g., conservative substitutions), deletions or insertions, or any combination thereof, relative to SEQ ID NO: 43, or an amino acid sequence having at least 90%, 91%, 92%, 93%, 94%, 95%, 96%, 97%, 98% or 99% identity to the amino acid sequence of SEQ ID NO: 43, or consists of SEQ ID NO: 43, and the light chain variable region comprises the amino acid sequence as set forth in SEQ ID NO: 47, or an amino acid sequence comprising one or more amino acid substitutions (e.g., conservative substitutions), deletions or insertions, or any combination thereof, relative to SEQ ID NO: 47, or an amino acid sequence having at least 90%, 91%, 92%, 93%, 94%, 95%, 96%, 97%, 98% or 99% identity to the amino acid sequence of SEQ ID NO: 47, or consists of SEQ ID NO: 47;
61) the heavy chain variable region comprises the amino acid sequence as set forth in SEQ ID NO: 43, or an amino acid sequence comprising one or more amino acid substitutions (e.g., conservative substitutions), deletions or insertions, or any combination thereof, relative to SEQ ID NO: 43, or an amino acid sequence having at least 90%, 91%, 92%, 93%, 94%, 95%, 96%, 97%, 98% or 99% identity to the amino acid sequence of SEQ ID NO: 43, or consists of SEQ ID NO: 43, and the light chain variable region comprises the amino acid sequence as set forth in SEQ ID NO: 48, or an amino acid sequence comprising one or more amino acid substitutions (e.g., conservative substitutions), deletions or insertions, or any combination thereof, relative to SEQ ID NO: 48, or an amino acid sequence having at least 90%, 91%, 92%, 93%, 94%, 95%, 96%, 97%, 98% or 99% identity to the amino acid sequence of SEQ ID NO: 48, or consists of SEQ ID NO: 48;
62) the heavy chain variable region comprises the amino acid sequence as set forth in SEQ ID NO: 43, or an amino acid sequence comprising one or more amino acid substitutions (e.g., conservative substitutions), deletions or insertions, or any combination thereof, relative to SEQ ID NO: 43, or an amino acid sequence having at least 90%, 91%, 92%, 93%, 94%, 95%, 96%, 97%, 98% or 99% identity to the amino acid sequence of SEQ ID NO: 43, or consists of SEQ ID NO: 43, and the light chain variable region comprises the amino acid sequence as set forth in SEQ ID NO: 49, or an amino acid sequence comprising one or more amino acid substitutions (e.g., conservative substitutions), deletions or insertions, or any combination thereof, relative to SEQ ID NO: 49, or an amino acid sequence having at least 90%, 91%, 92%, 93%, 94%, 95%, 96%, 97%, 98% or 99% identity to the amino acid sequence of SEQ ID NO: 49, or consists of SEQ ID NO: 49;
63) the heavy chain variable region comprises the amino acid sequence as set forth in SEQ ID NO: 43, or an amino acid sequence comprising one or more amino acid substitutions (e.g., conservative substitutions), deletions or insertions, or any combination thereof, relative to SEQ ID NO: 43, or an amino acid sequence having at least 90%, 91%, 92%, 93%, 94%, 95%, 96%, 97%, 98% or 99% identity to the amino acid sequence of SEQ ID NO: 43, or consists of SEQ ID NO: 43, and the light chain variable region comprises the amino acid sequence as set forth in SEQ ID NO: 50, or an amino acid sequence comprising one or more amino acid substitutions (e.g., conservative substitutions), deletions or insertions, or any combination thereof, relative to SEQ ID NO: 50, or an amino acid sequence having at least 90%, 91%, 92%, 93%, 94%, 95%, 96%, 97%, 98% or 99% identity to the amino acid sequence of SEQ ID NO: 50, or consists of SEQ ID NO: 50;
64) the heavy chain variable region comprises the amino acid sequence as set forth in SEQ ID NO: 44, or an amino acid sequence comprising one or more amino acid substitutions (e.g., conservative substitutions), deletions or insertions, or any combination thereof, relative to SEQ ID NO: 44, or an amino acid sequence having at least 90%, 91%, 92%, 93%, 94%, 95%, 96%, 97%, 98% or 99% identity to the amino acid sequence of SEQ ID NO: 44, or consists of SEQ ID NO: 44, and the light chain variable region comprises the amino acid sequence as set forth in SEQ ID NO: 47, or an amino acid sequence comprising one or more amino acid substitutions (e.g., conservative substitutions), deletions or insertions, or any combination thereof, relative to SEQ ID NO: 47, or an amino acid sequence having at least 90%, 91%, 92%, 93%, 94%, 95%, 96%, 97%, 98% or 99% identity to the amino acid sequence of SEQ ID NO: 47, or consists of SEQ ID NO: 47;
65) the heavy chain variable region comprises the amino acid sequence as set forth in SEQ ID NO: 44, or an amino acid sequence comprising one or more amino acid substitutions (e.g., conservative substitutions), deletions or insertions, or any combination thereof, relative to SEQ ID NO: 44, or an amino acid sequence having at least 90%, 91%, 92%, 93%, 94%, 95%, 96%, 97%, 98% or 99% identity to the amino acid sequence of SEQ ID NO: 44, or consists of SEQ ID NO: 44, and the light chain variable region comprises the amino acid sequence as set forth in SEQ ID NO: 48, or an amino acid sequence comprising one or more amino acid substitutions (e.g., conservative substitutions), deletions or insertions, or any combination thereof, relative to SEQ ID NO: 48, or an amino acid sequence having at least 90%, 91%, 92%, 93%, 94%, 95%, 96%, 97%, 98% or 99% identity to the amino acid sequence of SEQ ID NO: 48, or consists of SEQ ID NO: 48;
66) the heavy chain variable region comprises the amino acid sequence as set forth in SEQ ID NO: 44, or an amino acid sequence comprising one or more amino acid substitutions (e.g., conservative substitutions), deletions or insertions, or any combination thereof, relative to SEQ ID NO: 44, or an amino acid sequence having at least 90%, 91%, 92%, 93%, 94%, 95%, 96%, 97%, 98% or 99% identity to the amino acid sequence of SEQ ID NO: 44, or consists of SEQ ID NO: 44, and the light chain variable region comprises the amino acid sequence as set forth in SEQ ID NO: 49, or an amino acid sequence comprising one or more amino acid substitutions (e.g., conservative substitutions), deletions or insertions, or any combination thereof, relative to SEQ ID NO: 49, or an amino acid sequence having at least 90%, 91%, 92%, 93%, 94%, 95%, 96%, 97%, 98% or 99% identity to the amino acid sequence of SEQ ID NO: 49, or consists of SEQ ID NO: 49;
67) the heavy chain variable region comprises the amino acid sequence as set forth in SEQ ID NO: 44, or an amino acid sequence comprising one or more amino acid substitutions (e.g., conservative substitutions), deletions or insertions, or any combination thereof, relative to SEQ ID NO: 44, or an amino acid sequence having at least 90%, 91%, 92%, 93%, 94%, 95%, 96%, 97%, 98% or 99% identity to the amino acid sequence of SEQ ID NO: 44, or consists of SEQ ID NO: 44, and the light chain variable region comprises the amino acid sequence as set forth in SEQ ID NO: 50, or an amino acid sequence comprising one or more amino acid substitutions (e.g., conservative substitutions), deletions or insertions, or any combination thereof, relative to SEQ ID NO: 50, or an amino acid sequence having at least 90%, 91%, 92%, 93%, 94%, 95%, 96%, 97%, 98% or 99% identity to the amino acid sequence of SEQ ID NO: 50, or consists of SEQ ID NO: 50;
68) the heavy chain variable region comprises the amino acid sequence as set forth in SEQ ID NO: 45, or an amino acid sequence comprising one or more amino acid substitutions (e.g., conservative substitutions), deletions or insertions, or any combination thereof, relative to SEQ ID NO: 45, or an amino acid sequence having at least 90%, 91%, 92%, 93%, 94%, 95%, 96%, 97%, 98% or 99% identity to the amino acid sequence of SEQ ID NO: 45, or consists of SEQ ID NO: 45, and the light chain variable region comprises the amino acid sequence as set forth in SEQ ID NO: 47, or an amino acid sequence comprising one or more amino acid substitutions (e.g., conservative substitutions), deletions or insertions, or any combination thereof, relative to SEQ ID NO: 47, or an amino acid sequence having at least 90%, 91%, 92%, 93%, 94%, 95%, 96%, 97%, 98% or 99% identity to the amino acid sequence of SEQ ID NO: 47, or consists of SEQ ID NO: 47;
69) the heavy chain variable region comprises the amino acid sequence as set forth in SEQ ID NO: 45, or an amino acid sequence comprising one or more amino acid substitutions (e.g., conservative substitutions), deletions or insertions, or any combination thereof, relative to SEQ ID NO: 45, or an amino acid sequence having at least 90%, 91%, 92%, 93%, 94%, 95%, 96%, 97%, 98% or 99% identity to the amino acid sequence of SEQ ID NO: 45, or consists of SEQ ID NO: 45, and the light chain variable region comprises the amino acid sequence as set forth in SEQ ID NO: 48, or an amino acid sequence comprising one or more amino acid substitutions (e.g., conservative substitutions), deletions or insertions, or any combination thereof, relative to SEQ ID NO: 48, or an amino acid sequence having at least 90%, 91%, 92%, 93%, 94%, 95%, 96%, 97%, 98% or 99% identity to the amino acid sequence of SEQ ID NO: 48, or consists of SEQ ID NO: 48;
70) the heavy chain variable region comprises the amino acid sequence as set forth in SEQ ID NO: 45, or an amino acid sequence comprising one or more amino acid substitutions (e.g., conservative substitutions), deletions or insertions, or any combination thereof, relative to SEQ ID NO: 45, or an amino acid sequence having at least 90%, 91%, 92%, 93%, 94%, 95%, 96%, 97%, 98% or 99% identity to the amino acid sequence of SEQ ID NO: 45, or consists of SEQ ID NO: 45, and the light chain variable region comprises the amino acid sequence as set forth in SEQ ID NO: 49, or an amino acid sequence comprising one or more amino acid substitutions (e.g., conservative substitutions), deletions or insertions, or any combination thereof, relative to SEQ ID NO: 49, or an amino acid sequence having at least 90%, 91%, 92%, 93%, 94%, 95%, 96%, 97%, 98% or 99% identity to the amino acid sequence of SEQ ID NO: 49, or consists of SEQ ID NO: 49;
71) the heavy chain variable region comprises the amino acid sequence as set forth in SEQ ID NO: 45, or an amino acid sequence comprising one or more amino acid substitutions (e.g., conservative substitutions), deletions or insertions, or any combination thereof, relative to SEQ ID NO: 45, or an amino acid sequence having at least 90%, 91%, 92%, 93%, 94%, 95%, 96%, 97%, 98% or 99% identity to the amino acid sequence of SEQ ID NO: 45, or consists of SEQ ID NO: 45, and the light chain variable region comprises the amino acid sequence as set forth in SEQ ID NO: 50, or an amino acid sequence comprising one or more amino acid substitutions (e.g., conservative substitutions), deletions or insertions, or any combination thereof, relative to SEQ ID NO: 50, or an amino acid sequence having at least 90%, 91%, 92%, 93%, 94%, 95%, 96%, 97%, 98% or 99% identity to the amino acid sequence of SEQ ID NO: 50, or consists of SEQ ID NO: 50;
72) the heavy chain variable region comprises the amino acid sequence as set forth in SEQ ID NO: 46, or an amino acid sequence comprising one or more amino acid substitutions (e.g., conservative substitutions), deletions or insertions, or any combination thereof, relative to SEQ ID NO: 46, or an amino acid sequence having at least 90%, 91%, 92%, 93%, 94%, 95%, 96%, 97%, 98% or 99% identity to the amino acid sequence of SEQ ID NO: 46, or consists of SEQ ID NO: 46, and the light chain variable region comprises the amino acid sequence as set forth in SEQ ID NO: 47, or an amino acid sequence comprising one or more amino acid substitutions (e.g., conservative substitutions), deletions or insertions, or any combination thereof, relative to SEQ ID NO: 47, or an amino acid sequence having at least 90%, 91%, 92%, 93%, 94%, 95%, 96%, 97%, 98% or 99% identity to the amino acid sequence of SEQ ID NO: 47, or consists of SEQ ID NO: 47;
73) the heavy chain variable region comprises the amino acid sequence as set forth in SEQ ID NO: 46, or an amino acid sequence comprising one or more amino acid substitutions (e.g., conservative substitutions), deletions or insertions, or any combination thereof, relative to SEQ ID NO: 46, or an amino acid sequence having at least 90%, 91%, 92%, 93%, 94%, 95%, 96%, 97%, 98% or 99% identity to the amino acid sequence of SEQ ID NO: 46, or consists of SEQ ID NO: 46, and the light chain variable region comprises the amino acid sequence as set forth in SEQ ID NO: 48, or an amino acid sequence comprising one or more amino acid substitutions (e.g., conservative substitutions), deletions or insertions, or any combination thereof, relative to SEQ ID NO: 48, or an amino acid sequence having at least 90%, 91%, 92%, 93%, 94%, 95%, 96%, 97%, 98% or 99% identity to the amino acid sequence of SEQ ID NO: 48, or consists of SEQ ID NO: 48;
74) the heavy chain variable region comprises the amino acid sequence as set forth in SEQ ID NO: 46, or an amino acid sequence comprising one or more amino acid substitutions (e.g., conservative substitutions), deletions or insertions, or any combination thereof, relative to SEQ ID NO: 46, or an amino acid sequence having at least 90%, 91%, 92%, 93%, 94%, 95%, 96%, 97%, 98% or 99% identity to the amino acid sequence of SEQ ID NO: 46, or consists of SEQ ID NO: 46, and the light chain variable region comprises the amino acid sequence as set forth in SEQ ID NO: 49, or an amino acid sequence comprising one or more amino acid substitutions (e.g., conservative substitutions), deletions or insertions, or any combination thereof, relative to SEQ ID NO: 49, or an amino acid sequence having at least 90%, 91%, 92%, 93%, 94%, 95%, 96%, 97%, 98% or 99% identity to the amino acid sequence of SEQ ID NO: 49, or consists of SEQ ID NO: 49; or
75) the heavy chain variable region comprises the amino acid sequence as set forth in SEQ ID NO: 46, or an amino acid sequence comprising one or more amino acid substitutions (e.g., conservative substitutions), deletions or insertions, or any combination thereof, relative to SEQ ID NO: 46, or an amino acid sequence having at least 90%, 91%, 92%, 93%, 94%, 95%, 96%, 97%, 98% or 99% identity to the amino acid sequence of SEQ ID NO: 46, or consists of SEQ ID NO: 46, and the light chain variable region comprises the amino acid sequence as set forth in SEQ ID NO: 50, or an amino acid sequence comprising one or more amino acid substitutions (e.g., conservative substitutions), deletions or insertions, or any combination thereof, relative to SEQ ID NO: 50, or an amino acid sequence having at least 90%, 91%, 92%, 93%, 94%, 95%, 96%, 97%, 98% or 99% identity to the amino acid sequence of SEQ ID NO: 50, or consists of SEQ ID NO: 50.

4. The anti- FOLR1 antibody or antigen-binding fragment thereof according to any one of claims 1 to 3, comprising a constant region sequence, wherein at least a portion of the constant region sequence is a human consensus constant region sequence, preferably, the heavy chain constant region sequence is as set forth in SEQ ID NO: 33, and the light chain constant region sequence is as set forth in SEQ ID NO: 34.

5. The anti-FOLR1 antibody or antigen-binding fragment thereof according to any one of claims 1-4, comprising a heavy chain and/or a light chain, wherein:
1) the heavy chain comprises the amino acid sequence as set forth in SEQ ID NO: 65, or an amino acid sequence comprising one or more amino acid substitutions (e.g., conservative substitutions), deletions or insertions, or any combination thereof, relative to SEQ ID NO: 65, or an amino acid sequence having at least 90%, 91%, 92%, 93%, 94%, 95%, 96%, 97%, 98% or 99% identity to the amino acid sequence of SEQ ID NO: 65, or consists of SEQ ID NO: 65, and the light chain comprises the amino acid sequence as set forth in SEQ ID NO: 66, or an amino acid sequence comprising one or more amino acid substitutions (e.g., conservative substitutions), deletions or insertions, or any combination thereof, relative to SEQ ID NO: 66, or an amino acid sequence having at least 90%, 91%, 92%, 93%, 94%, 95%, 96%, 97%, 98% or 99% identity to the amino acid sequence of SEQ ID NO: 66, or consists of SEQ ID NO: 66;
2) the heavy chain comprises the amino acid sequence as set forth in SEQ ID NO: 67, or an amino acid sequence comprising one or more amino acid substitutions (e.g., conservative substitutions), deletions or insertions, or any combination thereof, relative to SEQ ID NO: 67, or an amino acid sequence having at least 90%, 91%, 92%, 93%, 94%, 95%, 96%, 97%, 98% or 99% identity to the amino acid sequence of SEQ ID NO: 67, or consists of SEQ ID NO: 67, and the light chain comprises the amino acid sequence as set forth in SEQ ID NO: 68, or an amino acid sequence comprising one or more amino acid substitutions (e.g., conservative substitutions), deletions or insertions, or any combination thereof, relative to SEQ ID NO: 68, or an amino acid sequence having at least 90%, 91%, 92%, 93%, 94%, 95%, 96%, 97%, 98% or 99% identity to the amino acid sequence of SEQ ID NO: 68, or consists of SEQ ID NO: 68;
3) the heavy chain comprises the amino acid sequence as set forth in SEQ ID NO: 69, or an amino acid sequence comprising one or more amino acid substitutions (e.g., conservative substitutions), deletions or insertions, or any combination thereof, relative to SEQ ID NO: 69, or an amino acid sequence having at least 90%, 91%, 92%, 93%, 94%, 95%, 96%, 97%, 98% or 99% identity to the amino acid sequence of SEQ ID NO: 69, or consists of SEQ ID NO: 69, and the light chain comprises the amino acid sequence as set forth in SEQ ID NO: 70, or an amino acid sequence comprising one or more amino acid substitutions (e.g., conservative substitutions), deletions or insertions, or any combination thereof, relative to SEQ ID NO: 70, or an amino acid sequence having at least 90%, 91%, 92%, 93%, 94%, 95%, 96%, 97%, 98% or 99% identity to the amino acid sequence of SEQ ID NO: 70, or consists of SEQ ID NO: 70;
4) the heavy chain comprises the amino acid sequence as set forth in SEQ ID NO: 71, or an amino acid sequence comprising one or more amino acid substitutions (e.g., conservative substitutions), deletions or insertions, or any combination thereof, relative to SEQ ID NO: 71, or an amino acid sequence having at least 90%, 91%, 92%, 93%, 94%, 95%, 96%, 97%, 98% or 99% identity to the amino acid sequence of SEQ ID NO: 71, or consists of SEQ ID NO: 71, and the light chain comprises the amino acid sequence as set forth in SEQ ID NO: 72, or an amino acid sequence comprising one or more amino acid substitutions (e.g., conservative substitutions), deletions or insertions, or any combination thereof, relative to SEQ ID NO: 72, or an amino acid sequence having at least 90%, 91%, 92%, 93%, 94%, 95%, 96%, 97%, 98% or 99% identity to the amino acid sequence of SEQ ID NO: 72, or consists of SEQ ID NO: 72;
5) the heavy chain comprises the amino acid sequence as set forth in SEQ ID NO: 73, or an amino acid sequence comprising one or more amino acid substitutions (e.g., conservative substitutions), deletions or insertions, or any combination thereof, relative to SEQ ID NO: 73, or an amino acid sequence having at least 90%, 91%, 92%, 93%, 94%, 95%, 96%, 97%, 98% or 99% identity to the amino acid sequence of SEQ ID NO: 73, or consists of SEQ ID NO: 73, and the light chain comprises the amino acid sequence as set forth in SEQ ID NO: 74, or an amino acid sequence comprising one or more amino acid substitutions (e.g., conservative substitutions), deletions or insertions, or any combination thereof, relative to SEQ ID NO: 74, or an amino acid sequence having at least 90%, 91%, 92%, 93%, 94%, 95%, 96%, 97%, 98% or 99% identity to the amino acid sequence of SEQ ID NO: 74, or consists of SEQ ID NO: 74;
6) the heavy chain comprises the amino acid sequence as set forth in SEQ ID NO: 75, or an amino acid sequence comprising one or more amino acid substitutions (e.g., conservative substitutions), deletions or insertions, or any combination thereof, relative to SEQ ID NO: 75, or an amino acid sequence having at least 90%, 91%, 92%, 93%, 94%, 95%, 96%, 97%, 98% or 99% identity to the amino acid sequence of SEQ ID NO: 75, or consists of SEQ ID NO: 75, and the light chain comprises the amino acid sequence as set forth in SEQ ID NO: 76, or an amino acid sequence comprising one or more amino acid substitutions (e.g., conservative substitutions), deletions or insertions, or any combination thereof, relative to SEQ ID NO: 76, or an amino acid sequence having at least 90%, 91%, 92%, 93%, 94%, 95%, 96%, 97%, 98% or 99% identity to the amino acid sequence of SEQ ID NO: 76, or consists of SEQ ID NO: 76;
7) the heavy chain comprises the amino acid sequence as set forth in SEQ ID NO: 77, or an amino acid sequence comprising one or more amino acid substitutions (e.g., conservative substitutions), deletions or insertions, or any combination thereof, relative to SEQ ID NO: 77, or an amino acid sequence having at least 90%, 91%, 92%, 93%, 94%, 95%, 96%, 97%, 98% or 99% identity to the amino acid sequence of SEQ ID NO: 77, or consists of SEQ ID NO: 77, and the light chain comprises the amino acid sequence as set forth in SEQ ID NO: 78, or an amino acid sequence comprising one or more amino acid substitutions (e.g., conservative substitutions), deletions or insertions, or any combination thereof, relative to SEQ ID NO: 78, or an amino acid sequence having at least 90%, 91%, 92%, 93%, 94%, 95%, 96%, 97%, 98% or 99% identity to the amino acid sequence of SEQ ID NO: 78, or consists of SEQ ID NO: 78;
8) the heavy chain comprises the amino acid sequence as set forth in SEQ ID NO: 79, or an amino acid sequence comprising one or more amino acid substitutions (e.g., conservative substitutions), deletions or insertions, or any combination thereof, relative to SEQ ID NO: 79, or an amino acid sequence having at least 90%, 91%, 92%, 93%, 94%, 95%, 96%, 97%, 98% or 99% identity to the amino acid sequence of SEQ ID NO: 79, or consists of SEQ ID NO: 79, and the light chain comprises the amino acid sequence as set forth in SEQ ID NO: 80, or an amino acid sequence comprising one or more amino acid substitutions (e.g., conservative substitutions), deletions or insertions, or any combination thereof, relative to SEQ ID NO: 80, or an amino acid sequence having at least 90%, 91%, 92%, 93%, 94%, 95%, 96%, 97%, 98% or 99% identity to the amino acid sequence of SEQ ID NO: 80, or consists of SEQ ID NO: 80;
9) the heavy chain comprises the amino acid sequence as set forth in SEQ ID NO: 81, or an amino acid sequence comprising one or more amino acid substitutions (e.g., conservative substitutions), deletions or insertions, or any combination thereof, relative to SEQ ID NO: 81, or an amino acid sequence having at least 90%, 91%, 92%, 93%, 94%, 95%, 96%, 97%, 98% or 99% identity to the amino acid sequence of SEQ ID NO: 81, or consists of SEQ ID NO: 81, and the light chain comprises the amino acid sequence as set forth in SEQ ID NO: 82, or an amino acid sequence comprising one or more amino acid substitutions (e.g., conservative substitutions), deletions or insertions, or any combination thereof, relative to SEQ ID NO: 82, or an amino acid sequence having at least 90%, 91%, 92%, 93%, 94%, 95%, 96%, 97%, 98% or 99% identity to the amino acid sequence of SEQ ID NO: 82, or consists of SEQ ID NO: 82;
10) the heavy chain comprises the amino acid sequence as set forth in SEQ ID NO: 83, or an amino acid sequence comprising one or more amino acid substitutions (e.g., conservative substitutions), deletions or insertions, or any combination thereof, relative to SEQ ID NO: 83, or an amino acid sequence having at least 90%, 91%, 92%, 93%, 94%, 95%, 96%, 97%, 98% or 99% identity to the amino acid sequence of SEQ ID NO: 83, or consists of SEQ ID NO: 83, and the light chain comprises the amino acid sequence as set forth in SEQ ID NO: 84, or an amino acid sequence comprising one or more amino acid substitutions (e.g., conservative substitutions), deletions or insertions, or any combination thereof, relative to SEQ ID NO: 84, or an amino acid sequence having at least 90%, 91%, 92%, 93%, 94%, 95%, 96%, 97%, 98% or 99% identity to the amino acid sequence of SEQ ID NO: 84, or consists of SEQ ID NO: 84;
11) the heavy chain comprises the amino acid sequence as set forth in SEQ ID NO: 85, or an amino acid sequence comprising one or more amino acid substitutions (e.g., conservative substitutions), deletions or insertions, or any combination thereof, relative to SEQ ID NO: 85, or an amino acid sequence having at least 90%, 91%, 92%, 93%, 94%, 95%, 96%, 97%, 98% or 99% identity to the amino acid sequence of SEQ ID NO: 85, or consists of SEQ ID NO: 85, and the light chain comprises the amino acid sequence as set forth in SEQ ID NO: 86, or an amino acid sequence comprising one or more amino acid substitutions (e.g., conservative substitutions), deletions or insertions, or any combination thereof, relative to SEQ ID NO: 86, or an amino acid sequence having at least 90%, 91%, 92%, 93%, 94%, 95%, 96%, 97%, 98% or 99% identity to the amino acid sequence of SEQ ID NO: 86, or consists of SEQ ID NO: 86;
12) the heavy chain comprises the amino acid sequence as set forth in SEQ ID NO: 87, or an amino acid sequence comprising one or more amino acid substitutions (e.g., conservative substitutions), deletions or insertions, or any combination thereof, relative to SEQ ID NO: 87, or an amino acid sequence having at least 90%, 91%, 92%, 93%, 94%, 95%, 96%, 97%, 98% or 99% identity to the amino acid sequence of SEQ ID NO: 87, or consists of SEQ ID NO: 87, and the light chain comprises the amino acid sequence as set forth in SEQ ID NO: 88, or an amino acid sequence comprising one or more amino acid substitutions (e.g., conservative substitutions), deletions or insertions, or any combination thereof, relative to SEQ ID NO: 88, or an amino acid sequence having at least 90%, 91%, 92%, 93%, 94%, 95%, 96%, 97%, 98% or 99% identity to the amino acid sequence of SEQ ID NO: 88, or consists of SEQ ID NO: 88;
13) the heavy chain comprises the amino acid sequence as set forth in SEQ ID NO: 89, or an amino acid sequence comprising one or more amino acid substitutions (e.g., conservative substitutions), deletions or insertions, or any combination thereof, relative to SEQ ID NO: 89, or an amino acid sequence having at least 90%, 91%, 92%, 93%, 94%, 95%, 96%, 97%, 98% or 99% identity to the amino acid sequence of SEQ ID NO: 89, or consists of SEQ ID NO: 89, and the light chain comprises the amino acid sequence as set forth in SEQ ID NO: 90, or an amino acid sequence comprising one or more amino acid substitutions (e.g., conservative substitutions), deletions or insertions, or any combination thereof, relative to SEQ ID NO: 90, or an amino acid sequence having at least 90%, 91%, 92%, 93%, 94%, 95%, 96%, 97%, 98% or 99% identity to the amino acid sequence of SEQ ID NO: 90, or consists of SEQ ID NO: 90; or
14) the heavy chain comprises the amino acid sequence as set forth in SEQ ID NO: 91, or an amino acid sequence comprising one or more amino acid substitutions (e.g., conservative substitutions), deletions or insertions, or any combination thereof, relative to SEQ ID NO: 91, or an amino acid sequence having at least 90%, 91%, 92%, 93%, 94%, 95%, 96%, 97%, 98% or 99% identity to the amino acid sequence of SEQ ID NO: 91, or consists of SEQ ID NO: 91, and the light chain comprises the amino acid sequence as set forth in SEQ ID NO: 92, or an amino acid sequence comprising one or more amino acid substitutions (e.g., conservative substitutions), deletions or insertions, or any combination thereof, relative to SEQ ID NO: 92, or an amino acid sequence having at least 90%, 91%, 92%, 93%, 94%, 95%, 96%, 97%, 98% or 99% identity to the amino acid sequence of SEQ ID NO: 92, or consists of SEQ ID NO: 92.

6. The anti-FOLR1 antibody or antigen-binding fragment thereof according to any one of claims 1 to 5, wherein the antibody is a chimeric monoclonal antibody or a humanized monoclonal antibody.

7. The anti-FOLR1 antibody or antigen-binding fragment thereof according to any one of claims 1 to 6, wherein the antigen-binding fragment is selected from Fab, Fab'-SH, Fv (e.g., scFv) or (Fab')₂ fragments.

8. An antibody-drug conjugate having the structure Ab-(L-D)n, wherein Ab is the anti-FOLR1 antibody or antigen-binding fragment thereof that specifically binds FOLR1 according to any one of claims 1-7, L is a linker, D is a therapeutically active substance or pharmaceutically active ingredient, and n = 1-8.

9. The antibody-drug conjugate according to claim 8, wherein the therapeutically active substance or pharmaceutically active ingredient is a cytotoxin, a plant toxin, a small molecule toxin, a radioisotope, a maytansinoid, or a camptothecin derivative, such as MMAE or DXD.

10. The antibody-drug conjugate according to claim 8 or 9, wherein the linker is a protease-cleavable peptide linker, for example glycine-glycine-phenylalanine-glycine (GGFG) or MC-VC-PAB, preferably, the linker may further comprise, for example, a maleimide conjugated to a cysteine residue of the antibody.

11. The antibody-drug conjugate according to any one of claims 8-10, wherein the antibody-drug conjugate has the following structure:

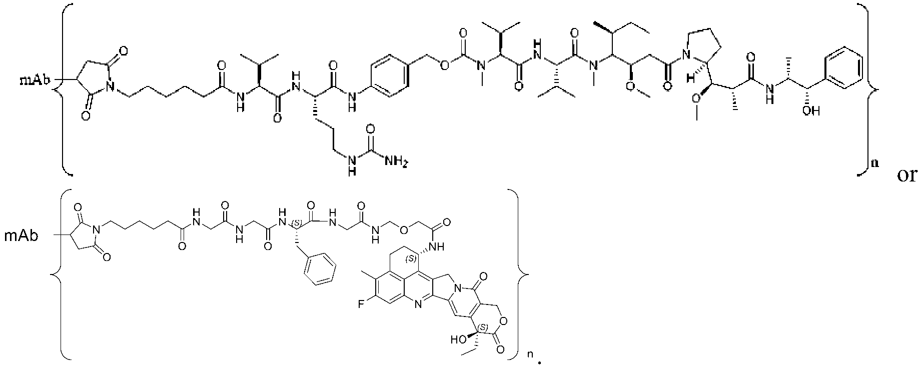

12. The antibody-drug conjugate according to any one of claims 8-11, wherein the antibody-drug conjugate has an average DAR of 1 to 8, for example an average DAR of 3.0-5.0.

13. The antibody-drug conjugate according to any one of claims 8-12, wherein the Ab comprises:
(1) HCDR1, HCDR2, HCDR3 comprising the sequences as set forth in SEQ ID NOs: 1, 2 and 3 and LCDR1, LCDR2, LCDR3 comprising the sequences as set forth in SEQ ID NOs: 5, 6 and 7, or
(2) HCDR1, HCDR2, HCDR3 comprising the sequences as set forth in SEQ ID NOs: 9, 10 and 11 and LCDR1, LCDR2, LCDR3 comprising the sequences as set forth in SEQ ID NOs: 13, 14 and 15, or
(3) HCDR1, HCDR2, HCDR3 comprising the sequences as set forth in SEQ ID NOs: 17, 18 and 19 and LCDR1, LCDR2, LCDR3 comprising the sequences as set forth in SEQ ID NOs: 21, 22 and 23, or
(4) HCDR1, HCDR2, HCDR3 comprising the sequences as set forth in SEQ ID NOs: 25, 26 and 27 and LCDR1, LCDR2, LCDR3 comprising the sequences as set forth in SEQ ID NOs: 29, 30 and 31.

14. The antibody-drug conjugate according to any one of claims 8-13, wherein the Ab comprises a heavy chain variable region and a light chain variable region, wherein:
(1) the heavy chain variable region comprises the amino acid sequence as set forth in SEQ ID NO: 36, and the light chain variable region comprises the amino acid sequence as set forth in SEQ ID NO: 40, or
(2) the heavy chain variable region comprises the amino acid sequence as set forth in SEQ ID NO: 36, and the light chain variable region comprises the amino acid sequence as set forth in SEQ ID NO: 41, or
(3) the heavy chain variable region comprises the amino acid sequence as set forth in SEQ ID NO: 37, and the light chain variable region comprises the amino acid sequence as set forth in SEQ ID NO: 40, or
(4) the heavy chain variable region comprises the amino acid sequence as set forth in SEQ ID NO: 37, and the light chain variable region comprises the amino acid sequence as set forth in SEQ ID NO: 41, or
(5) the heavy chain variable region comprises the amino acid sequence as set forth in SEQ ID NO: 51, and the light chain variable region comprises the amino acid sequence as set forth in SEQ ID NO: 55, or
(6) the heavy chain variable region comprises the amino acid sequence as set forth in SEQ ID NO: 51, and the light chain variable region comprises the amino acid sequence as set forth in SEQ ID NO: 57, or
(7) the heavy chain variable region comprises the amino acid sequence as set forth in SEQ ID NO: 53, and the light chain variable region comprises the amino acid sequence as set forth in SEQ ID NO: 55, or
(8) the heavy chain variable region comprises the amino acid sequence as set forth in SEQ ID NO: 54, and the light chain variable region comprises the amino acid sequence as set forth in SEQ ID NO: 55, or
(9) the heavy chain variable region comprises the amino acid sequence as set forth in SEQ ID NO: 54, and the light chain variable region comprises the amino acid sequence as set forth in SEQ ID NO: 56, or
(10) the heavy chain variable region comprises the amino acid sequence as set forth in SEQ ID NO: 61, and the light chain variable region comprises the amino acid sequence as set forth in SEQ ID NO: 55, or
(11) the heavy chain variable region comprises the amino acid sequence as set forth in SEQ ID NO: 61, and the light chain variable region comprises the amino acid sequence as set forth in SEQ ID NO: 56, or
(12) the heavy chain variable region comprises the amino acid sequence as set forth in SEQ ID NO: 62, and the light chain variable region comprises the amino acid sequence as set forth in SEQ ID NO: 55, or
(13) the heavy chain variable region comprises the amino acid sequence as set forth in SEQ ID NO: 62, and the light chain variable region comprises the amino acid sequence as set forth in SEQ ID NO: 57, or
(14) the heavy chain variable region comprises the amino acid sequence as set forth in SEQ ID NO: 64, and the light chain variable region comprises the amino acid sequence as set forth in SEQ ID NO: 55.

15. The antibody-drug conjugate according to any one of claims 8-14, wherein the Ab comprises:
1) a heavy chain whose amino acid sequence is as set forth in SEQ ID NO: 65 and a light chain whose amino acid sequence is as set forth in SEQ ID NO: 66; or
2) a heavy chain whose amino acid sequence is as set forth in SEQ ID NO: 67 and a light chain whose amino acid sequence is as set forth in SEQ ID NO: 68; or
3) a heavy chain whose amino acid sequence is as set forth in SEQ ID NO: 69 and a light chain whose amino acid sequence is as set forth in SEQ ID NO: 70; or
4) a heavy chain whose amino acid sequence is as set forth in SEQ ID NO: 71 and a light chain whose amino acid sequence is as set forth in SEQ ID NO: 72; or
5) a heavy chain whose amino acid sequence is as set forth in SEQ ID NO: 73 and a light chain whose amino acid sequence is as set forth in SEQ ID NO: 74; or
6) a heavy chain whose amino acid sequence is as set forth in SEQ ID NO: 75 and a light chain whose amino acid sequence is as set forth in SEQ ID NO: 76; or
7) a heavy chain whose amino acid sequence is as set forth in SEQ ID NO: 77 and a light chain whose amino acid sequence is as set forth in SEQ ID NO: 78; or
8) a heavy chain whose amino acid sequence is as set forth in SEQ ID NO: 79 and a light chain whose amino acid sequence is as set forth in SEQ ID NO: 80; or
9) a heavy chain whose amino acid sequence is as set forth in SEQ ID NO: 81 and a light chain whose amino acid sequence is as set forth in SEQ ID NO: 82; or
10) a heavy chain whose amino acid sequence is as set forth in SEQ ID NO: 83 and a light chain whose amino acid sequence is as set forth in SEQ ID NO: 84; or
11) a heavy chain whose amino acid sequence is as set forth in SEQ ID NO: 85 and a light chain whose amino acid sequence is as set forth in SEQ ID NO: 86; or
12) a heavy chain whose amino acid sequence is as set forth in SEQ ID NO: 87 and a light chain whose amino acid sequence is as set forth in SEQ ID NO: 88; or
13) a heavy chain whose amino acid sequence is as set forth in SEQ ID NO: 89 and a light chain whose amino acid sequence is as set forth in SEQ ID NO: 90; or
14) a heavy chain whose amino acid sequence is as set forth in SEQ ID NO: 91 and a light chain whose amino acid sequence is as set forth in SEQ ID NO: 92.

16. A nucleic acid molecule encoding the anti-FOLR1 antibody or antigen-binding fragment thereof according to any one of claims 1-7.

17. A vector comprising the nucleic acid molecule according to claim 16.

18. A host cell comprising the vector according to claim 17 or the nucleic acid molecule according to claim 16.

19. A pharmaceutical composition comprising:
1) the anti-FOLR1 antibody or antigen-binding fragment thereof according to any one of claims 1-7, or the antibody-drug conjugate according to any one of claims 8-15, the nucleic acid molecule according to claim 16, the vector according to claim 17, or the host cell according to claim 18; and
2) a pharmaceutically acceptable carrier.

20. A method of treating cancer in a subject in need thereof, comprising administering to the subject a therapeutically effective amount of the anti-FOLR1 antibody or antigen-binding fragment thereof according to any one of claims 1-7, or the antibody-drug conjugate according to any one of claims 8-15, the nucleic acid molecule according to claim 16, the vector according to claim 17, the host cell according to claim 18, or the pharmaceutical composition according to claim 19.

21. Use of the anti-FOLR1 antibody or antigen-binding fragment thereof according to any one of claims 1-7, or the antibody-drug conjugate according to any one of claims 8-15, the nucleic acid molecule according to claim 16, the vector according to claim 17, the host cell according to claim 18, or the pharmaceutical composition according to claim 19 in the manufacture of a medicament for treating cancer.

22. Use of the anti-FOLR1 antibody or antigen-binding fragment thereof according to any one of claims 1-7 in the manufacture of an antibody-drug conjugate for treating cancer.

23. The method according to claim 20, or the use according to claim 21 or 22, wherein the cancer is a cancer highly expressing FOLR1, such as ovarian cancer, triple-negative breast cancer, endometrial cancer, peritoneal cancer, and lung cancer.
